# EUROPÄISCHE PATENTSCHRIFT

(11) **EP 1 901 776 B1**
(45) Veröffentlichungstag und Bekanntmachung des Hinweises auf die Patenterteilung: **22.02.2012**
(21) Anmeldenummer: 06763946.8
(22) Anmeldetag: 28.06.2006
(51) Int. Cl.: A61K 45/06, A61K 31/538, A61P 11/08

(54) **NEUE ARZNEIMITTELKOMBINATIONEN ZUR BEHANDLUNG VON ATEMWEGSERKRANKUNGEN ENTHALTEND LANGWIRKSAME BETA-2-AGONISTEN UND WENIGSTENS EINEN WEITEREN WIRKSTOFF**
NOVEL MEDICINAL COMBINATIONS WHICH ARE USED FOR TREATING RESPIRATORY TRACT DISEASES AND CONTAIN BETA-2 LONG-ACTING AGONISTS AND AT LEAST ONE OTHER TYPE OF ACTIVE SUBSTANCE
NOUVELLES COMBINAISONS DE MEDICAMENTS PERMETTANT DE TRAITER DES MALADIES DES VOIES RESPIRATOIRES ET CONTENANT DES AGONISTES BETA-2 A ACTION PROLONGEE ET AU MOINS UNE AUTRE SUBSTANCE ACTIVE

(30) Priorität: 01.07.2005 DE 102005030733
(43) Veröffentlichungstag der Anmeldung: 26.03.2008
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: KONETZKI, Ingo, 88447 Warthausen (DE); BOUYSSOU, Thierry, 88447 Warthausen (DE); PESTEL, Sabine, 88448 Attenweiler (DE); SCHNAPP, Andreas, 88400 Biberach (DE)
(74) Vertreter: Hammann, Heinz
(86) Internationale Anmeldenummer: PCT/EP2006/063650
(87) Internationale Veröffentlichungsnummer: WO 2007/003554

(56) Entgegenhaltungen:
- WO-A-00/12078
- WO-A-2004/033412
- WO-A-2005/013994
- WO-A-2005/070908
- WO-A1-2004/047829
- WO-A2-2004/045618
- WO-A2-2005/013945
- WESSELING G ET AL: "COMPARISON OF THE EFFECTS OF ANTICHOLINERGIC AND BETA2-AGONIST AND COMBINATION THERAPY ON RESPIRATORY IMPEDANCE IN COPD" CHEST, THE COLLEGE, CHICAGO, IL, US, Bd. 101, Nr. 1, 1992, Seiten 166-173, XP000913861 ISSN: 0012-3692
- CALVO G M ET AL: "IS IT USEFUL TO ADD AN ANTICHOLINERGIC TREATMENT TO BETA2-ADRENERGIC MEDICATION IN ACUTE ASTHMA ATTACK" JOURNAL OF INVESTIGATIONAL ALLERGOLOGY AND CLINICAL IMMUNOLOGY, BARCELONA, ES, Bd. 8, Nr. 1, Januar 1998 (1998-01), Seiten 30-34, XP009018610 ISSN: 1018-9068
- FAN CHUNG K ET AL: "COMBINATION THERAPY OF LONG-ACTING BETA2-ADRENOCEPTOR AGONISTS AND CORTICOSTEROIDS FOR ASTHMA" TREATMENT IN RESPIRATORY MEDICINE, ADIS,, NZ, Bd. 3, Nr. 5, 2004, Seiten 279-289, XP008042552 ISSN: 1176-3450

## Beschreibung

Die vorliegende Erfindung betrifft neue Arzneimittelkombinationen, die neben einer oder mehrerer, bevorzugt einer Verbindung der allgemeinen Formel **1** 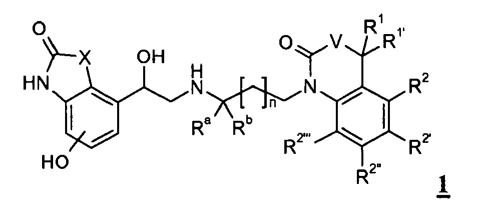 worin die Gruppen X, R^{a}, R^{b}, R¹, R¹, R², R^{2'}, R^{2"}, R^{2"'}, V und n die in den Ansprüchen und in der Beschreibung genannten Bedeutungen haben können, wenigstens einen weiteren Wirkstoff **2** enthalten, Verfahren zu deren Herstellung sowie deren Verwendung als Arzneimittel.

### Detaillierte Beschreibung der Erfindung

Die vorliegende Erfindung betrifft Arzneimittelkombinationen, die neben einer oder mehrerer, bevorzugt einer Verbindung der allgemeinen Formel **1** 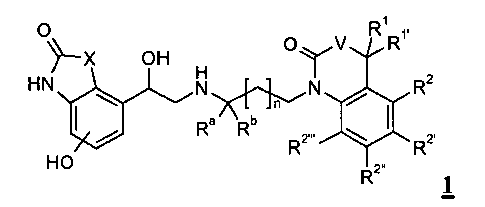 worin
- X: ein Gruppe -O-, -NH-, -CH₂-O-, -CHMe-O-, -C(Me)₂-O-, -CH₂-NH-, -CHMe-NH-, -C(Me)₂-NH-, -CH=CH- oder -CH₂-CH₂-;
- V: eine zweibindige Gruppe ausgewählt aus der Gruppe bestehend aus CH₂, NH und O, bevorzugt CH₂ und O, besonders bevorzugt O;
- R^{a} und R^{b}: gleich oder verschieden, ein Rest ausgewählt aus der Gruppe bestehend aus Wasserstoff, C₁₋₄-Alkyl, und Halogen-C₁₋₄-alkyl, oder
- R^{a} und R^{b}: gemeinsam eine C₂₋₅-alkylen-Brücke, in der ein oder mehrere Wasserstoffatome gegebenenfalls ersetzt sein können durch Halogen;
- R¹ und R^{1'}: gleich oder verschieden, ein Rest ausgewählt aus der Gruppe bestehend aus Wasserstoff, C₁₋₆-Alkyl, C₃₋₆-Cycloalkyl, Halogen-C₁₋₆-alkyl, Halogen-C₃₋₆-cycloalkyl oder C₁₋₆-Alkylen-C₃₋₆-cycloakl, oder
- R¹ und R^{1'}: gemeinsam eine C₂₋₅-alkylen-Brücke in der ein oder mehrere Wasserstoffatome gegebenenfalls ersetzt sein können durch Halogen;
- R², R^{2'}, R^{2"} und R^{2"'}: gleich oder verschieden, ein Rest ausgewählt aus der Gruppe bestehend aus Wasserstoff, C₁₋₆-Alkyl, Halogen-C₁₋₆-alkylen, OH, HO-C₁₋₆-alkylen, -O-C₁₋₆-Alkyl, C₆₋₁₀-Aryl, C₆₋₁₀-Aryl-C₁₋₄-alkylen, C₆₋₁₀-Aryl-C₁₋₆-alkylen-O-, COOH, COOC₁₋₆-alkyl, O-C₁₋₆-alkylen-COOH, O-C₁₋₆-alkylen-COOC₁₋₆-alkyl, NHSO₂-C₁₋₆-alkyl, CN, NH₂, NH-C₁₋₆-Alkyl, N(C₁₋₆-Alkyl)₂, NO₂, S-C₁₋₆-Alkyl, SO₂-C₁₋₆-Alkyl, SO-C₁₋₆-Alkyl, O(CO)C₁₋₆-Alkyl, COC₁₋₆-Alkyl, NHCOC₁₋₆-Alkyl oder Halogen;
- n: 0, 1 oder 2; bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, wenigstens einen weiteren Wirkstoff **2** enthalten, wobei als weiteren Wirkstoff **2** eine oder mehrere Verbindungen, die ausgewählt sind aus den Klassen der Anticholinergika (**2a**), PDEIV-Inhibitoren (**2b**), Steroide (**2c**), LTD4-Antagonisten (2d) und EGFR-Hemmer (**2e**) verstanden werden

Bevorzugt sind Arzneimittelkombinationen, die neben einer oder mehrerer, bevorzugt einer Verbindung der allgemeinen Formel **1**, worin
- X: -O-, -CH₂-O-, -C(Me)₂-O- oder -CH=CH-;
- V: eine zweibindige Gruppe ausgewählt aus der Gruppe bestehend aus CH₂, NH und O, bevorzugt CH₂ und O, besonders bevorzugt O;
- R^{a} und R^{b}: gleich oder verschieden, ein Rest ausgewählt aus der Gruppe bestehend aus Wasserstoff, C₁₋₄-Alkyl und Fluor-C₁₋₄-alkyl, oder
- R^{a} und R^{b}: gemeinsam eine Gruppe ausgewählt aus -CH₂-CH₂-, -CH₂-CH₂-CH₂-CH₂- und -CH₂-CH₂-CH₂-CH₂-CH₂-, in der ein oder mehrere Wasserstoffatome gegebenenfalls ersetzt sein können durch Fluor oder Chlor, bevorzugt Fluor;
- R¹ und R^{1'}: gleich oder verschieden, ein Rest ausgewählt aus der Gruppe bestehend aus Wasserstoff, C₁₋₆-Alkyl, C₃₋₆-Cycloalkyl, Halogen-C₁₋₆-alkyl oder C₁₋₆-Alkylen-C₃₋₆-Cycloalkyl, oder
- R¹ und R^{1'}: gemeinsam eine Gruppe ausgewählt aus -CH₂-CH₂-, -CH₂-CH₂-CH₂-CH₂- und -CH₂-CH₂-CH₂-CH₂-CH₂-, in der ein oder mehrere Wasserstoffatome gegebenenfalls ersetzt sein können durch Fluor oder Chlor, bevorzugt Fluor;
- R², R^{2'}, R^{2"} und R^{2"'}: gleich oder verschieden, ein Rest ausgewählt aus der Gruppe bestehend aus Wasserstoff, C₁₋₄-Alkyl, CF₃, CHF₂, CH₂F, OH, -O-C₁₋₄-Alkyl, Phenyl, Phenylethyl, Benzyl, Phenyloxy, Benzyloxy, COOH, COOC₁₋₄-alkyl, OCH₂COOH, OCH₂COOC₁₋₄-alkyl, NHSO₂-C₁₋₄-alkyl, Fluor, Chlor oder Brom;
- n: 0, 1 oder 2; bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, wenigstens einen weiteren Wirkstoff **2** enthalten.

Bevorzugt sind Arzneimittelkombinationen, die neben einer oder mehrerer, bevorzugt einer Verbindung der allgemeinen Formel **1**, worin
- X: -O-, -CH₂-O-, -C(Me)₂-O- oder -CH=CH-;
- V: eine zweibindige Gruppe ausgewählt aus der Gruppe bestehend aus CH₂ und O, bevorzugt O;
- R^{a} und R^{b}: gleich oder verschieden, ein Rest ausgewählt aus der Gruppe bestehend aus Wasserstoff, Methyl, Ethyl und CF₃, bevorzugt Wasserstoff, Methyl oder Ethyl, oder
- R^{a} und R^{b}: gemeinsam eine Gruppe ausgewählt aus -CH₂-CH₂- und -CH₂-CH₂-CH₂-CH₂-, bevorzugt -CH₂-CH₂-;
- R¹ und R^{1'}: gleich oder verschieden, ein Rest ausgewählt aus der Gruppe bestehend aus Wasserstoff, Methyl, Ethyl, Propyl, Cyclopropyl oder Methylcyclopropyl, oder
- R¹ und R^{1'}: gemeinsam -CH₂-CH₂-CH₂-CH₂- oder -CH₂-CH₂-CH₂-CH₂-CH₂-;
- R², R^{2'}, R^{2"} und R^{2"'}: gleich oder verschieden, ein Rest ausgewählt aus der Gruppe bestehend aus Wasserstoff, Methyl, Ethyl, Propyl, CF₃, CHF₂, CH₂F, OH, Methyloxy, Ethyloxy, Propyloxy, COOH, COOCH₃, COOCH₂CH₃, OCH₂COOH, OCH₂COOCH₃, NHSO₂-CH₃, Fluor, Chlor oder Brom;
- n: 0, 1 oder 2; bevorzugt 1
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren, wenigstens einen weiteren Wirkstoff **2** enthalten.

Von erfindungsgemäß besonderer Bedeutung sind Arzneimittelkombinationen, die Verbindungen der Formel **1**, in denen R^{a} und R^{b} beide Methyl und in denen die Gruppen X, R¹, R^{1'}, R², R^{2'}, R^{2"}, R^{2"'}, V und n die vorstehend genannten Bedeutungen haben können enthalten. Diese bevorzugten Verbindungen sind darstellbar durch die nachstehende allgemeine Formel **1.1** 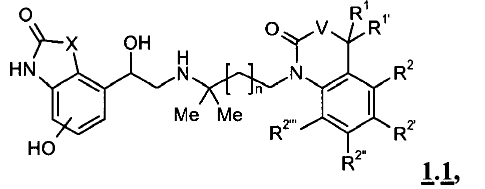 worin die Gruppen X, R¹, R^{1'}, R², R^{2'}, R^{2"}, R^{2"'}, V und n die vorstehend genannten Bedeutungen haben können.

Von erfindungsgemäß besonderer Bedeutung sind Arzneimittelkombinationen, die Verbindungen der Formel **1**, worin X der Gruppe -CH₂-O- entspricht enthalten. Diese Verbindungen sind darstellbar durch die Formel **1'** 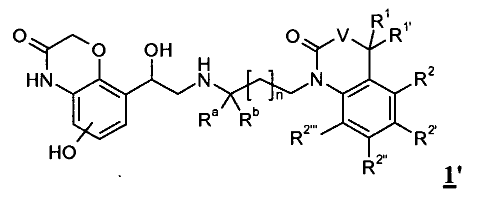 worin die Gruppen R^{a}, R^{b}, R¹, R^{1'}, R², R^{2'}, R^{2"}, R^{2"'} und V die oben stehende Bedeutung haben.

Bevorzugt sind Arzneimittelkombinationen, die Verbindungen der Formel **1'** enthalten, worin R^{a} und R^{b} die vorstehend genannten Bedeutungen haben können und worin
- V: eine zweibindige Gruppe ausgewählt aus der Gruppe bestehend aus CH₂ und O, bevorzugt O;
- R¹ und R^{1'}: gleich oder verschieden, ein Rest ausgewählt aus der Gruppe bestehend aus Wasserstoff, Methyl, Ethyl, Propyl oder Cyclopropyl, oder
- R¹ und R^{1'}: gemeinsam -CH₂-CH₂-CH₂-CH₂- oder -CH₂-CH₂-CH₂-CH₂-CH₂-;
- R² und R^{2"'}: Wasserstoff;
- R^{2'} und R^{2"}: gleich oder verschieden, ein Rest ausgewählt aus der Gruppe bestehend aus Wasserstoff, Methyl, CF₃, OH, Methyloxy, Benzyloxy, COOH, COOCH₃ oder Fluor;
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Erfindungsgemäß bevorzugt sind Arzneimittelkombinationen, die Verbindungen der Formel **1'** enthalten, worin R^{a} und R^{b} die vorstehend genannten Bedeutungen haben können und worin
- V: eine zweibindige Gruppe ausgewählt aus der Gruppe bestehend aus CH₂ und O, bevorzugt O;
- R¹ und R^{1'}: gleich oder verschieden Wasserstoff Methyl, Ethyl, Propyl oder Cyclopropyl, oder
- R¹ und R^{1'}: gemeinsam -CH₂-CH₂-CH₂-CH₂-CH₂-;
- R² und R^{2"'}: Wasserstoff;
- R^{2'} und R^{2"}: gleich oder verschieden, ein Rest ausgewählt aus der Gruppe bestehend aus Wasserstoff, Methyl, CF₃, OH, Methyloxy, Benzyloxy oder Fluor;
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß gleichrangiger Bedeutung sind ferner Arzneimittelkombinationen, die Verbindungen der Formel **1'** enthalten, worin R^{a} und R^{b} die vorstehend genannten Bedeutungen haben können und worin
- V: die zweibindige Gruppe O;
- R¹ und R^{1'}: gleich oder verschieden, bevorzugt gleich Wasserstoff, Methyl, Ethyl oder Propyl;
- R², R^{2"} und R^{2"}: Wasserstoff;
- R^{2'}: Wasserstoff, OH, Methyloxy oder Benzyloxy,
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß herausragender Bedeutung sind ferner Arzneimittelkombinationen, die Verbindungen der Formel **1'** enthalten, worin R^{a} und R^{b} die vorstehend genannten Bedeutungen haben können und worin
- V: die zweibindige Gruppe O;
- R¹ und R^{1'}: jeweils gleichzeitig Wasserstoff, Methyl, Ethyl oder Propyl, sind;
- R², R^{2"} und R^{2"'}: Wasserstoff;
- R^{2'}: Wasserstoff, OH oder Methyloxy,
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß besonderer Bedeutung sind ferner Arzneimittelkombinationen, die Verbindungen der Formel **1'** enthalten, in denen R^{a} und R^{b} beide Methyl bedeuten. Diese Verbindungen sind darstellbar durch die Formel **1.1'** 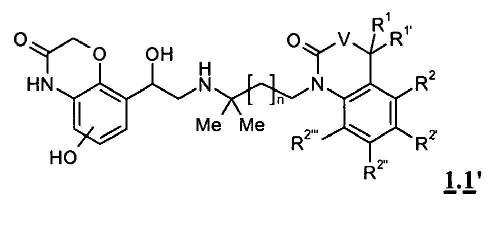 worin die Gruppen R¹, R^{1'}, R², R^{2'}, R^{2"}, R^{2"'} und V die vorstehend genannten Bedeutungen haben können.

Bevorzugt sind Arzneimittelkombinationen, die Verbindungen der Formel **1** enthalten, worin X der Gruppe -O- entspricht. Diese Verbindungen sind darstellbar durch die Formel **1"** 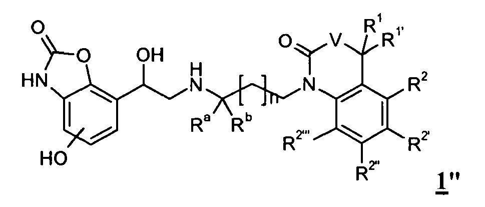 worin die Gruppen R^{a}, R^{b}, R¹, R^{1'}, R², R^{2'}, R^{2"}, R^{2"'} und V die oben stehende Bedeutung haben.

Besonders bevorzugt sind Arzneimittelkombinationen, die Verbindungen der Formel **1"** enthalten, worin R^{a} und R^{b} die vorstehend genannten Bedeutungen haben können und worin
- V: eine zweibindige Gruppe ausgewählt aus der Gruppe bestehend aus CH₂ und O, bevorzugt O;
- R¹ und R^{1'}: gleich oder verschieden, ein Rest ausgewählt aus der Gruppe bestehend aus Wasserstoff, Methyl, Ethyl, Propyl, Cyclopropyl, oder
- R¹ und R^{1'}: gemeinsam -CH₂-CH₂-CH₂-CH₂- oder -CH₂-CH₂-CH₂-CH₂-CH₂-;
- R² und R^{2"'}: Wasserstoff;
- R^{2'} und R^{2"}: gleich oder verschieden, ein Rest ausgewählt aus der Gruppe bestehend aus Wasserstoff, Methyl, CF₃, OH, Methyloxy, Benzyloxy, COOH, COOCH₃ oder Fluor;
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugt sind ferner Arzneimittelkombinationen, die Verbindungen der Formel **1"** enthalten, worin R^{a} und R^{b} die vorstehend genannten Bedeutungen haben können und worin
- V: eine zweibindige Gruppe ausgewählt aus der Gruppe bestehend aus CH₂ und O, bevorzugt O;
- R¹ und R^{1'}: gleich oder verschieden Wasserstoff Methyl, Ethyl oder Propyl, oder
- R¹ und R^{1'}: gemeinsam -CH₂-CH₂-CH₂-CH₂-CH₂-;
- R² und R^{2"'}: Wasserstoff;
- R^{2'} und R^{2"}: gleich oder verschieden, ein Rest ausgewählt aus der Gruppe bestehend aus Wasserstoff, Methyl, CF₃, OH, Methyloxy, Benzyloxy oder Fluor;
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß gleichrangiger Bedeutung sind ferner Arzneimittelkombinationen, die Verbindungen der Formel **1"** enthalten, worin R^{a} und R^{b} die vorstehend genannten Bedeutungen haben können und worin
- V: die zweibindige Gruppe O;
- R¹ und R^{1'}: gleich oder verschieden, bevorzugt gleich Wasserstoff, Methyl oder Ethyl;
- R², R^{2"} und R^{2"}: Wasserstoff;
- R^{2'}: Wasserstoff, OH, Methyloxy oder Benzyloxy,
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß besonderer Bedeutung sind ferner solche Arzneimittelkombinationen, die Verbindungen der Formel **1"** enthalten, in denen R^{a} und R^{b} beide Methyl bedeuten. Diese Verbindungen sind darstellbar durch die Formel **1.1"** 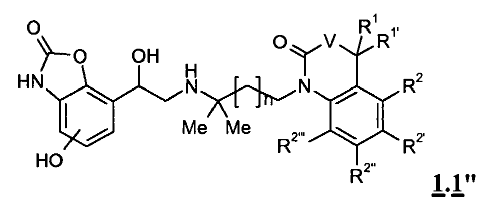 worin die Gruppen R¹, R^{1'}, R², R^{2'}, R^{2"}, R^{2"'} und V die vorstehend genannten Bedeutungen haben können.

Von erfindungsgemäß besonderer Bedeutung sind ferner Arzneimittelkombinationen, die Verbindungen der Formel **1** enthalten, worin X der Gruppe -CH=CH- entspricht. Diese Verbindungen sind darstellbar durch die Formel **1"'** 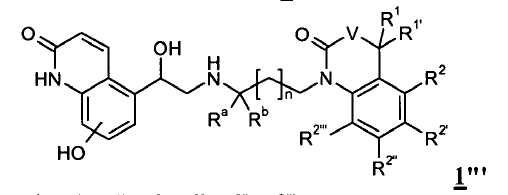 worin die Gruppen R^{a}, R^{b}, R¹, R^{1'}, R², R^{2'}, R^{2"}, R^{2"'} und V die oben stehende Bedeutung haben.

Bevorzugt sind Arzneimittelkombinationen, die Verbindungen der Formel **1"'** enthalten, worin R^{a} und R^{b} die vorstehend genannten Bedeutungen haben können und worin
- V: eine zweibindige Gruppe ausgewählt aus der Gruppe bestehend aus CH₂ und O, bevorzugt O;
- R¹ und R^{1'}: gleich oder verschieden, ein Rest ausgewählt aus der Gruppe bestehend aus Wasserstoff, Methyl, Ethyl, Propyl, Cyclopropyl, bevorzugt Methyl oder Ethyl, oder
- R¹ und R^{1'}: gemeinsam -CH₂-CH₂-, -CH₂-CH₂-CH₂-CH₂- oder -CH₂-CH₂-CH₂-CH₂-CH₂-;
- R² und R^{2"'}: Wasserstoff;
- R^{2'} und R^{2"}: gleich oder verschieden, ein Rest ausgewählt aus der Gruppe bestehend aus Wasserstoff, Methyl, CF₃, OH, Methyloxy, Benzyloxy, COOH, COOCH₃ oder Fluor;
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugt sind Arzneimittelkombinationen, die Verbindungen der Formel **1"'** enthalten, worin R^{a} und R^{b} die vorstehend genannten Bedeutungen haben können und worin
- V: eine zweibindige Gruppe ausgewählt aus der Gruppe bestehend aus CH₂ und O, bevorzugt O;
- R¹ und R^{1'}: gleich oder verschieden Wasserstoff Methyl, Ethyl oder Propyl, oder
- R¹ und R^{1'}: gemeinsam -CH₂-CH₂-, -CH₂-CH₂-CH₂-CH₂- oder -CH₂-CH₂-CH₂-CH₂-CH₂-;
- R² und R^{2"'}: Wasserstoff;
- R^{2'} und R^{2"}: gleich oder verschieden, ein Rest ausgewählt aus der Gruppe bestehend aus Wasserstoff, Methyl, CF₃, OH, Methyloxy, Benzyloxy oder Fluor;
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugt sind ferner Arzneimittelkombinationen, die Verbindungen der Formel **1"'** enthalten, worin R^{a} und R^{b} die vorstehend genannten Bedeutungen haben können und worin
- V: die zweibindige Gruppe O;
- R¹ und R^{1'}: gleich oder verschieden, bevorzugt gleich Wasserstoff, Methyl, Ethyl oder Propyl;
- R², R^{2"} und R^{2"}: Wasserstoff;
- R^{2'}: Wasserstoff, OH, Methyloxy oder Benzyloxy,
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß herausragender Bedeutung sind ferner Arzneimittelkombinationen, die Verbindungen der Formel **1"'** enthalten, worin R^{a} und R^{b} die vorstehend genannten Bedeutungen haben können und worin
- V: die zweibindige Gruppe O;
- R¹ und R^{1'}: jeweils gleichzeitig Wasserstoff, Methyl, Ethyl oder Propyl sind;
- R², R^{2"} und R^{2"'}: Wasserstoff;
- R^{2'}: Wasserstoff, OH oder Methyloxy,
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß besonderer Bedeutung sind ferner solche Arzneimittelkombinationen, die Verbindungen der Formel **1"'** enthalten, in denen R^{a} und R^{b} beide Methyl bedeuten. Diese Verbindungen sind darstellbar durch die Formel **1.1"'** 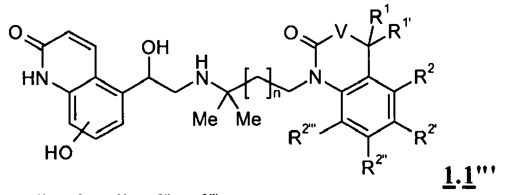 worin die Gruppen R¹, R^{1'}, R², R^{2'}, R^{2"}, R^{2"'} und V die vorstehend genannten Bedeutungen haben können.

Von erfindungsgemäß besonderer Bedeutung sind ferner Arzneimittelkombinationen, die Verbindungen der Formel **1** enthalten, worin X für die Gruppe -CMe₂-O- steht. Diese Verbindungen sind darstellbar durch die Formel **1""** 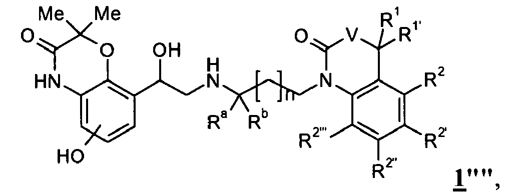 worin die Gruppen R^{a}, R^{b}, R¹, R^{1'}, R², R^{2'}, R^{2"}, R^{2"'} und V die oben stehende Bedeutung haben.

Bevorzugt sind Arzneimittelkombinationen, die Verbindungen der Formel **1""** enthalten, worin R^{a} und R^{b} die vorstehend genannten Bedeutungen haben können und worin
- V: eine zweibindige Gruppe ausgewählt aus der Gruppe bestehend aus CH₂ und O, bevorzugt O;
- R¹ und R^{1'}: gleich oder verschieden, ein Rest ausgewählt aus der Gruppe bestehend aus Wasserstoff, Methyl, Ethyl, Propyl, Cyclopropyl, oder
- R¹ und R^{1'}: gemeinsam -CH₂-CH₂-CH₂-CH₂- oder -CH₂-CH₂-CH₂-CH₂-CH₂-;
- R² und R^{2"'}: Wasserstoff;
- R^{2'} und R^{2"}: gleich oder verschieden, ein Rest ausgewählt aus der Gruppe bestehend aus Wasserstoff, Methyl, CF₃, OH, Methyloxy, Benzyloxy, COOH, COOCH₃ oder Fluor;
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugt sind Arzneimittelkombinationen, die Verbindungen der Formel **1""** enthalten, worin R^{a} und R^{b} die vorstehend genannten Bedeutungen haben können und worin
- V: eine zweibindige Gruppe ausgewählt aus der Gruppe bestehend aus CH₂ und O, bevorzugt O;
- R¹ und R^{1'}: gleich oder verschieden Wasserstoff Methyl, Ethyl oder Propyl oder
- R¹ und R^{1'}: gemeinsam -CH₂-CH₂-CH₂-CH₂-CH₂-;
- R² und R^{2"'}: Wasserstoff;
- R^{2'} und R^{2"}: gleich oder verschieden, ein Rest ausgewählt aus der Gruppe bestehend aus Wasserstoff, Methyl, CF₃, OH, Methyloxy, Benzyloxy oder Fluor;
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugt sind ferner Arzneimittelkombinationen, die Verbindungen der Formel **1""** enthalten, worin R^{a} und R^{b} die vorstehend genannten Bedeutungen haben können und worin
- V: die zweibindige Gruppe O;
- R¹ und R^{1'}: gleich oder verschieden, bevorzugt gleich Wasserstoff, Methyl, Ethyl oder Propyl;
- R², R^{2"} und R^{2"}: Wasserstoff;
- R^{2'}: Wasserstoff, OH, Methyloxy oder Benzyloxy,
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß herausragender Bedeutung sind ferner Arzneimittelkombinationen, die Verbindungen der Formel **1""** enthalten, worin R^{a} und R^{b} die vorstehend genannten Bedeutungen haben können und worin
- V: die zweibindige Gruppe O;
- R¹ und R^{1'}: jeweils gleichzeitig Wasserstoff, Methyl, Ethyl oder Propyl sind;
- R², R^{2"} und R^{2"'}: Wasserstoff;
- R^{2'}: Wasserstoff, OH oder Methyloxy,
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß besonderer Bedeutung sind ferner solche Arzneimittelkombinationen, die Verbindungen der Formel **1""** enthalten, in denen R^{a} und R^{b} beide Methyl bedeuten. Diese Verbindungen sind darstellbar durch die Formel **1.1""** 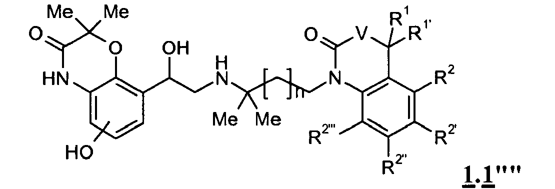 worin die Gruppen R¹, R^{1'}, R², R^{2'}, R^{2"}, R^{2"'} und V die vorstehend genannten Bedeutungen haben können.

Von erfmdungsgemäß herausragender Bedeutung sind ferner Arzneimittelkombinationen, die Verbindungen der Formel **1** enthalten, worin
- R¹ und R^{1'}: jeweils gleichzeitig Wasserstoff, Methyl oder Ethyl, bevorzugt Methyl oder Ethyl, besonders bevorzugt Ethyl sind;
und die Gruppen X, R², R^{2'}, R^{2"}, R^{2"'}, V und n eine der vorstehend genannten Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Ebenfalls von erfindungsgemäß herausragender Bedeutung sind ferner Arzneimittelkombinationen, die Verbindungen der Formel **1** enthalten, worin n 1
und die Gruppen X, R¹, R^{1'}, R², R^{2'}, R^{2"}, R^{2"'} und V eine der vorstehend genannten Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugte Arzneimittelkombinationen sind solche, die Verbindungen der Formel **1** enthalten, die ausgewählt sind aus der Gruppe bestehend aus:
- 1-{3-[2-Hydroxy-2-(6-hydroxy-3-oxo-3,4-dihydro-2H-benzo[1,4]oxazin-8-yl)-ethylamino]-3-methyl-butyl}-6-methoxy-4,4-dimethyl-1,4-dihydrobenzo[d][1,3]oxazin-2-on (**1.a**),
- 6-Hydroxy-1-{3-[2-hydroxy-2-(6-hydroxy-3-oxo-3,4-dihydro-2H-benzo[1,4]oxazin-8-yl)-ethylamino]-3-methyl-butyl}-4,4-dimethyl-1,4-dihydro-benzo[d][1,3]oxazin-2-on (**1.b**),
- 4,4-Diethyl-1-{3-[2-hydroxy-2-(6-hydroxy-3-oxo-3,4-dihydro-2H-benzo[1,4]oxazin-8-yl)-ethylamino]-3-methyl-butyl}-1,4-dihydro-benzo[d][1,3]oxazin-2-on (**1.c**),
- 6-Hydroxy-8-{1-hydroxy-2-[3-(3-hydroxy-4,4-dimethyl-1-oxo-3,4-dihydro-1H-isoquinolin-2-yl)-1,1-dimethyl-propylamino]-ethyl}-4H-benzo[1,4]oxazin-3-on (**1.d**),
- 1-{3-[2-Hydroxy-2-(6-hydroxy-3-oxo-3,4-dihydro-2H-benzo[1,4]oxazin-8-yl)-ethylamino]-3-methyl-butyl}-1,4-dihydro-benzo[1,3]oxazin-2-on (**1.e**),
- 4-Ethyl-1-{3-[2-hydroxy-2-(6-hydroxy-3-oxo-3,4-dihydro-2H-benzo[1,4]oxazin-8-yl)-ethylamino]-3-methyl-butyl}-1,4-dihydro-benzo[1,3]oxazin-2-on (**1.f**),
- 8-{2-[1,1-Dimethyl-3-(2-oxo-3,4-dihydro-2H-chinolin-1-yl)-propylamino]-1-hydroxyethyl}-6-hydroxy-4H-benzo[1,4]oxazin-3-on (**1.g**),
- 4,4-Diethyl-1-{3-[2-hydroxy-2-(8-hydroxy-2-oxo-1,2-dihydro-chinolin-5-yl)-ethylamino]-3-methyl-butyl}-1,4-dihydro-benzo[1,3]oxazin-2-on (**1.h**),
- 4,4-Diethyl-1-{3-[2-hydroxy-2-(6-hydroxy-2,2-dimethyl-3-oxo-3,4-dihydro-2H-benzo[1,4]oxazin-8-yl)-ethylamino]-3-methyl-butyl}-1,4-dihydrobenzo[d][1,3]oxazin-2-on (**1.i**),
- 4,4-Diethyl-1-{3-[2-hydroxy-2-(6-hydroxy-2,2-dimethyl-3-oxo-3,4-dihydro-2H-benzo[1,4]oxazin-8-yl)-ethylamino]-3-methyl-butyl}-1,4-dihydrobenzo[d][1,3]oxazin-2-on (**1.j**),
- 4,4-Diethyl-1-{3-[2-hydroxy-2-(7-hydroxy-2-oxo-1,2-dihydro-quinolin-5-yl)-ethylamino]-3-methyl-butyl}-1,4-dihydro-benzo[d][1,3]oxazin-2-on (**1.k**),
- 1-{3-[2-Hydroxy-2-(6-hydroxy-3-oxo-3,4-dihydro-2*H*-benzo[1,4]oxazin-8-yl)-ethylamino]-3-methyl-butyl}-4,4-dipropyl-1,4-dihydro-benzo[*d*][1,3]oxazin-2-on (**1.l**),
- 1-{3-[2-Hydroxy-2-(5-hydroxy-3-oxo-3,4-dihydro-2*H*-benzo[1,4]oxazin-8-yl)-ethylamino]-3-methyl-butyl}-4,4-dipropyl-1,4-dihydro-benzo[*d*][1,3]oxazin-2-on (**1.m**),
- 4,4-Diethyl-7-fluor-1-{3-[2-hydroxy-2-(6-hydroxy-3-oxo-3,4-dihydro-2*H* benzo [1,4]oxazin-8-yl)-ethylamino]-3-methyl-butyl}-1,4-dihydrobenzo[*d*][1,3]oxazin-2-on (**1.n**),
- 4,4-Diethyl-1-{3-[2-hydroxy-2-(5-hydroxy-3-oxo-3,4-dihydro-2*H*-benzo[1,4]oxazin-8-yl)-ethylamino]-3-methyl-butyl}-8-methoxy-1,4-dihydro-benzo[*d*][1,3]oxazin-2-on (**1.o**),
- 1-(2-{1-[2-Hydroxy-2-(5-hydroxy-3-oxo-3,4-dihydro-2H-benzo[1,4]oxazin-8-yl)-ethylamino]-cyclopropyl}-ethyl)-4,4-dimethyl-1,4-dihydro-benzo[d][1,3]oxazin-2-on (**1.p**),
- 1-(2-{1-[2-Hydroxy-2-(6-hydroxy-3-oxo-3,4-dihydro-2H-benzo[1,4]oxazin-8-yl)-ethylamino]-cyclopropyl}-ethyl)-4,4-dimethyl-1,4-dihydro-benzo[d][1,3]oxazin-2-on (**1.q**),
- 1-{3-[2-Hydroxy-2-(6-hydroxy-3-oxo-3,4-dihydro-2*H*-benzo[1,4]oxazin-8-yl)-ethylamino]-3-methyl-butyl}-spiro(cyclohexan-1,4'-2H-3',1'-benzoxazin)-2'-on (**1.r**),
- 1-{3-[2-Hydroxy-2-(5-hydroxy-3-oxo-3,4-dihydro-2*H*-benzo[1,4]oxazin-8-yl)-ethylamino]-3-methyl-butyl}-spiro(cyclohexan-1,4'-2H-3',1'-benzoxazin)-2'-on (**1.s**),
- 4,4-Dimethyl-1-{3-[2-hydroxy-2-(6-hydroxy-3-oxo-3,4-dihydro-2H-benzo[1,4]oxazin-8-yl)-ethylamino]-propyl}-1,4-dihydro-benzo[d][1,3]oxazin-2-on (**1.t**) und
- 4,4-Dimethyl-1-{3-[2-hydroxy-2-(5-hydroxy-3-oxo-3,4-dihydro-2H-benzo[1,4]oxazin-8-yl)-ethylamino]-propyl}-1,4-dihydro-benzo[d][1,3]oxazin-2-on (**1.u**),
gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren

Die OH-Gruppe kann in den vorstehend definierten Verbindungen der Formel **1** in drei unterschiedlichen Positionen konfiguriert sein. Die in den erfindungsgemäßen Kombinationen bevorzugt einsetzbaren Isomere sind durch die nachstehenden allgemeinen Formeln **1a** und **1b** darstellbar, 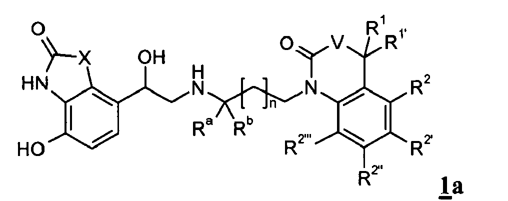 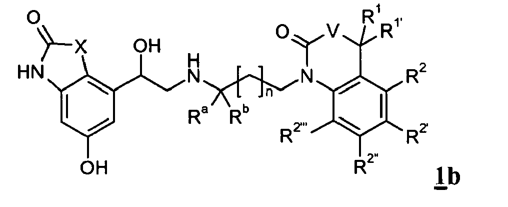 worin die Gruppen X, R^{a}, R^{b}, R¹, R^{1'}, R², R^{2'}, R^{2"}, R^{2"'}, V und n die vorstehend genannten Bedeutungen haben können.

Besonders bevorzugte Arzneimittelkombinationen sind insbesondere auch solche, die Verbindungen der Formel **1.1'-b** enthalten, 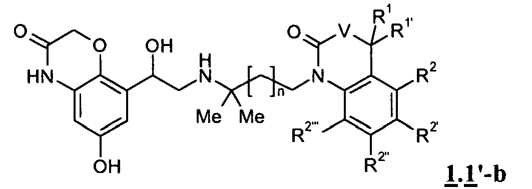 worin die Gruppen R¹, R^{1'}, R², R^{2'}, R^{2"}, R^{2"'} und V die vorstehend genannten Bedeutungen haben können.

Besonders bevorzugte Arzneimittelkombinationen sind insbesondere auch solche, die Verbindungen der Formel **1.1"-b** enthalten, 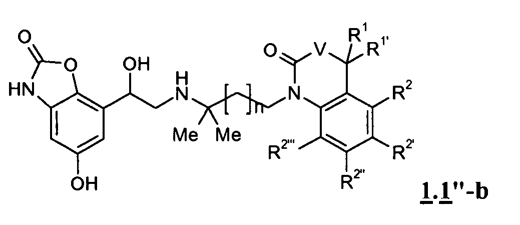 worin die Gruppen R¹, R^{1'}, R², R^{2'}, R^{2"}, R^{2"'} und V die vorstehend genannten Bedeutungen haben können.

Besonders bevorzugte Arzneimittelkombinationen sind insbesondere auch solche, die Verbindungen der Formel **1.1"'-a** enthalten, 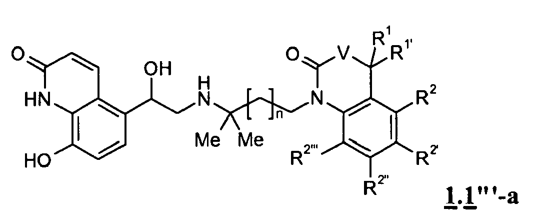 worin die Gruppen R¹, R^{1'}, R², R^{2'}, R^{2"}, R^{2"'} und V die vorstehend genannten Bedeutungen haben können.

Besonders bevorzugte Arzneimittelkombinationen sind insbesondere auch solche, die Verbindungen der Formel **1.1"'-b** enthalten, 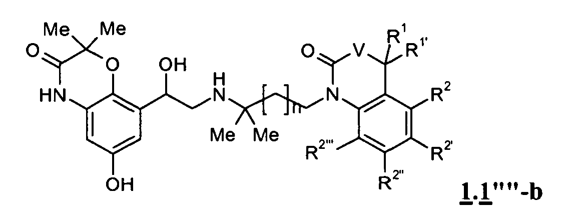 worin die Gruppen R¹, R^{1'}, R², R^{2'}, R^{2"}, R^{2"'} und V die vorstehend genannten Bedeutungen haben können.

Die Verbindungen der Formel **1** können gegebenenfalls in Form der einzelnen optischen Isomeren, Mischungen der einzelnen Enantiomeren oder Racemate zur Anwendung gelangen. Besonders bevorzugt gelangen Sie in Form der enantiomerenreinen Verbindungen zur Anwendung, wobei die Verbindungen der Formel **1**, in denen das zum Phenylring benzylische, asymmetrische Kohlenstoffzentrum "-CH(OH)-" R-konfiguriert ist. Die besonders bevorzugten R-Enantiomere der Verbindungen der allgemeinen Formel **1** sind durch die allgemeine Formel ***R*-1** darstellbar, 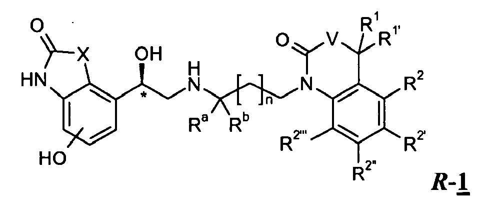 worin die Gruppen X, R^{a}, R^{b}, R¹, R^{1'}, R², R^{2'}, R^{2"}, R^{2"'}, V und n die vorstehend genannten Bedeutungen haben können.

Ein weiterer Aspekt der vorliegenden Erfindung betrifft Arzneimittelkombinationen, die vorstehend genannte Verbindungen der Formel **1** in Form der Säureadditionssalze mit pharmakologisch unbedenklichen Säuren sowie gegebenenfalls in Form der Solvate und/oder Hydrate enthalten.

Unter Säureadditionssalzen mit pharmakologisch unbedenklichen Säuren der Verbindungen **1** werden dabei beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Von den vorstehend genannten Säureadditionssalzen sind die Salze der Chlorwasserstoffsäure, der Methansulfonsäure, der Benzoesäure und der Essigsäure erfindungsgemäß besonders bevorzugt.

Bevorzugte Arzneimittelkombinationen enthalten neben ein oder mehrerer, bevorzugt einer Verbindung der Formel **1** als weiteren Wirkstoff ein oder mehrere, bevorzugt ein Anticholinergikum **2a**, gegebenenfalls in Kombination mit pharmazeutisch verträglichen Hilfsstoffen.

In den erfindungsgemäßen Arzneimittelkombinationen ist das Anticholinergikum **2a** bevorzugt ausgewählt aus der Gruppe bestehend aus Tiotropiumsalzen (**2a.1**), Oxitropiumsalzen (**2a.2**), Flutropiumsalzen (**2a.3**), Ipratropiumsalzen (**2a.4**), Glycopyrroniumsalzen (**2a.5**), Trospiumsalzen (**2a.6**) und den Verbindungen der Formeln **2a.7** bis **2a.13.**

In den vorstehend genannten Salzen **2a.1** bis **2a.6** stellen die Kationen Tiotropium, Oxitropium, Flutropium, Ipratropium, Glycopyrronium und Trospium die pharmakologisch aktiven Bestandteile dar. Eine explizite Bezugnahme auf die vorstehend genannten Kationen erfolgt durch die Bezeichnungen **2a.1'** bis **2a.6'**. Jede Bezugnahme auf die vorstehend genannten Salze **2a.1** bis **2a.6** schließt naturgemäß eine Bezugnahme auf die entsprechenden Kationen Tiotropium (**2a.1'**), Oxitropium (**2a.2'**), Flutropium (**2a.3'**), Ipratropium (**2a.4'**), Glycopyrronium (**2a.5'**), Trospium (**2a.6'**) mit ein.

Unter den Salzen **2a.1** bis **2a.6** werden erfindungsgemäß diejenigen Verbindungen verstanden, die neben den Kationen Tiotropium (**2a.1'**), Oxitropium (**2a.2'**), Flutropium (**2a.3'**), Ipratropium (**2a.4'**), Glycopyrronium (**2a.5'**) und Trospium (**2a.6'**) als Gegenion (Anion) Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat enthalten, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Im Fall der Trospiumsalze (**2a.6**) ist das Chlorid besonders bevorzugt. Bei den anderen Salzen **2a.1** bis **2a.5** sind die Methansulfonate und Bromide von besonderer Bedeutung. Von besonderer Bedeutung sind Arzneimittelkombinationen, die Tiotropiumsalze (**2a.1**), Oxitropiumsalze (**2a.2**) oder Ipratropiumsalze (**2a.4**) enthalten, wobei die jeweiligen Bromide erfindungsgemäß besonders bedeutsam sind. Von besonderer Bedeutung ist das Tiotropiumbromid (**2a.1**). Die vorstehend genannten Salze können in den erfindungsgemäßen Arzneimittelkombinationen gegebenenfalls in Form ihrer Solvate oder Hydrate, bevorzugt in Form ihrer Hydrate vorliegen. Im Falle des Tiotropiumbromids enthalten die erfindungsgemäßen Arzneimittelkombinationen dieses bevorzugt in Form des kristallinen Tiotropiumbromid Monohydrats, welches aus der WO 02/30928 bekannt ist. Wird das Tiotropiumbromid in den erfindungsgemäßen Arzneimittelkombinationen in wasserfreier Form eingesetzt, so gelangt bevorzugt das wasserfreie kristalline Tiotropiumbromid zur Anwendung, welches aus der WO 03/000265 bekannt ist.

Beispiele für erfindungsgemäße bevorzugte Arzneimittelkombinationen bevorzugter Verbindungen der Formel **1** mit den vorstehend genannten Anticholinergika **2a.1** bis **2a.6** sind Kombinationen enthaltend die Verbindungen **1.a** und **2a.1**; **1.a** und **2a.2**; **1.a** und **2a.3**; **1.a** und **2a.4**; **1.a** und **2a.5**; **1.a** und **2a.6**; **1.b** und **2a.1**; **1.b** und **2a.2**; **1.b** und **2a.3**; **1.b** und **2a.4**; **1.b** und **2a.5**; **1.b** und **2a.6**; **1.d** und **2a.1**; **1.d** und **2a.2**; **1.d** und **2a.3**; **1.d** und **2a.4**; **1.d** und **2a.5**; **1.d** und **2a.6**; **1.f** und **2a.1**; **1.f** und **2a.2**; **1.f** und **2a.3**; **1.f** und **2a.4**; **1.f** und **2a.5**; **1.f** und **2a.6**; **1.h** und **2a.1**; **1.h** und **2a.2**; **1.h** und **2a.3**; **1.h** und **2a.4**; **1.h** und **2a.5**; **1.h** und **2a.6**; **1.j** und **2a.1**; **1.j** und **2a.2**; **1.j** und **2a.3**; **1.j** und **2a.4**; **1.j** und **2a.5**; **1.j** und **2a.6**; **1.k** und **2a.1**; **1.k** und **2a.2**; **1.k** und **2a.3**; **1.k** und **2a.4**; **1.k** und **2a.5**; **1.k** und **2a.6**; **1.l** und **2a.1**; **1.l** und **2a.2**; **1.l** und **2a.3**; **1.l** und **2a.4**; **1.l** und **2a.5**; **1.l** und **2a.6**; **1.m** und **2a.1**; **1.m** und **2a.2**; **1.m** und **2a.3**; **1.m** und **2a.4**; **1.m** und **2a.5**; **1.m** und **2a.6**; **1.q** und **2a.1**; **1.q** und **2a.2**; **1.q** und **2a.3**; **1.q** und **2a.4**; **1.q** und **2a.5** oder **1.q** und **2a.6** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Von den vorstehend genannten Kombinationen sind erfindungsgemäß diejenigen bevorzugt, die als Verbindung der Formel **1** eine der Verbindungen **1.a**, **1.h**, **1.j**, oder **1.k** enthalten, wobei jene Kombinationen, die eine der Verbindungen **1.h** oder **1.k** enthalten, erfindungsgemäß besonders bedeutsam sind. Von den vorstehend genannten Kombinationen sind erfindungsgemäß ferner diejenigen bevorzugt, die als Verbindung **2a** eine der Verbindungen **2a.1**, **2a.2** oder **2a.4** enthalten, wobei jene Kombinationen, die die Verbindung **2a.1** enthalten, erfindungsgemäß besonders bedeutsam sind.

Gegebenenfalls weisen die vorstehend genannten Anticholinergika chirale Kohlenstoffzentren auf. In diesem Fall können die erfindungsgemäßen Arzneimittelkombinationen die Anticholinergika in Form ihrer Enantiomere, Mischungen der Enantiomere oder Racemate enthalten, wobei vorzugsweise enantiomerenreine Anticholinergika zum Einsatz gelangen.

In einer ebenfalls bevorzugten Ausführungsform der vorliegenden Erfindung sind die in den erfindungsgemäßen Arzneimittelkombinationen enthaltenen Anticholinergika **2a** ausgewählt aus den Salzen der Formel **2a.7** 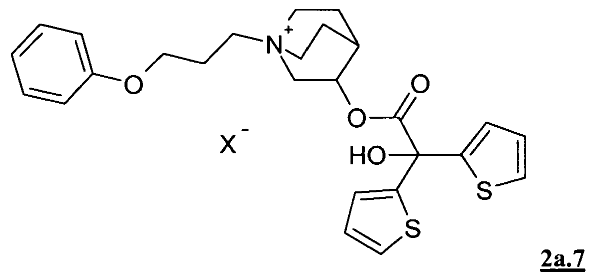 worin
- X ⁻: ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bedeutet
gegebenenfalls in Form ihrer Racemate, Enantiomere oder Hydrate.

Bevorzugte Arzneimittelkombinationen enthalten Salze der Formel **2a.7**, worin
- X ⁻: ein einfach negativ geladenes Anion, bevorzugt ein Anion ausgewählt aus der Gruppe bestehend aus Fluorid, Chlorid, Bromid, Methansulfonat und p-Toluolsulfonat, bevorzugt Bromid, bedeuten,
gegebenenfalls in Form ihrer Racemate, Enantiomere oder Hydrate.

Bevorzugte Arzneimittelkombinationen enthalten Salze der Formel **2a.7**, worin
- X ⁻: ein einfach negativ geladenes Anion, bevorzugt ein Anion ausgewählt aus der Gruppe bestehend aus Chlorid, Bromid und Methansulfonat, bevorzugt Bromid, bedeuten,
gegebenenfalls in Form ihrer Racemate, Enantiomere oder Hydrate.

Besonders bevorzugte Arzneimittelkombinationen enthalten die Verbindung der Formel **2a.7** in Form des Bromids.

Von besonderer Bedeutung sind solche Arzneimittelkombinationen, die die Enantiomere der Formel **2a.7-en** 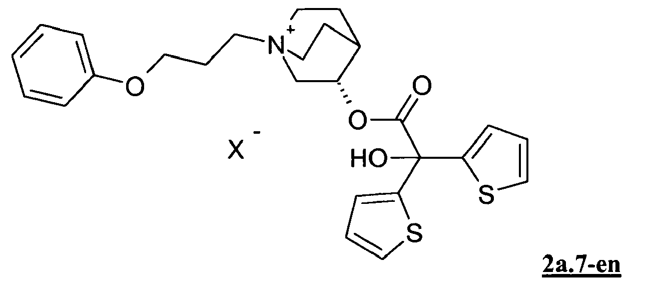 enthalten, worin X ⁻ die vorstehend genannten Bedeutungen aufweisen kann.

Beispiele für erfindungsgemäße Arzneimittelkombinationen bevorzugter Verbindungen der Formel **1** mit den vorstehend genannten Anticholinergika **2a.7** sind Kombinationen enthaltend die Verbindungen **1.a** und **2a.7**; **1.a** und **2a.7-en**; **1.b** und **2a.7**; **1.b** und **2a.7-en**; **1.d** und **2a.7**; **1.d** und **2a.7-en**; **1.f** und **2a.7**; **1.f** und **2a.7-en**; **1.h** und **2a.7**; **1.h** und **2a.7-en**; **1.j** und **2a.7**; **1.j** und **2a.7-en**; **1.k** und **2a.7**; **1.k** und **2a.7-en**; **1.l** und **2a.7**; **1.l** und **2a.7-en**; **1.m** und **2a.7**; **1.m** und **2a.7-en**; **1.q** und **2a.7**; **1.q** und **2a.7-en**, jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Von den vorstehend genannten Kombinationen sind erfindungsgemäß diejenigen bevorzugt, die als Verbindung der Formel **1** eine der Verbindungen **1.a**, **1.h**, **1.j**, oder **1.k** enthalten, wobei jene Kombinationen, die eine der Verbindungen **1.h** oder **1.k** enthalten, erfindungsgemäß besonders bedeutsam sind. Von den vorstehend genannten Kombinationen sind erfindungsgemäß ferner diejenigen bevorzugt, die als Verbindung **2a** die Verbindung **2a.7-en** enthalten.

In einer weiteren bevorzugten Ausführungsform der vorliegenden Erfindung sind die in den erfindungsgemäßen Arzneimittelkombinationen enthaltenen Anticholinergika **2a** ausgewählt aus den Salzen der Formel **2a.8** 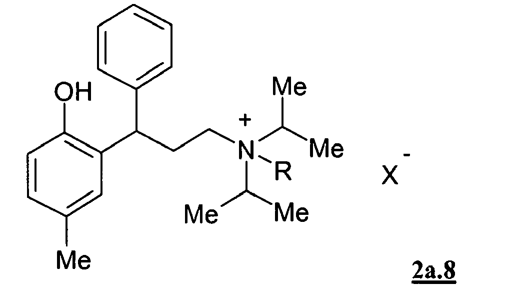 worin R entweder Methyl (**2a.8.1**) oder Ethyl (**2a.8.2**) bedeuten und worin X ⁻ die vorstehend genannten Bedeutungen aufweisen kann. In einer alternativen Ausführungsform ist die Verbindung der Formel **2a.8** in Form der freien Base **2a.8-base** 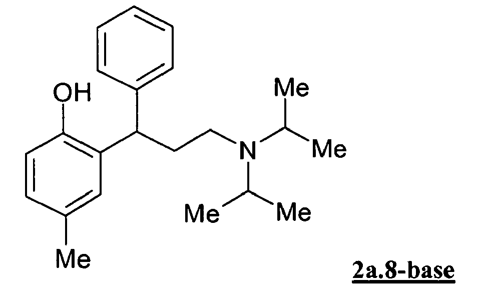 enthalten.

Die erfindungsgemäßen Arzneimittelkombinationen können das Anticholinergikum der Formel **2a.8** (oder **2a.8-base**) in Form ihrer Enantiomere, Mischungen der Enantiomere oder Racemate enthalten. Vorzugsweise sind die Anticholinergika der Formel **2a.8** (or **2a.8-base**) in Form ihrer R-Enantiomere enthalten.

Beispiele für erfindungsgemäße Arzneimittelkombinationen bevorzugter Verbindungen der Formel **1** mit den vorstehend genannten Anticholinergika **2a.8** sind Kombinationen enthaltend die Verbindungen **1.a** und **2a.8.1**; **1.a** und **2a.8.2**; **1.b** und **2a.8.1**; **1.b** und **2a.8.2**; **1.d** und **2a.8.1**; **1.d** und **2a.8.2**; **1.f** und *2***a.8.1**; **1.f** und **2a.8.2**; **1.h** und **2a.8.1**; **1.h** und **2a.8.2**; **1.j** und **2a.8.1**; **1.j** und **2a.8.2**; **1.k** und **2a.8.1**; **1.k** und **2a.8.2**; **1.1** und **2a.8.1**; **1.l** und **2a.8.2**; **1.m** und **2a.8.1**; **1.m** und **2a.8.2**; **1.q** und **2a.8.1**; **1.q** und **2a.8**.2, jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Von den vorstehend genannten Kombinationen sind erfindungsgemäß diejenigen bevorzugt, die als Verbindung der Formel **1** eine der Verbindungen **1.a**, **1.h**, **1.j**, oder **1.k** enthalten, wobei jene Kombinationen, die eine der Verbindungen **1.h** oder **1.k** enthalten, erfindungsgemäß besonders bedeutsam sind.

In einer weiteren bevorzugten Ausführungsform der vorliegenden Erfindung sind die in den erfindungsgemäßen Arzneimittelkombinationen enthaltenen Anticholinergika **2a** ausgewählt aus den Verbindungen der Formel **2a.9** 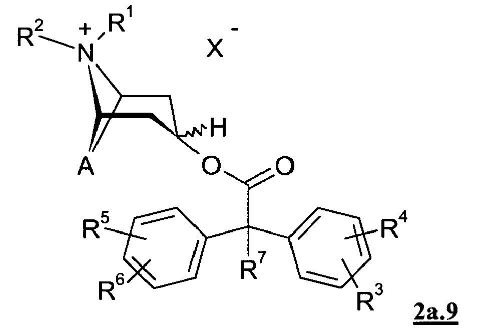 worin
- A: eine zweibindige Gruppe ausgewählt aus den Gruppen 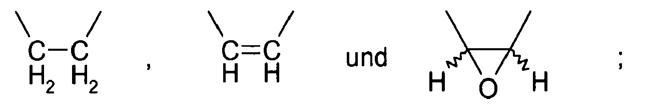
- X ⁻: eine der vorstehend genannten einfach negativ geladenen Anionen, bevorzugt Chlorid, Bromid oder Methansulfonat,
- R¹ und R²: gleich oder verschieden eine Gruppe ausgewählt aus Methyl, Ethyl, n-Propyl und iso-Propyl, die gegebenenfalls substituiert sein kann durch Hydroxy oder Fluor, bevorzugt unsubstitutiertes Methyl;
- R³, R⁴, R⁵ und R⁶,: gleich oder verschieden Wasserstoff, Methyl, Ethyl, Methyloxy, Ethyloxy, Hydroxy, Fluor, Chlor, Brom, CN, CF₃ oder NO₂;
- R⁷: Wasserstoff, Methyl, Ethyl, Methyloxy, Ethyloxy, -CH₂-F, -CH₂-CH₂-F, -O-CH₂-F, -O-CH₂-CH₂-F, -CH₂-OH, -CH₂-CH₂-OH, CF₃, -CH₂-OMe, -CH₂-CH₂-OMe, -CH₂-OEt, -CH₂-CH₂-OEt, -O-COMe, -O-COEt, -O-COCF₃, -O-COCF₃, Fluor, Chlor oder Brom, bedeuten.

Die Verbindungen der Formel **2a.9** sind im Stand der Technik bekannt (WO 02/32899).

Im Rahmen der erfindungsgemäßen Arzneimittelkombinationen bevorzugte Verbindungen der Formel **2a.9** sind solche, in denen
- X⁻: Bromid;
- R¹ und R²: gleich oder verschieden, Methyl oder Ethyl, bevorzugt Methyl;
- R³, R⁴, R⁵ und R⁶,: gleich oder verschieden, Wasserstoff, Methyl, Methyloxy, Chlor oder Fluor;
- R⁷: Wasserstoff, Methyl oder Fluor, bedeuten.

Von besonderer Bedeutung sind Arzneimittelkombinationen, die solche Verbindungen der Formel **2a.9** enthalten, in denen
- A: eine zweibindige Gruppe ausgewählt aus 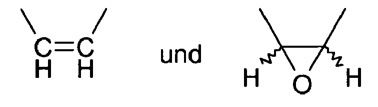
bedeutet.

Von besonderer Bedeutung sind diejenigen Arzneimittelkombinationen, die neben einer Verbindung der Formel **1** eine der nachfolgenden Verbindungen der Formel **2a.9** enthalten:
- 2,2-Diphenylpropionsäuretropenolester-methobromid **(2a.9.1),**
- 2,2-Diphenylpropionsäurescopinester-methobromid **(2a.9.2),**
- 2-Fluor-2,2-Diphenylessigsäurescopinester-methobromid **(2a.9.3),**
- 2-Fluor-2,2-Diphenylessigsäuretropenolester-methobromid **(2a.9.4),;**

Die Verbindungen der Formel **2a.9** können gegebenenfalls in Form ihrer Enantiomere, Mischungen ihrer Enantiomere oder Racemate, sowie gegebenenfalls in Form ihrer Hydrate und/oder Solvate enthalten sein.

Beispiele für erfindungsgemäße Arzneimittelkombinationen bevorzugter Verbindungen der Formel **1** mit den vorstehend genannten Anticholinergika **2a.9** sind Kombinationen enthaltend die Verbindungen **1.a** und **2a.9.1; 1.a** und **2a.9.2; 1.a** und **2a.9.3; 1.a** und **2a.9.4; 1.b** und **2a.9.1; 1.b** und **2a.9.2; 1.b** und **2a.9.3; 1.b** und **2a.9.4; 1.d** und **2a.9.1.; 1.d** und **2a.9.2; 1.d** und **2a.9.3; 1.d** und **2a.9.4; 1.f** und **2a.9.1; 1.f** und **2a.9.2; 1.f** und **2a.9.3; 1.f** und **2a.9.4; 1.h** und **2a.9.1. 1.h** und **2a.9.2; 1.h** und **2a.9.3; 1.h** und **2a.9.4; 1.i** und **2a.9.1; 1.j** und **2a.9.2; 1.j** und **2a.9.3; 1.j** und **2a.9.4; 1.k** und **2a.9.1, 1.k** und **2a.9.2; 1.k** und **2a.9.3; 1.k** und **2a.9.4; 1.1** und **2a.9.1; 1.l** und **2a.9.2; 1.l** und **2a.9.3; 1.l** und **2a.9.4; 1.m** und **2a.9.1; 1.m** und **2a.9.2; 1.m** und **2a.9.3; 1.m** und **2a.9.4; 1.q** und **2a.9.1; 1.q** und **2a.9.2; 1.q** und **2a.9.3; 1.q** und **2a.9.4,** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Von den vorstehend genannten Kombinationen sind erfindungsgemäß diejenigen bevorzugt, die als Verbindung der Formel **1** eine der Verbindungen **1.a, 1.h, 1.j,** oder **1.k** enthalten, wobei jene Kombinationen, die eine der Verbindungen **1.h** oder **1.k** enthalten, erfindungsgemäß besonders bedeutsam sind. Von den vorstehend genannten Kombinationen sind erfindungsgemäß ferner diejenigen bevorzugt, die als Verbindung **2a.9** eine der Verbindungen **2a.9.1** oder **2a.9.2** enthalten, wobei jene Kombinationen, die die Verbindung **2a.9.2** enthalten, erfindungsgemäß besonders bedeutsam sind.

In einer weiteren bevorzugten Ausführungsform der vorliegenden Erfindung sind die in den erfindungsgemäßen Arzneimittelkombinationen enthaltenen Anticholinergika **2a** ausgewählt aus den Verbindungen der Formel **2a.10** 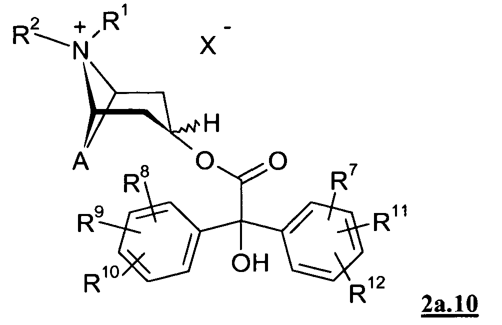 worin
A, X⁻, R¹ und R² die vorstehend genannten Bedeutungen haben können und worin R⁷, R⁸, R⁹, R¹⁰, R¹¹ und R¹², gleich oder verschieden, Wasserstoff, Methyl, Ethyl, Methyloxy, Ethyloxy, Hydroxy, Fluor, Chlor, Brom, CN, CF₃ oder NO₂, bedeuten, wobei zumindest eine der Gruppen R⁷, R⁸, R⁹, R¹⁰, R¹¹ und R¹² nicht Wasserstoff sein kann.

Die Verbindungen der Formel **2a.10** sind im Stand der Technik bekannt (WO 02/32898).

Im Rahmen der erfindungsgemäßen Arzneimittelkombinationen bevorzugte Verbindungen der Formel **2a.10** sind solche, in denen
- A: eine zweibindige Gruppe ausgewählt aus 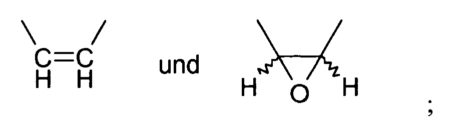
- X⁻: Bromid;
- R¹ und R²: gleich oder verschieden, Methyl oder Ethyl, bevorzugt Methyl;
- R⁷, R⁸, R⁹, R¹⁰, R¹¹ und R¹²,: gleich oder verschieden Wasserstoff, Fluor, Chlor oder Brom, bevorzugt Fluor, bedeuten, wobei zumindest eine der Gruppen R⁷, R⁸, R⁹, R¹⁰, R¹¹ und R¹² nicht Wasserstoff sein kann.

Von besonderer Bedeutung sind diejenigen Arzneimittelkombinationen, die neben einer Verbindung der Formel **1** eine der nachfolgenden Verbindungen der Formel **2a.10** enthalten:
- 3,3',4,4'-Tetrafluorbenzilsäuretropenolester-Methobromid **(2a.10.1),**
- 3,3',4,4'-Tetrafluorbenzilsäurescopinester-Methobromid **(2a.10.2),**
- 4,4'-Difluorbenzilsäuretropenolester-Methobromid **(2a.10.3),**
- 4,4'-Difluorbenzilsäurescopinester-Methobromid **(2a.10.4),**
- 3,3'-Difluorbenzilsäuretropenolester-Methobromid **(2a.10.5),**
- 3,3'-Difluorbenzilsäurescopinester-Methobromid **(2a.10.6).**

Die Verbindungen der Formel **2a.10** können gegebenenfalls in Form ihrer Enantiomere, Mischungen ihrer Enantiomere oder Racemate, sowie gegebenenfalls in Form ihrer Hydrate und/oder Solvate enthalten sein.

Beispiele für erfindungsgemäße Arzneimittelkombinationen bevorzugter Verbindungen der Formel **1** mit den vorstehend genannten Anticholinergika **2a.10** sind Kombinationen enthaltend die Verbindungen **1.a** und **2a.10.1; 1.a** und **2a.10.2; 1.a** und **2a.10.3; 1.a** und **2a.10.4; 1.a** und **2a.10.5; 1.a** und **2a.10.6; 1.b** und **2a.10.1; 1.b** und **2a.10.2; 1.b** und **2a.10.3; 1.b** und **2a.10.4; 1.b** und **2a.10.5, 1.b** und **2a.10.6; 1.d** und **2a.10.1; 1.d** und **2a.10.2; 1.d** und **2a.10.3; 1.d** und **2a.10.4; 1.d** und **2a.10.5; 1.d** und **2a.10.6; 1.f** und **2a.10.1; 1.f** und **2a.10.2; 1.f** und **2a.10.3; 1.f** und **2a.10.4; 1.f** und **2a.10.5; 1.f** und **2a.10.6; 1.h** und **2a.10.1; 1.h** und **2a.10.2; 1.h** und **2a.10.3; 1.h** und **2a.10.4; 1.h** und **2a.10.5; 1.h** und **2a.10.6; 1.j** und **2a.10.1; 1.j** und **2a.10.2; 1.j** und **2a.10.3; 1.j** und **2a.10.4; 1.j** und **2a.10.5; 1.j** und **2a.10.6; 1.k** und **2a.10.1; 1.k** und **2a.10.2; 1.k** und **2a.10.3; 1.k** und **2a.10.4; 1.k** und **2a.10.5; 1.k** und **2a.10.6; 1.l** und **2a.10.1; 1.l** und **2a.10.2; 1.l** und **2a.10.3; 1.l** und **2a.10.4; 1.l** und **2a.10.5; 1.l** und **2a.10.6; 1.m** und **2a.10.1; 1.m** und **2a.10.2; 1.m** und **2a.10.3; 1.m** und **2a.10.4; 1.m** und **2a.10.5; 1.m** und **2a.10.6; 1.q** und **2a.10.1; 1.q** und **2a.10.2; 1.q** und **2a.10.3; 1.q** und **2a.10.4; 1.q** und **2a.10.5. 1.q** und **2a.10.6,** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Von den vorstehend genannten Kombinationen sind erfindungsgemäß diejenigen bevorzugt, die als Verbindung der Formel **1** eine der Verbindungen **1.a, 1.h, 1.j,** oder **1.k** enthalten, wobei jene Kombinationen, die eine der Verbindungen **1.h** oder **1.k** enthalten, erfindungsgemäß besonders bedeutsam sind. Von den vorstehend genannten Kombinationen sind erfindungsgemäß ferner diejenigen bevorzugt, die als Verbindung **2a.10** eine der Verbindungen **2a.10.1, 2a.10.2, 2a.10.3** oder **2a.10.4** enthalten, wobei jene Kombinationen, die die Verbindungen **2a.10.1** oder **2a.10.2** enthalten, erfindungsgemäß besonders bedeutsam sind.

In einer weiteren bevorzugten Ausführungsform der vorliegenden Erfindung sind die in den erfindungsgemäßen Arzneimittelkombinationen enthaltenen Anticholinergika **2a** ausgewählt aus den Verbindungen der Formel **2a.11** 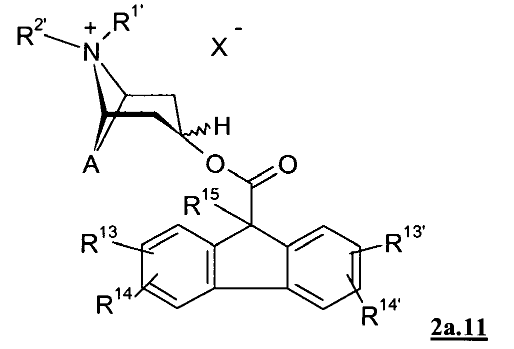 worin
- A und X ⁻: die vorstehend genannten Bedeutungen haben können und worin
- R¹⁵: Wasserstoff, Hydroxy, Methyl, Ethyl, -CF₃, CHF₂ oder Fluor;
- R^{1'} und R^{2'}: gleich oder verschieden, C₁-C₅-Alkyl, welches gegebenenfalls substituiert sein kann durch C₃-C₆-Cycloalkyl, Hydroxy oder Halogen, oder R^{1'} und R^{2'} gemeinsam eine -C₃-C₅-Alkylenbrücke;
- R¹³, R¹⁴, R^{13'} und R^{14'}: gleich oder verschieden, Wasserstoff, -C₁-C₄-Alkyl, -C₁-C₄-Alkyloxy, Hydroxy, -CF₃, -CHF₂, CN, NO₂ oder Halogen,
bedeuten.

Die Verbindungen der Formel **2a.11** sind im Stand der Technik bekannt (WO 03/064419).

Im Rahmen der erfindungsgemäßen Arzneimittelkombinationen bevorzugte Verbindungen der Formel **2a.11** sind solche, in denen
- A: eine zweibindige Gruppe ausgewählt aus

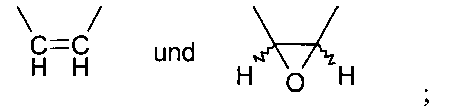

- X⁻: ein Anion ausgewählt aus Chlorid, Bromid und Methansulfonat, bevorzugt Bromid;
- R¹⁵: Hydroxy, Methyl oder Fluor, bevorzugt Methyl oder Hydroxy;
- R^{1'} und R^{2'}: gleich oder verschieden, Methyl oder Ethyl, bevorzugt Methyl;
- R¹³, R¹⁴, R^{13'} und R^{14'}: gleich oder verschieden, Wasserstoff, -CF₃, -CHF₂ oder Fluor, bevorzugt Wasserstoff oder Fluor, bedeuten.

Im Rahmen der erfindungsgemäßen Arzneimittelkombinationen besonders bevorzugte Verbindungen der Formel **2a.11** sind solche, in denen
- A: eine zweibindige Gruppe ausgewählt aus 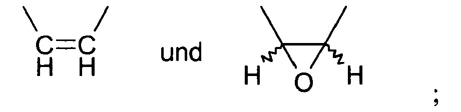
- X ⁻: Bromid;
- R¹⁵: Hydroxy oder Methyl, bevorzugt Methyl;
- R^{1'} und R^{2'}: gleich oder verschieden, Methyl oder Ethyl, bevorzugt Methyl;
- R¹³, R¹⁴, R^{13'} und R^{14'}: gleich oder verschieden, Wasserstoff oder Fluor, bedeuten.

Von besonderer Bedeutung sind diejenigen Arzneimittelkombinationen, die neben einer Verbindung der Formel **1** eine der nachfolgenden Verbindungen der Formel **2a.11** enthalten:
- 9-Hydroxy-fluoren-9-carbonsäuretropenolester -Methobromid **(2a.11.1);**
- 9-Fluor-fluoren-9-carbonsäuretropenolester-Methobromid **(2a.11.2)** ;
- 9-Hydroxy-fluoren-9-carbonsäurescopinester-Methobromid **(2a.11.3)** ;
- 9-Fluor-fluoren-9-carbonsäurescopinester Methobromid **(2a.11.4)** ;
- 9-Methyl-fluoren-9-carbonsäuretropenolester Methobromid **(2a.11.5) ;**
- 9-Methyl-fluoren-9-carbonsäurescopinester Methobromid **(2a.11.6)** ;

Die Verbindungen der Formel **2a.11** können gegebenenfalls in Form ihrer Enantiomere, Mischungen ihrer Enantiomere oder Racemate, sowie gegebenenfalls in Form ihrer Hydrate und/oder Solvate enthalten sein.

Beispiele für erfindungsgemäße Arzneimittelkombinationen bevorzugter Verbindungen der Formel **1** mit den vorstehend genannten Anticholinergika **2a.11** sind Kombinationen enthaltend die Verbindungen **1.a** und **2a.11.1; 1.a** und **2a.11.2; 1.a** und **2a.11.3; 1.a** und **2a.11.4; 1.a** und **2a.11.5; 1.a** und **2a.11.6; 1.b** und **2a.11.1; 1.b** und **2a.11.2; 1.b** und **2a.11.3; 1.b** und **2a.11.4; 1.b** und **2a.11.5; 1.b** und **2a.11.6; 1.d** und **2a.11.1; 1.d** und **2a.11.2; 1.d** und **2a.11.3; 1.d** und **2a.11.4; 1.d** und **2a.11.5; 1.d** und **2a.11.6; 1.f** und **2a.11.1; 1.f** und **2a.11.2; 1.f** und **2a.11.3; 1.f** und **2a.11.4; 1.f** und **2a.11.5; 1.f** und **2a.11.6; 1.h** und **2a.11.1; 1.h** und **2a.11.2; 1.h** und **2a.11.3; 1.h** und **2a.11.4; 1.h** und **2a.11.5; 1.h** und **2a.11.6; 1.j** und **2a.11.1; 1.j** und **2a.11.2; 1.j** und **2a.11.3; 1.j.** und **2a.11.4; 1.j** und **2a.11.5; 1.j** und **2a.11.6; 1.k** und **2a.11.1; 1.k** und **2a.11.2; 1.k** und **2a.11.3; 1.k** und **2a.11.4; 1.k** und **2a.11.5; 1.k** und **2a.11.6; 1.l** und **2a.11.1; 1.l** und **2a.11.2; 1.l** und **2a.11.3; 1.l** und **2a.11.4; 1.l** und **2a.11.5; 1.l** und **2a.11.6; 1.m** und **2a.11.1; 1.m** und **2a.11.2; 1.m** und **2a.11.3; 1.m** und **2a.11.4; 1.m** und **2a.11.5; 1.m** und **2a.11.6; 1.q** und **2a.11.1; 1.q** und **2a.11.2; 1.q** und **2a.11.3; 1.q** und **2a.11.4; 1.q** und **2a.11.5. 1.q** und **2a.11.6,** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Von den vorstehend genannten Kombinationen sind erfindungsgemäß diejenigen bevorzugt, die als Verbindung der Formel **1** eine der Verbindungen **1.a, 1.h, 1.j,** oder **1.k** enthalten, wobei jene Kombinationen, die eine der Verbindungen **1.h** oder **1.k** enthalten, erfindungsgemäß besonders bedeutsam sind. Von den vorstehend genannten Kombinationen sind erfindungsgemäß ferner diejenigen bevorzugt, die als Verbindung **2a.11** eine der Verbindungen **2a.11.2, 2a.11.4 , 2a.11.5** oder **2a.11.6** enthalten, wobei jene Kombinationen, die die Verbindungen **2a.11.5** oder **2a.11.6** enthalten, erfindungsgemäß besonders bedeutsam sind.

In einer weiteren bevorzugten Ausführungsform der vorliegenden Erfindung sind die in den erfindungsgemäßen Arzneimittelkombinationen enthaltenen Anticholinergika **2a** ausgewählt aus den Verbindungen der Formel **2a.12** 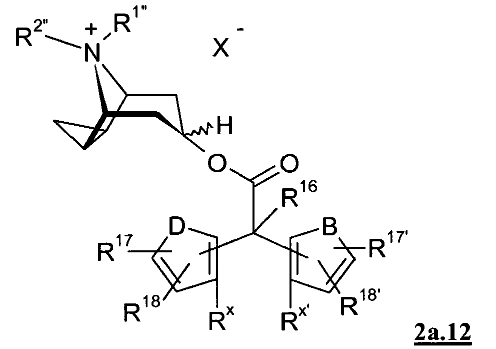 worin X ⁻ die vorstehend genannten Bedeutungen haben kann und worin
- D und B: gleich oder verschieden, bevorzugt gleich O, S, NH, CH₂, CH=CH oder N(C₁-C₄-Alkyl);
- R¹⁶: Wasserstoff, Hydroxy, -C₁-C₄-Alkyl, -C₁-C₄-Alkyloxy, -C₁-C₄-Alkylen-Halogen, -O-C₁-C₄-Alkylen-Halogen, -C₁-C₄-Alkylen-OH, -CF₃, CHF₂, -C₁-C₄-Alkylen-C₁-C₄-alkyloxy, -O-COC₁-C₄-Alkyl, -O-COC₁-C₄-Alkylen-halogen, -C₁-C₄-Alkylen-C₃-C₆-cycloalkyl, -O-COCF₃ oder Halogen;
- R^{1"} und R^{2"}: gleich oder verschieden, -C₁-C₅-Alkyl, das gegebenenfalls durch -C₃-C₆-Cycloalkyl, Hydroxy oder Halogen substituiert sein kann, oder R^{1"} und R^{2"} gemeinsam eine -C₃-C₅-Alkylenbrücke;
- R¹⁷, R¹⁸, R^{17'} und R^{18'},: gleich oder verschieden, Wasserstoff, -C₁-C₄-Alkyl, -C₁-C₄-Alkyloxy, Hydroxy, -CF₃, -CHF₂, CN, NO₂ oder Halogen;
- R^{x} und R^{x'}: gleich oder verschieden, Wasserstoff, -C₁-C₄-Alkyl, -C₁-C₄-Alkyloxy, Hydroxy, -CF₃, -CHF₂, CN, NO₂ oder Halogen, oder R^{x} und R^{x'} gemeinsam eine Einfachbindung oder eine der zweibindigen Gruppen O, S, NH, CH₂, CH₂-CH₂, N(C₁-C₄-alkyl), CH(C₁-C₄-alkyl) und -C(C₁-C₄-alkyl)₂, bedeuten.

Die Verbindungen der Formel **2a.12** sind im Stand der Technik bekannt (WO 03/064418).

Im Rahmen der erfindungsgemäßen Arzneimittelkombinationen bevorzugte Verbindungen der Formel **2a.12** sind solche, in denen
- X ⁻: Chlorid, Bromid oder Methansulfonat, bevorzugt Bromid;
- D und B: gleich oder verschieden, bevorzugt gleich O, S, NH oder CH=CH;
- R¹⁶: Wasserstoff, Hydroxy, -C₁-C₄-Alkyl, -C₁-C₄-Alkyloxy, -CF₃, -CHF₂, Fluor, Chlor oder Brom;
- R^{1'} und R^{2"}: gleich oder verschieden, C₁-C₄-Alkyl, welches gegebenenfalls substituiert sein kann durch Hydroxy, Fluor, Chlor oder Brom, oder R^{1"} und R^{2"} gemeinsam eine -C₃-C₄-Alkylenbrücke;
- R¹⁷, R¹⁸, R^{17'} und R^{18'},: gleich oder verschieden, Wasserstoff, C₁-C₄-Akyl, C₁-C₄-Alkyloxy, Hydroxy, -CF₃, -CHF₂, CN, NO₂, Fluor, Chlor oder Brom;
- R^{x} und R^{x'}: gleich oder verschieden, Wasserstoff, C₁-C₄-Alkyl, C₁-C₄-Alkyloxy, Hydroxy, -CF₃, -CHF₂, CN, NO₂, Fluor, Chlor oder Brom, oder R^{x} und R^{X'} gemeinsam eine Einfachbindung oder eine zweibindige Gruppe ausgewählt aus O, S, NH- und CH₂, bedeuten.

Im Rahmen der erfindungsgemäßen Arzneimittelkombinationen besonders bevorzugte Verbindungen der Formel **2a.12** sind solche, in denen
- X⁻: Chlorid, Bromid, oder Methansulfonat, bevorzugt Bromid;
- D und B: gleich oder verschieden, bevorzugt gleich, S oder CH=CH;
- R¹⁶: Wasserstoff, Hydroxy oder Methyl;
- R^{1"} und R^{2"}: gleich oder verschieden, Methyl oder Ethyl;
- R¹⁷, R¹⁸, R^{17'} und R^{18'},: gleich oder verschieden, Wasserstoff, -CF₃ oder Fluor, bevorzugt Wasserstoff;
- R^{x} und R^{x'}: gleich oder verschieden, Wasserstoff, -CF₃ oder Fluor, bevorzugt Wasserstoff, oder R^{x} und R^{x'} gemeinsam eine Einfachbindung oder -O-, bedeuten.

Im Rahmen der erfindungsgemäßen Arzneimittelkombinationen besonders bevorzugte Verbindungen der Formel **2a.12** sind ferner solche, in denen
- X ⁻: Bromid;
- D und B: -CH=CH-;
- R¹⁶: Wasserstoff, Hydroxy oder Methyl;
- R^{1"} und R^{2"}: Methyl;
- R¹⁷, R¹⁸, R^{17'} und R^{18'},: gleich oder verschieden, Wasserstoff oder Fluor, bevorzugt Wasserstoff;
- R^{x} und R^{x'}: gleich oder verschieden, Wasserstoff oder Fluor, bevorzugt Wasserstoff, oder R^{x} und R^{x'} gemeinsam eine Einfachbindung oder die Gruppe -O-, bedeuten.

Von besonderer Bedeutung sind diejenigen Arzneimittelkombinationen, die neben einer Verbindung der Formel **1** eine der nachfolgenden Verbindungen der Formel **2a.12** enthalten:
- Benzilsäurecyclopropyltropinester-Methobromid **(2a.12.1);**
- 2,2-Diphenylpropionsäurecyclopropyltropinester -Methobromid **(2a.12.2);**
- 9-Hydroxy-xanthen-9-carbonsäurecyclopropyltropinesterMethobromid **(2a.12.3);**
- 9-Methyl-fluoren-9-carbonsäurecyclopropyltropinester-Methobromid **(2a.12.4);**
- 9-Methyl-xanthen-9-carbonsäurecyclopropyltropinester -Methobromid **(2a.12.5);**
- 9-Hydroxy-fluoren-9-carbonsäurecyclopropyltropinester -Methobromid **(2a.12.6);**
- 4,4'-Difluorbenzilsäuremethylestercyclopropyltropinester -Methobromid (**2a.12.7**).

Die Verbindungen der Formel **2a.12** können gegebenenfalls in Form ihrer Enantiomere, Mischungen ihrer Enantiomere oder Racemate, sowie gegebenenfalls in Form ihrer Hydrate und/oder Solvate enthalten sein.

Beispiele für erfindungsgemäße Arzneimittelkombinationen bevorzugter Verbindungen der Formel **1** mit den vorstehend genannten Anticholinergika **2a.12** sind Kombinationen enthaltend die Verbindungen **1.a** und **2a.12.1; 1.a** und **2a.12.2; 1.a** und **2a.12.3; 1.a** und **2a.12.4; 1.a** und **2a.12.5; 1.a** und **2a.12.6; 1.a** und **2a.12.7; 1.b** und **2a.12.1; 1.b** und **2a.12.2; 1.b** und **2a.12.3; 1.b** und **2a.12.4; 1.b** und **2a.12.5; 1.b** und **2a.12.6; 1.b** und **2a.12.7; 1.d** und **2a.12.1; 1.d** und **2a.12.2; 1.d** und **2a.12.3; 1.d** und **2a.12.4**; **1.d** und **2a.12.5; 1.d** und **2a.12.6;** **1.d** und **2a.12.7**; **1.f** und **2a.12.1; 1.f** und **2a.12.2; 1.f** und **2a.12.3; 1.f** und **2a.12.4; 1.f** und **2a.12.5. 1.f** und **2a.12.6; 1.f** und **2a.12.7; 1.h** und **2a.12.1; 1.h** und **2a.12.2; 1.h** und **2a.12.3; 1.h** und **2a.12.4; 1.h** und **2a.12.5; 1.h** und **2a.12.6; 1.h** und **2a.12.7**; **1.j** und **2a.12.1; 1.j** und **2a.12.2; 1.j** und **2a.12.3; 1.j** und **2a,12.4; 1.j** und **2a.12.5; 1.j** und **2a.12.6; 1.j** und **2a.12.7; 1.k** und **2a.12.1; 1.k** und **2a.12.2; 1.k** und **2a.12.3; 1.k** und **2a.12.4; 1.k** und **2a.12.5; 1.k** und **2a.12.6; 1.k** und **2a.12.7; 1.l** und **2a.12.1; 1.1** und **2a.12.2; 1.1** und **2a.12.3; 1.1** und **2a.12.4; 1.1** und **2a.12.5; 1.1** und **2a.12.6; 1.l** und **2a.12.7; 1.m** und **2a.12.1. 1.m** und **2a.12.2; 1.m** und **2a.12.3; 1.m** und **2a.12.4; 1.m** und **2a.12.5; 1.m** und **2a.12.6; 1.m** und **2a.12.7; 1.q** und **2a.12.1; 1.q** und **2a.12.2; 1.q** und **2a.12.3; 1.q** und **2a.12.4; 1.q 2a.12.5; 1.q** und **2a.12.6; 1.q** und **2a.12.7,** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Von den vorstehend genannten Kombinationen sind erfindungsgemäß diejenigen bevorzugt, die als Verbindung der Formel **1** eine der Verbindungen **1.a, 1.h, 1.j,** oder **1.k** enthalten, wobei jene Kombinationen, die eine der Verbindungen **1.h** oder **1.k** enthalten, erfindungsgemäß besonders bedeutsam sind. Von den vorstehend genannten Kombinationen sind erfindungsgemäß ferner diejenigen bevorzugt, die als Verbindung **2a.11** eine der Verbindungen **2a.12.1, 2a.12.2 , 2a.12.5** oder **2a.12.7** enthalten, wobei jene Kombinationen, die die Verbindungen **2a.12.1** oder **2a.12.2** enthalten, erfindungsgemäß besonders bedeutsam sind.

In einer weiteren bevorzugten Ausführungsform der vorliegenden Erfindung sind die in den erfindungsgemäßen Arzneimittelkombinationen enthaltenen Anticholinergika **2a** ausgewählt aus den Verbindungen der Formel **2a.13** 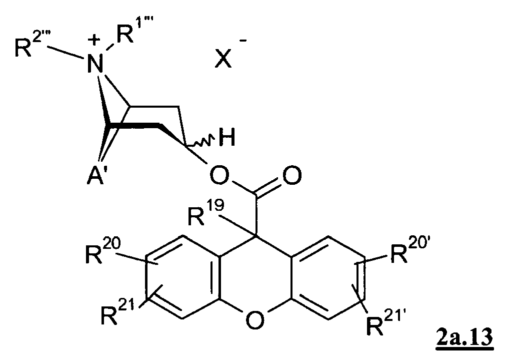 worin X ⁻ die vorstehend genannten Bedeutungen haben kann und worin
- A': eine zweibindige Gruppe ausgewählt aus 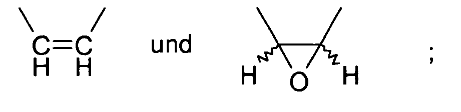
- R¹⁹: Hydroxy, Methyl, Hydroxymethyl, Ethyl, -CF₃, CHF₂ oder Fluor;
- R^{1"'} und R^{2"'}: gleich oder verschieden, C₁-C₅-Alkyl, welches gegebenenfalls substituiert sein kann durch C₃-C₆-Cycloalkyl, Hydroxy oder Halogen, oder R^{1'"} und R^{2"'} gemeinsam eine -C₃-C₅-Alkylenbrücke;
- R²⁰, R²¹, R^{20'} und R^{21'}: gleich oder verschieden, Wasserstoff, -C₁-C₄-Alkyl, -C₁-C₄-Alkyloxy, Hydroxy, -CF₃, -CHF₂, CN, NO₂ oder Halogen,
bedeuten.

Die Verbindungen der Formel **2a.13** sind im Stand der Technik bekannt (WO 03/064417).

Im Rahmen der erfindungsgemäßen Arzneimittelkombinationen bevorzugte Verbindungen der Formel **2a.13** sind solche, in denen
- A': eine zweibindige Gruppe ausgewählt aus 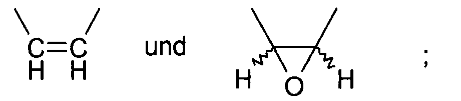
- X ⁻: Chlorid, Bromid oder Methansulfnat, bevorzugt Bromid;
- R¹⁹: Hydroxy oder Methyl;
- R^{1"'} und R^{2'"}: gleich oder verschieden, Methyl oder Ethyl, bevorzugt Methyl;
- R²⁰, R²¹, R^{20'} und R^{21'}: gleich oder verschieden, Wasserstoff, -CF₃, -CHF₂ oder Fluor, bevorzugt Wasserstoff oder Fluor, bedeuten.

Im Rahmen der erfindungsgemäßen Arzneimittelkombinationen besonders bevorzugte Verbindungen der Formel **2a.13** sind solche, in denen
- A': eine zweibindige Gruppe ausgewählt aus

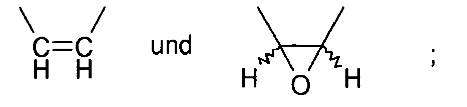

- X ⁻: Bromid;
- R¹⁹: Hydroxy oder Methyl, bevorzugt Methyl;
- R^{1"'} und R^{2'"}: gleich oder verschieden, Methyl oder Ethyl, bevorzugt Methyl;
- R²⁰, R²¹, R^{20'} und R^{21'}: gleich oder verschieden, Wasserstoff oder Fluor, bedeuten.

Von besonderer Bedeutung sind diejenigen Arzneimittelkombinationen, die neben einer Verbindung der Formel **1** eine der nachfolgenden Verbindungen der Formel **2a.13** enthalten:
- 9-Hydroxy-xanthen-9-carbonsäuretropenolester -Methobromid **(2a.13.1);**
- 9-Hydroxy-xanthen-9-carbonsäurescopinester Methobromid **(2a.13.2);**
- 9-Methyl-xanthen-9-carbonsäuretropenolester -Methobromid **(2a.13.3);**
- 9-Methyl-xanthen-9-carbonsäurescopinester -Methobromid **(2a.13.4);**
- 9-Ethyl-xanthen-9-carbonsäuretropenolester Methobromid **(2a.13.5);**
- 9-Difluormethyl-xanthen-9-carbonsäuretropenolester -Methobromid **(2a.13.6);**
- 9-Hydroxymethyl-xanthen-9-carbonsäurescopinester -Methobromid **(2a.13.7).**

Die Verbindungen der Formel **2a.13** können gegebenenfalls in Form ihrer Enantiomere, Mischungen ihrer Enantiomere oder Racemate, sowie gegebenenfalls in Form ihrer Hydrate und/oder Solvate enthalten sein.

Beispiele für erfindungsgemäße Arzneimittelkombinationen bevorzugter Verbindungen der Formel **1** mit den vorstehend genannten Anticholinergika **2a.13** sind Kombinationen enthaltend die Verbindungen **1.a** und **2a.13.1; 1.a** und **2a.13.2; 1.a** und **2a.13.3; 1.a** und **2a.13.4; 1.a** und **2a.13.5. 1.a** und **2a.13.6; 1.a** und **2a.13.7; 1.b** und **2a.13.1; 1.b** und **2a.13.2; 1.b** und **2a.13.3; 1.b** und **2a.13.4; 1.b** und **2a.13.5; 1.b** und **2a.13.6; 1.b** und **2a.13.7; 1.d** und **2a.13.1; 1.d** und **2a.13.2; 1.d** und **2a.13.3; 1.d** und **2a.13.4; 1.d** und **2 a.13.5; 1.d** und **2 a.13.6; 1.d** und **2 a.13.7; 1.f** und **2a.13.1; 1.f und 2a.13.2; 1.f und 2a.13.3; 1.f** und **2a.13.4;** **1.f** und **2a.13.5; 1.f** und **2a.13.6; 1.f** und **2a.13.7; 1.h** und **2a.13.1; 1.h** und **2a.13.2; 1.h** und **2a.13.3; 1.h** und **2a.13.4; 1.h** und **2a.13.5; 1.h** und **2a.13.6; 1.h** und **2a.13.7; 1.j** und **2a.13.1; 1.j** und **2a.13.2; 1.j** und **2a.13.3; 1.j** und **2a.13.4; 1.j** und **2a.13.5; 1.j** und **2a.13.6; 1.j** und **2a.13.7; 1.k** und **2a.13.1; 1.k** und **2a.13.2; 1.k** und **2a.13.3; 1.k** und **2a.13.4; 1.k** und **2a.13.5. 1.k** und **2a.13.6; 1.k** und **2a.13.7; 1.l** und **2a.13.1; 1.l** und **2a.13.2; 1.l** und **2a.13.3; 1.l** und **2a.13.4; 1.l** und **2a.13.5; 1.l** und **2a.13.6; 1.l** und **2a.13.7; 1.m** und **2a.13.1; 1.m** und **2a.13.2; 1.m** und **2a.13.3; 1.m** und **2a.13.4; 1.m** und **2a.13.5; 1.m** und **2a.13.6; 1.m** und **2a.13.7; 1.q** und **2a.13.1; 1.q** und **2a.13.2; 1.q** und **2a.13.3; 1.q** und **2a.13.4; 1.q** und **2a.13.5; 1.q** und **2a.13.6; 1.q** und **2a.13.7,** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Von den vorstehend genannten Kombinationen sind erfindungsgemäß diejenigen bevorzugt, die als Verbindung der Formel **1** eine der Verbindungen **1.a, 1.h, 1.j,** oder **1.k** enthalten, wobei jene Kombinationen, die eine der Verbindungen **1.h** oder **1.k** enthalten, erfindungsgemäß besonders bedeutsam sind. Von den vorstehend genannten Kombinationen sind erfindungsgemäß ferner diejenigen bevorzugt, die als Verbindung **2a.11** eine der Verbindungen **2a.13.2, 2a.13.3 , 2a.13.4** oder **2a.13.5** enthalten, wobei jene Kombinationen, die die Verbindungen **2a.13.3** oder **2a.13.4** enthalten, erfindungsgemäß besonders bedeutsam sind.

Im Rahmen der vorliegenden Erfindung ist eine Bezugnahme auf Anticholinergika **1'** als Bezugnahme auf die pharmakologisch aktiven Kationen der jeweiligen Salze zu verstehen. Diese Kationen sind Tiotropium (**2a.1'**), Oxitropium **(2a.2'),** Flutropium **(2a.3'),** Ipratropium **(2a.4'),** Glycopyrronium **(2a.5'),** Trospium **(2a.6')** sowie die nachstehenden Kationen 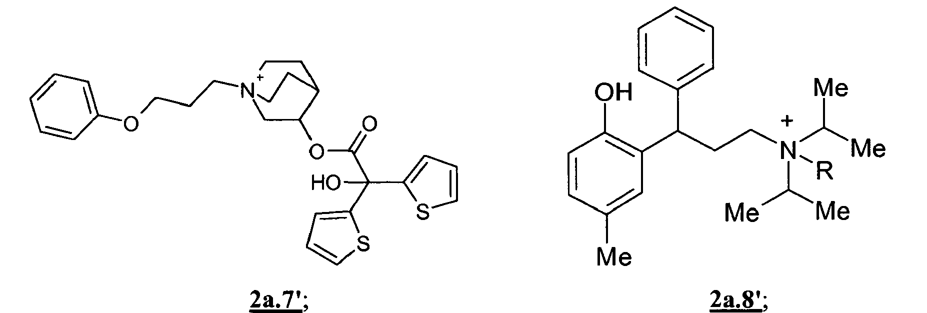 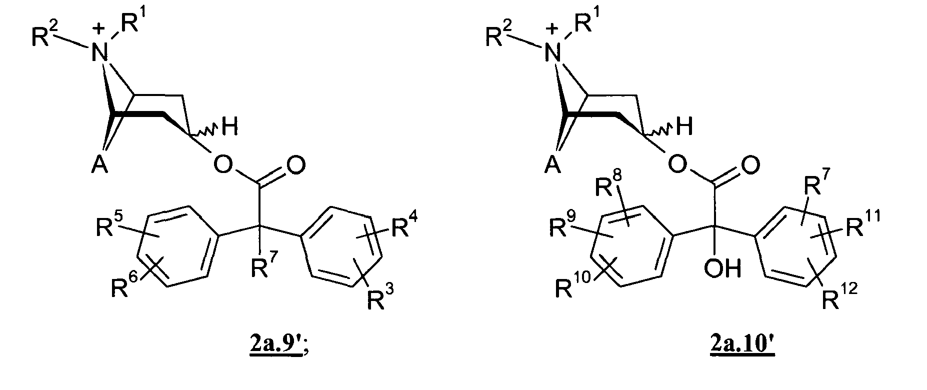 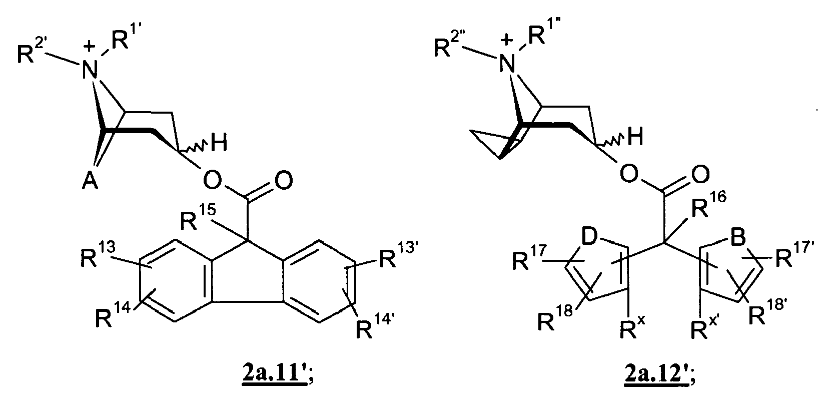 oder

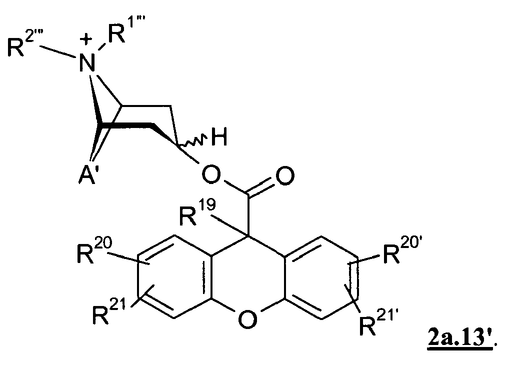

Erfindungsgemäß ferner bevorzugte Arzneimittelkombinationen enthalten neben ein oder mehrerer, bevorzugt einer Verbindung der Formel **1** als weiteren Wirkstoff ein oder mehrere, bevorzugt einen PDE IV-Inhibitor **2b,** gegebenenfalls in Kombination mit pharmazeutisch verträglichen Hilfsstoffen.

In solchen Arzneimittelkombinationen ist der PDE IV-Inhibitor **2b** bevorzugt ausgewählt aus der Gruppe bestehend aus Enprofyllin, Theophyllin, Roflumilast, Ariflo (Cilomilast), CP-325,366, BY343, D-4396 (Sch-351591), AWD-12-281 (GW-842470), N-(3,5-Dichloro-1-oxo-pyridin-4-yl)-4-difluoromethoxy-3-cyclopropylmethoxybenzamid, NCS-613, Pumafentine, (-)p-[(4αR*,10bS*)-9-Ethoxy-1,2,3,4,4a,10b-hexahydro-8-methoxy-2-methylbenzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluoromethoxyphenyl)cyclohexan-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, CDP840, Bay-198004, D-4418, PD-168787, T-440, T-2585, Arofyllin, Atizoram, V-11294A, Cl-1018, CDC-801, CDC-3052, D-22888, YM-58997, Z-15370, 9-Cyclopentyl-5,6-dihydro-7-ethyl-3-(2-thienyl)-9*H*-pyrazolo[3,4-c]-1,2,4-triazolo[4,3-a]pyridin und 9-Cyclopentyl-5,6-dihydro-7-ethyl-3-(tert-butyl)-9*H*-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate.

In besonders bevorzugten Arzneimittelkombinationen ist der PDE IV-Inhibitor **2b** ausgewählt aus der Gruppe bestehend aus Enprofyllin **(2b.1)**, Roflumilast **(2b.2),** Ariflo (Cilomilast) **(2b.3),** AWD-12-281 (GW-842470) **(2b.4),** N-(3,5-Dichloro-1-oxo-pyridin-4-yl)-4-difluoromethoxy-3-cyclopropylmethoxybenzamid **(2b.5)**, T-440 **(2b.6),** T-2585 **(2b.7)**, Arofyllin **(2b.8),** Cis[4-Cyano-4-(3-cyclopentyloxy-4-methoxyphenyl)cyclohexan-1-carbonsäure] (**2b.9**), 2-Carbomethoxy-4-cyano-4-(3-cyclopropylmethoxy-4-difluoromethoxyphenyl)cyclohexan-1-on **(2b.10),** Cis[4-cyano-4-(3-cyclopropylmethoxy-4-difluoromethoxyphenyl)cyclohexan-1-ol] **(2b.11),** PD-168787 **(2b.12),** Atizoram **(2b.13),** V-11294A **(2b.14),** Cl-1018 **(2b.15),** CDC-801 **(2b.16),** D-22888 **(2b.17),** YM-58997 **(2b.18),** Z-15370 **(2b.19),** 9-Cyclopentyl-5,6-dihydro-7-ethyl-3-(2-thienyl)-9*H-*pyrazolo[3,4-c]-1,2,4-triazolo[4,3-a]pyridin **(2b.20)** und 9-Cyclopentyl-5,6-dihydro-7-ethyl-3-(tert-butyl)-9H-pyrazolo[3,4-c]-1,2,4-triazolo[4,3-a]pyridin **(2b.21),** gegebenenfalls in Form ihrer Racemate, Enantiomere oder Diastereomere und gegebenenfalls in Form ihrer pharmakologisch verträglichen Säureadditionssalze, Solvate und/oder Hydrate.

In besonders bevorzugten Arzneimittelkombinationen ist der PDE IV-Inhibitor **2b** ausgewählt aus der Gruppe bestehend aus Roflumilast **(2b.2),** Ariflo (Cilomilast) **(2b.3),** AWD-12-281 (GW-842470) **(2b.4),** Arofyllin **(2b.8),** 2-Carbomethoxy-4-cyano-4-(3-cyclopropylmethoxy-4-difluoromethoxyphenyl)cyclohexan-1-on **(2b.10),** Cis[4-cyano-4-(3-cyclopropylmethoxy-4-difluoromethoxyphenyl)cyclohexan-1-ol] **(2b.11),** Atizoram **(2b.13),** Z-15370 **(2b.19),** 9-Cyclopentyl-5,6-dihydro-7-ethyl-3-(2-thienyl)-9H-pyrazolo[3,4-c]-1,2,4-triazolo[4,3-a]pyridin **(2b.20)** und 9-Cyclopentyl-5,6-dihydro-7-ethyl-3-(tert-butyl)-9*H*-pyrazolo[3,4-c]-1,2,4-triazolo[4,3-a]pyridin **(2b.21),** wobei Roflumilast **(2b.2),** Z-15370 **(2b.19)** und AWD-12-281 **(2b.4)** besondere Bedeutung zukommt, gegebenenfalls in Form ihrer Racemate, Enantiomere oder Diastereomere und gegebenenfalls in Form ihrer pharmakologisch verträglichen Säureadditionssalze, Solvate und/oder Hydrate.

Unter Säureadditionssalzen mit pharmakologisch verträglichen Säuren zu deren Bildung die Verbindungen **2b** gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Beispiele für erfindungsgemäße bevorzugte Arzneimittelkombinationen bevorzugter Verbindungen der Formel **1** mit den vorstehend genannten PDE IV-Inhibitoren **2b** sind Kombinationen enthaltend die Verbindungen **1.a** und **2b.1; 1.a** und **2b.2; 1.a** und **2b.3; 1.a** und **2b.4. 1.a** und **2b.5; 1.a** und **2b.6; 1.a** und **2b.7; 1.a** und **2b.8; 1.a** und **2b.9; 1.a** und **2b.10; 1.a** und **2b.11; 1.a** und **2b.12; 1.a** und **2b.13**; **1.a** und **2b.14**; **1.a** und **2b.15; 1.a** und **2b.16; 1.a** und **2b.17; 1.a** und **2b.18; 1.a** und **2b.19; 1.a** und **2b.20; 1.a** und **2b.21; 1.b** und **2b.1; 1.b** und **2b.2; 1.b** und **2b.3; 1.b** und **2b.4; 1.b** und **2b.5; 1.b** und **2b.6; 1.b** und **2b.7; 1.b** und **2b.8; 1.b** und **2b.9; 1.b** und **2b.10; 1.b** und **2b.11; 1.b** und **2b.12; 1.b** und **2b.13; 1.b** und **2b.14; 1.b** und **2b.15; 1.b** und **2b.16; 1.b** und **2b.17; 1.b** und **2b.18; 1.b** und **2b.19; 1.b** und **2b.20; 1.b** und **2b.21; 1.d** und **2b.1; 1.d** und **2b.2; 1.d** und **2b.3; 1.d** und **2b.4; 1.d** und **2b.5; 1.d** und **2b.6; 1.d** und **2b.7; 1.d** und **2b.8; 1.d** und **2b.9; 1.d** und **2b.10; 1.d** und **2b.11; 1.d** und **2b.12; 1.d** und **2b.13; 1.d** und **2b.14; 1.d** und **2b.15; 1.d** und **2b.16; 1.d** und **2b.17; 1.d** und **2b.18; 1.d** und **2b.19; 1.d** und **2b.20; 1.d** und **2b.21; 1.f** und **2b.1; 1.f** und **2b.2; 1.f und 2b.3; 1.f und 2b.4; 1.f und 2b.5; 1.f** und **2b.6; 1.f** und **2b.7; 1.f** und **2b.8; 1.f** und **2b.9; 1.f** und **2b.10; 1.f** und **2b.11; 1.f** und **2b.12; 1.f** und **2b.13; 1.f** und **2b.14; 1.f** und **2b.15; 1.f** und **2b.16; 1.f und 2b.17**; **1.f und 2b.18; 1.f und 2b.19; 1.f und 2b.20; 1.f** und **2b.21; 1.h** und **2b.1; 1.h** und **2b.2**; **1.h** und **2b.3; 1.h** und **2b.4; 1.h** und **2b.5; 1.h** und **2b.6; 1.h** und **2b.7; 1.h** und **2b.8; 1.h** und **2b.9; 1.h** und **2b.10; 1.h** und **2b.11; 1.h** und **2b.12; 1.h** und **2b.13; 1.h** und **2b.14**; **1.h** und **2b.15; 1.h** und **2b.16**; **1.h** und **2b.17; 1.h** und **2b.18; 1.h** und **2b.19; 1.h** und **2b.20; 1.h** und **2b.21; 1.j** und **2b.1; 1.j** und **2b.2; 1.j** und
**2b.3; 1.j** und **2b.4; 1.j und 2b.5; 1.j** und **2b.6; 1.j und 2b.7; 1.j und 2b.8; 1.j und 2b.9; 1.j** und **2b.10; 1.j** und **2b.11; 1.j** und **2b.12; 1.j und 2b.13; 1.j und 2b.14; 1.j und 2b.15; 1.j** und **2b.16; 1.j und 2b.17; 1.j** und **2b.18; 1.j und 2b.19; 1.j und 2b.20; 1.j und 2b.21; 1.j** und **2b.1; 1.k und 2b.2**; **1.k** und **2b.3. 1.k und 2b.4; 1.k** und **2b.5; 1.k** und **2b.6; 1.k und 2b.7; 1.k** und **2b.8; 1.k** und **2b.9; 1.k** und **2b.10; 1.k** und **2b.11; 1.k** und **2b.12; 1.k und 2b.13; 1.k** und **2b.14; 1.k** und **2b.15; 1.k** und **2b.16; 1.k** und **2b.17; 1.k und 2b.18; 1.k** und **2b.19; 1.k** und **2b.20; 1.k** und **2b.21; 1.1** und **2b.1; 1.1** und **2b.2; 1.1** und **2b.3; 1.1 und 2b.4; 1.1** und **2b.5; 1.1** und **2b.6; 1.1** und **2b.7; 1.1 und 2b.8; 1.1 und 2b.9; 1.1** und **2b.10; 1.1** und **2b.11; 1.1** und **2b.12; 1.1** und **2b.13; 1.1** und **2b.14; 1.1** und **2b.15; 1.1 und 2b.16; 1.1** und **2b.17; 1.1** und **2b.18; 1.1** und **2b.19; 1.1** und **2b.20; 1.1 und 2b.21; 1.m und 2b.1; 1.m** und **2b.2; 1.m und 2b.3; 1.m und 2b.4; 1.m und 2b.5; 1.m** und **2b.6; 1.m** und **2b.7; 1.m** und **2b.8; 1.m und 2b.9; 1.m und 2b.10; 1.m** und **2b.11; 1.m** und **2b.12; 1.m** und **2b.13; 1.m** und **2b.14; 1.m** und **2b.15; 1.m** und **2b.16; 1.m** und **2b.17; 1.m und 2b.18; 1.m** und **2b.19; 1.m** und **2b.20; 1.m** und **2b.21; 1.q** und **2b.1; 1.q** und **2b.2; 1.q** und **2b.3; 1.q** und **2b.4; 1.q** und **2b.5; 1.q** und **2b.6; 1.q** und **2b.7; 1.q** und **2b.8; 1.q und 2b.9; 1.q** und **2b.10; 1.q** und **2b.11; 1.q** und **2b.12; 1.q** und **2b.13; 1.q** und **2b.14; 1.q und 2b.15; 1.q** und **2b.16; 1.q** und **2b.17; 1.q** und **2b.18; 1.q** und **2b.19; 1.q** und **2b.20 oder 1.q** und **2b.21,** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate jeweils gegebenenfalls in Form ihrer Racemate, Enantiomere oder **Diastereomere** und gegebenenfalls in Form ihrer pharmakologisch verträglichen Säureadditionssalze, Solvate und/oder Hydrate.

Von den vorstehend genannten Kombinationen sind erfindungsgemäß diejenigen bevorzugt, die als Verbindung der Formel **1** eine der Verbindungen **1.a, 1.h, 1.j,** oder **1.k** enthalten, wobei jene Kombinationen, die eine der Verbindungen **1.h** oder 1.k enthalten, erfindungsgemäß besonders bedeutsam sind. Von den vorstehend genannten Kombinationen sind erfindungsgemäß ferner diejenigen bevorzugt, die als Verbindung **2b** eine der Verbindungen **2b.2, 2b.3, 2b.4, 2b.8, 2b.10, 2b.11, 2b.13, 2b.19, 2b.20** oder **2b.21,** enthalten, wobei jene Kombinationen, die eine der Verbindungen **2b.2, 2b.4** oder **2b.19** enthalten, erfindungsgemäß besonders bedeutsam sind.

Erfindungsgemäß ferner bevorzugte Arzneimittelkombinationen enthalten neben ein oder mehrerer, bevorzugt einer Verbindung der Formel **1** als weiteren Wirkstoff ein oder mehrere, bevorzugt ein Steroid **2c**, gegebenenfalls in Kombination mit pharmazeutisch verträglichen Hilfsstoffen.

In solchen Arzneimittelkombinationen ist das Steroid **2c** bevorzugt ausgewählt aus der Gruppe bestehend aus Prednisolon **(2c.1),** Prednison **(2c.2),** Butixocortpropionat **(2c.3),** RPR-106541 **(2c.4),** Flunisolid **(2c.5),** Beclomethason (**2c.6**), Triamcinolon **(2c.7),** Budesonid **(2c.8),** Fluticason **(2c.9),** Mometason **(2c.10),** Ciclesonid **(2c.11),** Rofleponid **(2c.12),** ST-126 **(2c.13),** Dexamethason **(2c.14),** 6α,9α-Difluoro-17α-[(2-furanylcarbonyl)oxy]-11β-hydroxy-16α-methyl-3-oxo-androsta-1,4-dien-17β-carbothionsäure (S)-fluoromethylester **(2c.15),** 6α,9α-Difluoro-1β-hydroxy-16α-methyl-3-oxo-17α-propionyloxy-androsta-1,4-dien-17β-carbothionsäure (S)-(2-oxo-tetrahydrofuran-3S-yl)ester **(2c.16)** und Etiprednol-dichloroacetat (BNP-166, **2c.17),** gegebenenfalls in Form ihrer Racemate, Enantiomere oder Diastereomere und gegebenenfalls in Form ihrer Salze und Derivate, ihrer Solvate und/oder Hydrate.

In besonders bevorzugten Arzneimittelkombinationen ist das Steroid **2c** ausgewählt aus der Gruppe bestehend aus Flunisolid **(2c.5),** Beclomethason **(2c.6),** Triamcinolon **(2c.7),** Budesonid (**2c.8**). Fluticason **(2c.9),** Mometason **(2c.10),** Ciclesonid **(2c.11),** Rofleponid **(2c.12)**, ST-126 **(2c.13)**, Dexamethason (**2c.14) ,** 6α,9α-Difluoro-17α-[(2-furanylcarbonyl)oxy]-11β-hydroxy-16α-methyl-3-oxo-androsta-1,4-dien-17β-carbothionsäure (S)-fluoromethylester **(2c.15),** 6α,9α-Difluoro-1β-hydroxy-16α-methyl-3-oxo-17α-propionyloxy-androsta-1,4-dien-17β-carbothionsäure (S)-(2-oxo-tetrahydrofuran-3S-yl)ester **(2c.16)** und Etiprednol-dichloroacetat **(2c.17),** gegebenenfalls in Form ihrer Racemate, Enantiomere oder Diastereomere und gegebenenfalls in Form ihrer Salze und Derivate, ihrer Solvate und/oder Hydrate.

In besonders bevorzugten Arzneimittelkombinationen ist das Steroid **2c** ausgewählt aus der Gruppe bestehend aus Budesonid **(2c.8),** Fluticason **(2c.9),** Mometason **(2c.10),** Ciclesonid **(2c.11),** 6α,9α-Difluoro-17α-[(2-furanylcarbonyl)oxy]-11β-hydroxy-16α-methyl-3-oxo-androsta-1,4-dien-17β-carbothionsäure (S)-fluoromethylester **(2c.15)** und Etiprednol-dichloroacetat **(2c.17),** gegebenenfalls in Form ihrer Racemate, Enantiomere oder Diastereomere und gegebenenfalls in Form ihrer Salze und Derivate, ihrer Solvate und/oder Hydrate.

Jede Bezugnahme auf Steroide **2c** schließt eine Bezugnahme auf deren gegebenenfalls existierende Salze oder Derivate, Hydrate oder Solvate mit ein. Beispiele möglicher Salze und Derivate der Steroide **2c** können sein: Alkalisalze, wie beispielsweise Natrium- oder Kaliumsalze, Sulfobenzoate, Phosphate, Isonicotinate, Acetate, Propionate, Dihydrogenphosphate, Palmitate, Pivalate oder auch Furoate.

Beispiele für erfindungsgemäße bevorzugte Arzneimittelkombinationen bevorzugter Verbindungen der **Formel 1** mit den vorstehend genannten Steroiden **2c** sind Kombinationen enthaltend die Verbindungen **1.a** und **2c.1; 1.a** und **2c.2; 1.a** und **2c.3; 1.a** und **2c.4; 1.a** und **2c.5; 1.a** und **2c.6; 1.a und 2c.7; 1.a** und **2c.8; 1.a** und **2c.9; 1.a** und **2c.10; 1.a** und **2c.11; 1.a** und **2c.12; 1.a** und **2c.13; 1.a** und **2c.14; 1.a** und **2c.15; 1.a** und **2c.16; 1.a** und **2c.17; 1.b** und **2c.1; 1.b** und **2c.2; 1.b** und **2c.3; 1.b** und **2c.4; 1.b** und **2c.5; 1.b** und **2c.6; 1.b** und **2c.7; 1.b** und **2c.8; 1.b** und **2c.9; 1.b** und **2c.10; 1.b** und **2c.11; 1.b** und **2c.12; 1.b** und **2c.13; 1.b** und **2c.14; 1.b** und **2c.15; 1.b** und **2c.16; 1.b** und **2c.17; 1.d** und **2c.1; 1.d** und **2c.2; 1.d** und **2c.3; 1.d** und **2c.4; 1.d** und **2c.5; 1.d** und **2c.6; 1.d** und **2c.7; 1.d** und **2c.8; 1.d** und **2c.9; 1.d** und **2c.10; 1.d** und **2c.11; 1.d** und **2c.12; 1.d** und **2c.13; 1.d** und **2c.14; 1.d** und **2c.15; 1.d** und **2c.16; 1.d** und **2c.17; 1.f und 2c.1; 1.f** und **2c.2; 1.f** und **2c.3; 1.f** und **2c.4; 1.f** und **2c.5**; **1.f** und **2c.6; 1.f** und **2c.7; 1.f** und **2c.8**; **1.f** und **2c.9; 1.f** und **2c.10; 1.f** und **2c.11; 1.f** und **2c.12; 1.f** und **2c.13; 1.f** und **2c.14; 1.f** und **2c.15; 1.f** und **2c.16; 1.f und 2c.17; 1.h** und **2c.1; 1.h** und **2c.2; 1.h** und **2c.3; 1.h** und **2c.4; 1.h** und **2c.5; 1.h** und **2c.6; 1.h** und **2c.7; 1.h** und **2c.8. 1.h** und **2c.9; 1.h** und **2c,10; 1.h** und **2c.11; 1.h** und **2c.12; 1.h** und **2c.13; 1.h** und **2c.14; 1.h** und **2c.15; 1.h** und **2c.16; 1.h** und **2c.17; 1.j** und **2c.1; 1.j** und **2c.2; 1.j** und **2c.3; 1.j** und **2c.4; 1.j** und **2c.5; 1.j** und **2c.6; 1.i** und **2c.7; 1.j** und **2c.8; 1.j** und **2c.9; 1.j** und **2c,10; 1.j** und **2c.11; 1.j** und **2c.12; 1.j** und **2c.13; 1.j** und **2c.14; 1.j** und **2c.15; 1.j** und **2c.16; 1.j** und **2c.17; 1.k** und **2c.1; 1.k** und **2c.2; 1.k** und 2c.3; 1.k und **2c.4; 1.k** und **2c.5; 1.k** und **2c.6; 1.k** und **2c.7; 1.k** und **2c.8; 1.k** und **2c.9; 1.k** und **2c.10; 1.k** und **2c.11; 1.k** und **2c.12; 1.k** und **2c.13; 1.k** und **2c.14; 1.k** und **2c.15; 1.k** und **2c.16; 1.k** und **2c.17; 1.1** und **2c.1; 1.1** und 2c.2; **1.1** und **2c.3; 1.1** und **2c.4; 1.1** und **2c.5; 1.1** und **2c.6; 1.1** und **2c.7; 1.1** und **2c.8; 1.1** und **2c.9; 1.1** und **2c.10; 1.1** und **2c**.**11** ;**1.1** und **2c.12**; **1.1** und **2c.13**; **1.1** und **2c.14**; **1.1** und **2c.15**; **1.1** und **2c.16**; **1.1** und **2c.17**; **1.m** und **2c.1**; **1.m** und **2c.2**; **1.m** und **2c.3**; **1.m** und **2c.4; 1.m** und **2c.5**; **1.m** und **2c.6**; **1.m** und **2c.7; 1.m** und **2c.8**; **1.m** und **2c.9**; **1.m** und **2c.10**; **1.m** und **2c.11**; **1.m** und **2c.12; 1.m** und **2c.13; 1.m** und **2c.14; 1.m** und **2c.15; 1.m** und **2c.16; 1.m** und **2c.17**; **1.q** und **2c.1; 1.q** und **2c.2; 1.q** und **2c.3; 1.q** und **2c.4; 1.q** und **2c.5; 1.q** und **2c.6; 1.q** und **2c.7; 1.q** und **2c.8; 1.q** und **2c.9; 1.q** und **2c.10**; **1.q** und **2c.11; 1.q** und **2c.12; 1.q** und **2c.13; 1.q** und 2**c.14; 1.q** und **2c.15; 1.q** und **2c.16** oder **1.q** und **2c.17** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Von den vorstehend genannten Kombinationen sind erfindungsgemäß diejenigen bevorzugt, die als Verbindung der Formel **1** eine der Verbindungen **1.a, 1.h, 1.j,** oder **1.k** enthalten, wobei jene Kombinationen, die eine der Verbindungen **1.h** oder **1.k** enthalten, erfindungsgemäß besonders bedeutsam sind. Von den vorstehend genannten Kombinationen sind erfindungsgemäß ferner diejenigen bevorzugt, die als Verbindung **2c** eine der Verbindungen **2c.5, 2c.6, 2c.7, 2c.8, 2c.9, 2c.10, 2c.11, 2c.12, 2c.13, 2c.14, 2c.15, 2c.16** oder **2c.17** enthalten, wobei jene Kombinationen, die eine der Verbindungen **2c.8, 2c.9, 2c.10, 2c.11, 2c.15** oder **2c.17** enthalten, erfindungsgemäß besonders bedeutsam sind.

Erfindungsgemäß ferner bevorzugte Arzneimittelkombinationen enthalten neben ein oder mehrerer, bevorzugt einer Verbindung der Formel **1** als weiteren Wirkstoff ein oder mehrere, bevorzugt einen LTD4-Antagonist **2d,** gegebenenfalls in Kombination mit pharmazeutisch verträglichen Hilfsstoffen.

In solchen Arzneimittelkombinationen ist der LTD4-Antagonist **2d** bevorzugt ausgewählt aus der Gruppe bestehend aus Montelukast **(2d.1),** 1-(((R)-(3-(2-(6,7-Difluoro-2-quinolinyl)ethenyl)phenyl)-3-(2-(2-hydroxy-2-propyl)phenyl)thio)methylcyclopropanessigsäure **(2d.2),** 1-(((1(R)-3(3-(2-(2,3-Dichlorothieno[3,2-b]pyridin-5-yl)-(E)-ethenyl)phenyl)-3-(2-(1-hydroxy-1-methylethyl)phenyl)propyl)thio)methyl)cyclopropanessigsäure **(2d.3),** Pranlukast **(2d.4),** Zafirlukast **(2d.5),** [2-[[2-(4-tert-Butyl-2-thiazolyl)-5-benzofuranyl]oxymethyl]phenyl]essigsäure (**2d.6**). MCC-847 (ZD-3523) **(2d.7),** MN-001 **(2d.8),** MEN-91507 (LM-1507) **(2d.9),** VUF-5078 **(2d.10),** VUF-K-8707 **(2d.11)** und L-733321 **(2d.12),** gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

In bevorzugten Arzneimittelkombinationen ist der LTD4-Antagonist **2d** ausgewählt aus der Gruppe bestehend aus Montelukast **(2d.1),** Pranlukast **(2d.4),** Zafirlukast **(2d.5),** MCC-847 (ZD-3523) **(2d.7),** MN-001 **(2d.8),** MEN-91507 (LM-1507) (**2d.9),** VUF-5078 **(2d.10),** VUF-K-8707 **(2d.11)** und L-733321 **(2d.12),** gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

In besonders bevorzugten Arzneimittelkombinationen ist der LTD4-Antagonist **2d** ausgewählt aus der Gruppe bestehend aus Montelukast **(2d.1),** Pranlukast **(2d.4),** Zafirlukast **(2d.5),** MCC-847 (ZD-3523) **(2d.7),** MN-001 **(2d.8)** und MEN-91507 (LM-1507) **(2d.9),** wobei Montelukast **(2d.1),** Pranlukast **(2d.4)** und Zafirlukast **(2d.5)** besonders bevorzugt sind, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate.

Unter Säureadditionssalzen mit pharmakologisch verträglichen Säuren zu deren Bildung die Verbindungen **2d** gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Unter Salzen oder Derivaten zu deren Bildung die Verbindungen **2d** gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Beispiele für erfindungsgemäße bevorzugte Arzneimittelkombinationen bevorzugter Verbindungen der Formel **1** mit den vorstehend genannten LTD4-Antagonisten **2d** sind Kombinationen enthaltend die Verbindungen **1.a** und **2d.1; 1.a** und **2d.2; 1.a** und **2d.3; 1.a** und **2d.4; 1.a** und **2d.5; 1.a** und **2d.6; 1.a** und **2d.7; 1.a** und **2d.8; 1.a** und **2d.9; 1.a** und **2d.10; 1.a** und **2d.11; 1.a** und **2d.12; 1.b** und **2d.1; 1.b** und **2d.2; 1.b** und **2d.3; 1.b** und **2d.4; 1.b** und **2d.5; 1.b** und **2d.6; 1.b** und **2d.7; 1.b** und **2d.8; 1.b** und **2d.9; 1.b** und **2d.10; 1.b** und **2d.11; 1.b** und **2d.12; 1.d** und **2d.1; 1.d** und **2d.2; 1.d** und **2d.3; 1.d** und **2d.4; 1.d** und **2d.5; 1.d** und **2d.6; 1.d** und **2d.7; 1.d** und **2d.8; 1.d** und **2d.9; 1.d** und **2d.10; 1.d** und **2d.11; 1.d** und **2d.12; 1.f** und **2d.1; 1.f** und **2d.2; 1.f** und **2d.3; 1.f** und **2d.4; 1.f** und **2d.5; 1.f** und **2d.6; 1.f** und **2d.7; 1-f** und **2d.8; 1.f** und **2d.9; 1.f** und **2d.10; 1.f** und **2d.11; 1.f** und **2d.12; 1.h** und **2d.1; 1.h** und **2d.2; 1.h** und **2d.3; 1.h** und **2d.4; 1.h** und **2d.5; 1.h** und **2d.6; 1.h** und **2d.7**; **1.h** und **2d.8; 1.h** und **2d.9; 1.h** und **2d.10; 1.h** und **2d.11; 1.h** und **2d.12; 1.j** und **2d.1; 1.j** und **2d.2; 1.j** und **2d.3; 1.j** und **2d.4; 1.j** und **2d.5; 1.j** und **2d.6; 1.j** und **2d.7; 1.j** und **2d.8; 1.j** und **2d.9; 1.j** und **2d.10; 1.j** und **2d.11; 1.j** und **2d.12; 1.k** und **2d.1; 1.k** und **2d.2; 1.k** und **2d.3; 1.k** und **2d.4; 1.k** und **2d.5; 1.k** und **2d.6; 1.k** und **2d.7; 1.k** und **2d.8; 1.k** und **2d.9; 1.k** und **2d.10; 1.k** und **2d.11; 1.k** und **2d.12; 1.I** und **2d.1; 1.1** und **2d.2; 1.1** und **2d.3; 1.1** und **2d.4; 1.I** und **2d.5; 1.I** und **2d.6; 1.I** und **2d.7; 1.I** und **2d.8; 1.I** und **2d.9; 1.I** und **2d.10; 1.I** und **2d.11; 1.I** und **2d.12; 1.m** und **2d.1; 1.m** und **2d.2; 1.m** und **2d.3; 1.m** und **2d.4; 1.m** und **2d.5; 1.m** und **2d.6; 1.m** und **2d.7; 1.m** und **2d.8; 1.m** und **2d.9; 1.m** und **2d.10; 1.m** und **2d.11; 1.m** und **2d.12; 1.q** und **2d.1; 1.g.** und **2d.2; 1.q** und **2d.3; 1.q** und **2d.4; 1.q** und **2d.5; 1.q** und **2d.6; 1.q** und **2d.7; 1.q** und **2d.8; 1.q** und **2d.9; 1.q** und **2d.10; 1.q** und **2d.11** oder **1.q** und **2d.12,** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Von den vorstehend genannten Kombinationen sind erfindungsgemäß diejenigen bevorzugt, die als Verbindung der Formel **1** eine der Verbindungen **1.a**, **1.h, 1.j,** oder **1.k** enthalten, wobei jene Kombinationen, die eine der Verbindungen **1.h** oder **1.k** enthalten, erfindungsgemäß besonders bedeutsam sind. Von den vorstehend genannten Kombinationen sind erfindungsgemäß ferner diejenigen bevorzugt, die als Verbindung **2d** eine der Verbindungen **2d.1, 2d.4, 2d.5, 2d.7, 2d.8, 2d.9, 2d.10, 2d.11** oder **2d.12,** enthalten, wobei jene Kombinationen, die eine der Verbindungen **2d.1, 2d.4, 2d.5, 2d.7, 2d.8** oder **2d.9** enthalten, erfindungsgemäß besonders bedeutsam sind, wobei jenen Kombinationen, die eine der Verbindungen **2d.1, 2d.4** oder **2d.5** enthalten, herausragende Bedeutung zukommt.

Erfindungsgemäß ferner bevorzugte Arzneimittelkombinationen enthalten neben ein oder mehrerer, bevorzugt einer Verbindung der Formel **1** als weiteren Wirkstoff ein oder mehrere, bevorzugt einen EGFR-Hemmer **2e**, gegebenenfalls in Kombination mit pharmazeutisch verträglichen Hilfsstoffen.

In solchen Arzneimittelkombinationen ist der EGFR-Hemmer **2e** beispielsweise ausgewählt aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(*R*)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((*R*)-6-methyl-2-oxo-morpholin-4-yl)-1-oxo-2-buten-1-yl]amino}-7-cyclopropylmethoxy-chinazolin, 4-[(3-Chlor-4-fluor-phenyl)amino]-6-{[4-((*R*)-6-methyl-2-oxo-morpholin-4-yl)-1-oxo-2-buten-1-yl]amino}-7-[(*S*)-(tetrahydrofuran-3-yl)oxy]-chinazolin, 4-[(3-Chlor-4-fluorphenyl)amino]-6- {[4-((*R*)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((*S*)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopentyloxy-chinazolin, 4-[(*R*)-(1-Phenyl-ethyl)amino]-6- { [4-(N,N-bis-(2-methoxy-ethyl)-amino)-1-oxo-2-buten-1-yl]amino}-7-cyclopropylmethoxy-chinazolin, 4-[(*R*)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin, 4-[(*R*)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(*R*)-(1-Phenylethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-4{[4-(N,N-dimethylamino)-1-oxo-2-buten-1-yl]amino}-7-((*R*)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((*S*)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(*R*)-(tetrahydrofuran-2-yl)methoxy]-chinazolin, 4-[(3-Chlor-4-fluorphenyl)amino]-6-{[4-(N,N-dimethylamino)-1-oxo-2-buten-1-yl]amino}-7-[(*S*)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(*R*)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluorbenzyloxy)-phenyl]amino}-6-(5-{[(2-methansulfonyl-ethyl)amino]methyl}-furan-2-yl)chinazolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinylphenyl)amino]-6-{[4-(5,5-dimethyl-2-oxo-morpholin-4-yl)-1-oxo-2-buten-1-yl]amino}chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluorphenyl)amino]-6-[1-(tert.-butyloxycarbonyl)-piperidin-4-yloxy]-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylaminocyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluorphenyl)amino]-6- {1-[(methoxymethyl)carbonyl]-piperidin-4-yloxy} -7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{trans-4-[(morpholin-4-yl)sulfonylamino]-cyclohexan-1-yloxy}-7-methoxy-chinazolin, 4-[(3-Chlor-4-fluorphenyl)amino]-6-(tetrahydropyran-4-yloxy)-7-(2-acetylamino-ethoxy)-chinazolin, 4-[(3-Chlor-4-fluor-phenyl)amino]-6-(tetrahydropyran-4-yloxy)-7-(2-methansulfonylaminoethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(1-aminocarbonylmethyl-piperidin-4-yloxy)-7-methoxy-chinazolin, 4-[(3-Chlor-4-fluorphenyl)amino]-6-(cis-4-{N-[(tetrahydropyran-4-yl)carbonyl]-N-methyl-amino}cyclohexan-1-yloxy)-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin, 4-[(3-Chlor-4-fluorphenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-ethoxychinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxyacetyl)-piperidin-4-yloxy]-7-(2-methoxy-ethoxy)-chinazolin, 4-[(3-Chlor-4-fluorphenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluorphenyl)amino]-6-(cis-4-{N-[(4-methyl-piperazin-1-yl)carbonyl]-N-methyl-amino}cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy} -7-methoxy-chinazolin, 4-[(3-Chlor-4-fluor-phenyl)amino]-6-{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluorphenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxyacetyl)-N-methyl-amino]-cyclohexan-1-yloxy}-7-methoxy-chinazolin, 4-[(3-Ethinylphenyl)amino]-6-(piperidin-4-yloxy)-7-methoxy-chinazolin, 4-[(3-Ethinyl-phenyl)amino]-6-[1-(2-methoxy-acetyl)-piperidin-4-yloxy]-7-methoxy-chinazolin, 4-[(3-Ethinylphenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6- {1-[(2-methoxyethyl)carbonyl]-piperidin-4-yloxy} -7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin , 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylaminocyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin , 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluorphenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonylpiperidin-4-yloxy)-7-methoxy-chinazolin, 4-[(3-Chlor-4-fluor-phenyl)amino]-6-(1-cyanopiperidin-4-yloxy)-7-methoxy-chinazolin, Cetuximab, Trastuzumab, ABX-EGF und Mab ICR-62, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

In solchen Arzneimittelkombinationen ist der EGFR-Hemmer **2e** bevorzugt ausgewählt aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluorphenyl)amino]-6-{[4-(N,N-diethylamino)-1-oxo-2-buten-1-yl]amino}-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopropylmethoxy-chinazolin, 4-[(R)-(1-Phenyl-ethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxomorpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluorphenyl)amino]-6-{[4-((R)-2-methoxymethyl-6-oxo-morpholin-4-yl)-1-oxo-2-buten-1-yl]amino}-7-cyclopropylmethoxy-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-cyclopentyloxy-chinazolin, 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin, 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin, 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin, 4-[(R)-(1-Phenylethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin, 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin , 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin, 4-[(3-Ethinyl-phenyl)amino]-6,7-bis-(2-methoxy-ethoxy)-chinazolin, 4-[(3-Chlor-4-fluorphenyl)amino]-7-[3-(morpholin-4-yl)-propyloxy]-6-[(vinylcarbonyl)amino]-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxy-chinolin, 4-{[3-Chlor-4-(3-fluor-benzyloxy)-phenyl]amino}-6-(5-{[(2-methansulfonyl-ethyl)amino]methyl}-furan-2-yl)chinazolin , 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin , 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin, 4-[(3-Ethinylphenyl)amino]-6-{[4-(5,5-dimethyl-2-oxo-morpholin-4-yl)-1-oxo-2-buten-1-yl]amino}chinazolin, 4-[(3-Chlor-4-fluor-phenyl)amino]-6-[2-(2,2-dimethyl-6-oxo-morpholin-4-yl)-ethoxy]-7-methoxy-chinazolin, 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin, 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluorphenyl)amino]-6-[1-(tert.-butyloxycarbonyl)-piperidin-4-yloxy]-7-methoxy-chinazolin, 4-[(3-Chlor-4-fluor-phenyl)amino]-6-(trans-4-amino-cyclohexan-1-yloxy)-7-methoxy-chinazolin, 4-[(3-Chlor-4-fluor-phenyl)amino]-6-(trans-4-methansulfonylaminocyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-(1-methyl-piperidin-4-yloxy)-7-methoxy-chinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluorphenyl)amino]-6-{1-[(methoxymethyl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-((S)-tetrahydrofuran-3-yloxy)-7-hydroxy-chinazolin, 4-[(3-Chlor-4-fluor-phenyl)amino]-6-(tetrahydropyran-4-yloxy)-7-(2-methoxy-ethoxy)-chinazolin, 4-[(3-Chlor-4-fluor-phenyl)amino]-6-{trans-4-[(dimethylamino)sulfonylamino]-cyclohexan-1-yloxy}-7-methoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{trans-4-[(morpholin-4-yl)sulfonylamino]-cyclohexan-1-yloxy}-7-methoxy-chinazolin, 4-[(3-Chlor-4-fluorphenyl)amino]-6-(tetrahydropyran-4-yloxy)-7-(2-acetylamino-ethoxy)-chinazolin , 4-[(3-Chlor-4-fluor-phenyl)amino]-6-(tetrahydropyran-4-yloxy)-7-(2-methansulfonylamino-ethoxy)-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluorphenyl)amino]-6-(1-aminocarbonylmethyl-piperidin-4-yloxy)-7-methoxy-chinazolin, 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin , 4-[(3-Chlor-4-fluorphenyl)amino]-6-(cis-4-{N-[(morpholin-4-yl)carbonyl]-N-methyl-amino}-cyclohexan-1 - yloxy)-7-methoxy-chinazolin, 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-ethoxy-chinazolin, 4-[(3-Chlor-4-fluor-phenyl)amino]-6-(1-methansulfonylpiperidin-4-yloxy)-7-(2-methoxy-ethoxy)-chinazolin , 4-[(3-Chlor-4-fluor-phenyl)amino]-6-[1-(2-methoxy-acetyl)-piperidin-4-yloxy]-7-(2-methoxy-ethoxy)-chinazolin , 4-[(3-Chlor-4-fluor-phenyl)amino]-6-(cis-4-acetylamino-cyclohexan-1-yloxy)-7-methoxy-chinazolin, 4-[(3-Ethinyl-phenyl)amino]-6-[1-(tert.-butyloxycarbonyl)-piperidin-4-yloxy]-7-methoxy-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-(cis-4-{N-[(4-methyl-piperazin-1-yl)carbonyl]-N-methyl-amino}cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Chlor-4-fluor-phenyl)amino]-6-{1-[(morpholin-4-yl)carbonyl]-piperidin-4-yloxy}-7-(2-methoxy-ethoxy)-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Chlor-4-fluorphenyl)amino]-6-(1-methyl-piperidin-4-yloxy)-7(2-methoxy-ethoxy)-chinazolin, 4-[(3-Chlor-4-fluor-phenyl)amino]-6-(1-isopropyloxycarbonyl-piperidin-4-yloxy)-7-methoxy-chinazolin, 4-[(3-Chlor-4-fluor-phenyl)amino]-6-(cis-4-methylamino-cyclohexan-1-yloxy)-7-methoxy-chinazolin, 4-[(3-Chlor-4-fluor-phenyl)amino]-6-{cis-4-[N-(2-methoxyacetyl)-N-methyl-amino]-cyclohexan-1-yloxy}-7-methoxy-chinazolin, 4-[(3-Ethinylphenyl)amino]-6-(piperidin-4-yloxy)-7-methoxy-chinazolin, 4-[(3-Ethinyl-phenyl)amino]-6-[1-(2-methoxy-acetyl)-piperidin-4-yloxy]-7-methoxy-chinazolin, 4-[(3-Ethinylphenyl)amino]-6- {1-[(morpholin-4-yl)carbonyl]-piperidin-4-yloxy} -7-methoxy-chinazolin, 4-[(3-Chlor-4-fluor-phenyl)amino]-6-{1-[(cis-2,6-dimethyl-morpholin-4-yl)carbonyl]-piperidin-4-yloxy}-7-methoxy-chinazolin, 4-[(3-Chlor-4-fluor-phenyl)amino]-6-{1-[(2-methyl-morpholin-4-yl)carbonyl]-piperidin-4-yloxy}-7-methoxy-chinazolin, 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-{1-[(2-methoxyethyl)carbonyl]-piperidin-4-yloxy}-7-methoxy-chinazolin, 4-[(3-Chlor-4-fluor-phenyl)amino]-6-{1-[(3-methoxypropyl-amino)-carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin , 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylaminocyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin , 4-[(3-Chlor-4-fluor-phenyl)amino]-6-(trans-4-dimethylamino-cyclohexan-1-yloxy)-7-methoxy-chinazolin , 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluorphenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonylpiperidin-4-yloxy)-7-methoxy-chinazolin, 4-[(3-Chlor-4-fluor-phenyl)amino]-6-(1-cyanopiperidin-4-yloxy)-7-methoxy-chinazolin, und Cetuximab, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Besonders bevorzugt gelangen im Rahmen der erfindungsgemäßen Arzneimittelkombinationen die EGFR-Hemmer **2a** zur Anwendung, die ausgewählt sind aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(R)-(1-Phenylethyl)amino]-6-{[4-(morpholin-4-yl)-1-oxo-2-buten-1-yl]amino}-7-cyclopentyloxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methylamino]-1-oxo-2-buten-1-yl}amino)-7-cyclopropylmethoxy-chinazolin, 4-[(R)-(1-Phenylethyl)amino]-6-({4-[N-(tetrahydropyran-4-yl)-N-methyl-amino]-1-oxo-2-buten-1-yl}amino)-7-cyclopropylmethoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin, 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin, 4-[(3-Ethinylphenyl)amino]-6,7-bis-(2-methoxy-ethoxy)-chinazolin, 4-[(R)-(1-Phenyl-ethyl)amino]-6-(4-hydroxy-phenyl)-7H-pyrrolo[2,3-d]pyrimidin, 3-Cyano-4-[(3-chlor-4-fluorphenyl)amino]-6-{[4-(N,N-dimethylamino)-1-oxo-2-buten-1-yl]amino}-7-ethoxychinolin, 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin, 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-{2-[4-(2-oxo-morpholin-4-yl)-piperidin-1-yl]-ethoxy}-7-methoxy-chinazolin, 4-[(3-Chlor-4-fluorphenyl)amino]-6-(trans-4-amino-cyclohexan-1-yloxy)-7-methoxy-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluor-phenyl)amino]-6-(piperidin-3-yloxy)-7-methoxy-chinazolin, 4-[(3-Chlor-4-fluor-phenyl)amino]-6-[1-(2-acetylamino-ethyl)-piperidin-4-yloxy]-7-methoxy-chinazolin, 4-[(3-Chlor-4-fluor-phenyl)amino]-6-(tetrahydropyran-4-yloxy)-7-ethoxy-chinazolin, 4-[(3-Chlor-4-fluor-phenyl)amino]-6-{trans-4-[(morpholin-4-yl)carbonylamino]-cyclohexan-1-yloxy}-7-methoxy-chinazolin, 4-[(3-Chlor-4-fluor-phenyl)amino]-6-{1-[(piperidin-1-yl)carbonyl]-piperidin-4-yloxy}-7-methoxy-chinazolin, 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-(trans-4-ethansulfonylamino-cyclohexan-1-yloxy)-7-methoxy-chinazolin, 4-[(3-Chlor-4-fluor-phenyl)amino]-6-(1-methansulfonyl-piperidin-4-yloxy)-7-(2-methoxy-ethoxy)-chinazolin, 4-[(3-Chlor-4-fluor-phenyl)amino]-6-[1-(2-methoxyacetyl)-piperidin-4-yloxy]-7-(2-methoxy-ethoxy)-chinazolin, 4-[(3-Ethinyl-phenyl)amino]-6-(tetrahydropyran-4-yloxy]-7-methoxy-chinazolin, 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin, 4-[(3-Chlor-4-fluor-phenyl)amino]-6-{cis-4-[(morpholin-4-yl)carbonylamino]-cyclohexan-1-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Ethinylphenyl)amino]-6-(1-acetyl-piperidin-4-yloxy)-7-methoxy-chinazolin, 4-[(3-Ethinylphenyl)amino]-6-(1-methyl-piperidin-4-yloxy)-7-methoxy-chinazolin, 4-[(3-Ethinylphenyl)amino]-6-(1-methansulfonyl-piperidin-4-yloxy)-7-methoxy-chinazolin, 4-[(3-Chlor-4-fluor-phenyl)amino]-6-(1-methyl-piperidin-4-yloxy)-7(2-methoxy-ethoxy)-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(N-methyl-N-2-methoxyethyl-amino)carbonyl]-piperidin-4-yloxy}-7-methoxy-chinazolin, 4-[(3-Chlor-4-fluor-phenyl)amino]-6-(1-ethyl-piperidin-4-yloxy)-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methylamino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluorphenyl)amino]-6-[trans-4-(N-methansulfonyl-N-methyl-amino)-cyclohexan-1-yloxy]-7-methoxy-chinazolin , 4-[(3-Chlor-4-fluor-phenyl)amino]-6-(trans-4-dimethylaminocyclohexan-1-yloxy)-7-methoxy-chinazolin , 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluorphenyl)amino]-6-(1-methansulfonyl-piperidin-4-yloxy)-7-methoxy-chinazolin, 4-[(3-Chlor-4-fluor-phenyl)amino]-6-(1-cyano-piperidin-4-yloxy)-7-methoxy-chinazolin, und 4-[(3-Chlor-4-fluor-phenyl)amino]-6-{1-[(2-methoxyethyl)carbonyl]-piperidin-4-yloxy}-7-methoxy-chinazolin, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Erfindungsgemäß besonders bevorzugte Arzneimittelkombinationen enthalten als EGFR-Hemmer **2e** diejenigen Verbindungen, die ausgewählt sind aus der Gruppe bestehend aus
- 4-[(3-Chlor-4-fluorphenyl)amino]-6-{[4-(morpholin-4-yl)-1-oxo-2-buten-1-yl]amino}-7-cyclopropylmethoxy-chinazolin (**2e.1**),
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin (**2e.2**),
- 4-[(3-Chlor-4-fluor-phenyl)amino]-6-[2-((S)-6-methyl-2-oxo-morpholin-4-yl)-ethoxy]-7-methoxy-chinazolin (**2e.3**),
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin (**2e.4**),
- 4-[(3-Ethinyl-phenyl)amino]-6,7-bis-(2-methoxy-ethoxy)-chinazolin (**2e.5**),
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin (**2e.6**),
- 4-[(3-Ethinyl-phenyl)amino]-6-{[4-(5,5-dimethyl-2-oxo-morpholin-4-yl)-1-oxo-2-buten-1-yl]amino}-chinazolin (**2e.7**),
- 4-[(3-Chlor-4-fluor-phenyl)amino]-6-(trans-4-methansulfonylamino-cyclohexan-1-yloxy)-7-methoxy-chinazolin (**2e.8**),
- 4-[(3-Chlor-4-fluor-phenyl)amino]-6-(tetrahydropyran-3-yloxy)-7-methoxy-chinazolin (**2e.9**),
- 4-[(3-Chlor-4-fluor-phenyl)amino]-6- {1-[(morpholin-4-yl)carbonyl]-piperidin-4-yl-oxy}-7-methoxy-chinazolin (**2e.10**),
- 4-[(3-Chlor-4-fluor-phenyl)amino]-6- {1-[2-(2-oxopyrrolidin-1-yl)ethyl]-piperidin-4-yloxy}-7-methoxy-chinazolin (**2e.11**),
- 4-[(3-Ethinyl-phenyl)amino]-6-(1-acetyl-piperidin-4-yloxy)-7-methoxy-chinazolin (**2e.12**),
- 4-[(3-Ethinyl-phenyl)amino]-6-(1-methyl-piperidin-4-yloxy)-7-methoxy-chinazolin (**2e.13**),
- 4-[(3-Ethinyl-phenyl)amino]-6-(1-methansulfonyl-piperidin-4-yloxy)-7-methoxy-chinazolin (**2e.14**),
- 4-[(3-Ethinyl-phenyl)amino]-6-{1-[(morpholin-4-yl)carbonyl]-piperidin-4-yloxy}-7-methoxy-chinazo lin (**2e.15**),
- 4-[(3-Chlor-4-fluor-phenyl)amino]-6-{1-[(2-methoxyethyl)carbonyl]-piperidin-4-yloxy}-7-methoxy-chinazolin (**2e.16**),
- 4-[(3-Chlor-4-fluor-phenyl)amino]-6-[cis-4-(N-methansulfonyl-N-methyl-amino)-cyclohexan-1-yloxy]-7-methoxy-chinazolin (**2e.17**),
- 4-[(3-Chlor-4-fluor-phenyl)amino]-6-[cis-4-(N-acetyl-N-methyl-amino)-cyclohexan-1-yloxy]-7-methoxy-chinazolin (**2e.18**),
- 4-[(3-Chlor-4-fluor-phenyl)amino]-6-(trans-4-methylamino-cyclohexan-1-yloxy)-7-methoxy-chinazolin (**2e.19**),
- 4-[(3-Chlor-4-fluor-phenyl)amino]-6-[trans-4-(N-methansulfonyl-N-methyl-amino)-cyclohexan-1-yloxy]-7-methoxy-chinazolin (**2e.20**),
- 4-[(3-Chlor-4-fluor-phenyl)amino]-6-(trans-4-dimethylamino-cyclohexan-1-yloxy)-7-methoxy-chinazolin (**2e.21**),
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin (**2e.22**),
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin (**2e.23**),
- 4-[(3-Chlor-4-fluor-phenyl)amino]-6-(1-methansulfonyl-piperidin-4-yloxy)-7-methoxy-chinazolin (**2e.24**) und
- 4-[(3-Chlor-4-fluor-phenyl)amino]-6-(1-cyano-piperidin-4-yloxy)-7-methoxy-chinazolin (**2e.25**),
gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Unter Säureadditionssalzen mit pharmakologisch verträglichen Säuren zu deren Bildung die Verbindungen **2e** gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Beispiele für erfindungsgemäße bevorzugte Arzneimittelkombinationen bevorzugter Verbindungen der Formel **1** mit den vorstehend genannten EGFR-Hemmern **2e** sind Kombinationen enthaltend die Verbindungen **1.a** und **2e.1; 1.a** und **2e.2; 1.a** und **2e.3; 1.a** und **2e.4; 1.a** und **2e.5; 1.a** und **2e.6; 1.a** und **2e.7; 1.a** und **2e.8; 1.a** und **2e.9; 1.a** und **2e.10; 1.a** und **2e.11; 1.a** und **2e.12; 1.a** und **2e.13; 1.a** und **2e.14; 1.a** und **2e.15; 1.a** und **2e.16; 1.a** und **2e.17; 1.a** und **2e.18; 1.a** und **2e.19; 1.a** und **2e.20; 1.a** und **2e.21; 1.a** und **2e.22; 1.a** und **2e.23; 1.a** und **2e.24; 1.a** und **2e.25; 1.b** und **2e.1; 1.b** und **2e.2; 1.b** und **2e.3; 1.b** und **2e.4; 1.b** und **2e.5; 1.b** und **2e.6; 1.b** und **2e.7; 1.b** und **2e.8; 1.b** und **2e.9: 1.b** und **2e.10; 1.b** und **2e.11; 1.b** und **2e.12; 1.b** und **2e.13; 1.b** und **2e.14; 1.b** und **2e.15; 1.b** und **2e.16; 1.b** und **2e.17; 1.b** und **2e.18; 1.b** und **2e1.9; 1.b** und **2e.20; 1.b** und **2e.21; 1.b** und **2e.22; 1.b** und **2e.23; 1.b** und **2e.24; 1.b** und **2e.25; 1.d** und **2e.1; 1.d** und **2e.2; 1.d** und **2e.3; 1.d** und **2e.4; 1.d** und **2e.5; 1.d** und **2e.6; 1.d** und **2e.7; 1.d** und **2e.8; 1.d** und **2e.9; 1.d** und **2e.10; 1.d** und **2e.11; 1.d** und **2e.12; 1.d** und **2e.13; 1.d** und **2e.14; 1.d** und **2e.15; 1.d** und **2e.16; 1.d** und **2e.17; 1.d** und **2e.18; 1.d** und **2e.19; 1.d** und **2e.20; 1.d** und **2e.21; 1.d** und **2e.22; 1.d** und **2e.23; 1.d** und **2e.24; 1.d** und **2e.25; 1.f** und **2e.1. 1.f** und **2e.2; 1.f** und **2e.3; 1.f** und **2e.4; 1.f** und **2e.5; 1.f** und **2e.6; 1.f** und **2e.7; 1.f** und **2e.8; 1.f** und **2e.9; 1.f** und **2e.10; 1.f und 2e.11; 1.f** und **2e.12; 1.f** und 2e.13; **1.f** und **2e.14; 1.f** und **2e.15; 1.f** und **2e.16; 1.f und 2e.17; 1.f** und **2e.18; 1.f** und **2e.19; 1.f und 2e.20; 1.f** und **2e.21; 1.f** und **2e.22; 1.f und 2e.23; 1.f** und **2e.24; 1.f** und **2e.25; 1.h** und **2e.1; 1.h** und **2e.2; 1.h** und **2e.3; 1.h** und **2e.4; 1.h** und **2e.5; 1.h** und **2e.6; 1.h** und **2e.7; 1.h** und **2e.8; 1.h** und **2e.9; 1.h** und **2e.10; 1.h** und **2e.11; 1.h** und **2e.12; 1.h** und **2e.13; 1.h** und **2e.14; 1.h** und **2e.15; 1.h** und **2e.16; 1.h** und **2e.17; 1.h** und **2e.18; 1.h** und **2e.19; 1.h** und **2e.20; 1.h** und **2e.21; 1.h** und **2e.22; 1.h** und **2e.23; 1.h** und **2e.24; 1.h** und **2e.25; 1.j** und **2e.1; 1.j** und **2e.2; 1.j** und **2e.3; 1.j** und **2e.4; 1.j** und **2e.5; 1.j** und **2e.6; 1.j** und **2e.7; 1.j** und **2e.8; 1.j** und **2e.9; 1.j** und **2e.10; 1.j** und **2e.11; 1.j** und **2e.12; 1.j** und **2e.13; 1.j** und **2e.14; 1.j** und **2e.15; 1.j** und **2e.16; 1.j** und **2e.17; 1.j** und **2e.18; 1.j** und **2e.19; 1.j** und **2e.20; 1.j** und **2e.21; 1.j** und **2e.22; 1.j** und **2e.23; 1.j** und **2e.24; 1.j** und **2e.25; 1.k** und **2e.1; 1.k** und **2e.2; 1.k** und **2e.3; 1.k** und **2e.4; 1.k** und **2e.5; 1.k** und **2e.6; 1.k** und **2e.7; 1.k** und **2e.8; 1.k** und **2e.9; 1.k** und **2e.10; 1.k** und **2e.11; 1.k** und **2e.12; 1.k** und **2e.13; 1.k** und **2e.14; 1.k** und **2e.15; 1.k** und **2e.16; 1.k** und **2e.17; 1.k** und **2e.18; 1.k** und **2e.19; 1.k** und **2e.20; 1.k** und **2e.21; 1.k** und **2e.22; 1.k** und **2e.23; 1.k** und **2e.24; 1.k** und **2e.25; 1.l** und **2e.1; 1.l** und **2e.2; 1.l** und **2e.3; 1.l** und **2e.4; 1.l** und **2e.5; 1.l** und **2e.6; 1.l** und **2e.7; 1.l** und **2e.8; 1.l** und **2e.9; 1.l** und **2e.10; 1.l** und **2e.11; 1.l** und **2e.12; 1.l** und **2e.13; 1.l** und **2e.14; 1.l** und **2e.15; 1.l** und **2e.16; 1.l** und **2e.17; 1.l** und **2e.18; 1.l** und **2e.19; 1.l** und **2e.20; 1.l** und **2e.21; 1.l** und **2e.22; 1.l** und **2e.23; 1.l** und **2e.24; 1.l** und **2e.25. 1.m** und **2e.1; 1.m** und **2e.2; 1.m** und **2e.3; 1.m** und **2e.4; 1.m** und **2e.5; 1.m** und **2e.6; 1.m** und **2e.7; 1.m** und **2e.8; 1.m** und **2e.9; 1.m** und **2e.10; 1.m** und **2e.11; 1.m** und **2e.12; 1.m** und **2e.13; 1.m** und **2e.14; 1.m** und **2e.15; 1.m** und **2e.16; 1.m** und **2e.17; 1.m** und **2e.18; 1.m** und **2e.19; 1.m** und **2e.20; 1.m** und **2e.21; 1.m** und **2e.22; 1.m** und **2e.23; 1.m** und **2e.24; 1.m** und **2e.25; 1.q** und **2e.1; 1.q** und **2e.2; 1.q** und **2e.3; 1.q** und **2e.4; 1.q** und **2e.5; 1.q** und **2e.6; 1.q** und **2e.7; 1.q** und **2e.8; 1.q** und **2e.9; 1.q** und **2e.10; 1.q** und **2e.11; 1.q** und **2e.12;** und **2e.13; 1.q** und **2e.14; 1.q** und **2e.15; 1.q** und **2e.16, 1.q** und **2e.17; 1.q** und **2e.18; 1.q** und **2e.19; 1.q** und **2e.20; 1.q** und **2e.21; 1.q** und **2e.22; 1.q** und **2e.23; 1.q** und **2e.24** oder **1.q** und **2e.25,** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Von den vorstehend genannten Kombinationen sind erfindungsgemäß diejenigen bevorzugt, die als Verbindung der Formel **1** eine der Verbindungen **1.a**, **1.h**, **1.j**, oder **1.k** enthalten, wobei jene Kombinationen, die eine der Verbindungen **1.h** oder **1.k** enthalten, erfindungsgemäß besonders bedeutsam sind. Von den vorstehend genannten Kombinationen sind erfindungsgemäß ferner diejenigen bevorzugt, die als Verbindung **2e** eine der Verbindungen 2e.1, **2e.2, 2e.3, 2e.4, 2e.10, 2e.11, 2e.14, 2e.16, 2e.17, 2e.18, 2e.19, 2e.20, 2e.21, 2e.22, 2e.23, 2e.24** oder **2e.25** enthalten, wobei jene Kombinationen, die eine der Verbindungen **2e.2, 2e.3** oder **2e.4** enthalten, erfindungsgemäß besonders bedeutsam sind.

Die erfindungsgemäßen Arzneimittelkombinationen aus Verbindungen der Formel **1** mit wenigstens einem weiteren Wirkstoff **2** sind nicht beschränkt auf binäre Wirkstoffkombinationen. Die vorstehend teils exemplarisch genannten Kombinationen, die neben einer Verbindung der Formel **1** einen weiteren Wirkstoff **2** enthalten, können ferner einen dritten oder vierten, vorzugsweise einen dritten Wirkstoff enthalten, der ebenfalls aus der vorstehend genannten Gruppe von Anticholinergika (**2a**), PDEIV-Inhibitoren **(2b),** Steroide (**2c**), LTD4-Antagonisten (**2d**) und EGFR-Hemmern (**2e**) ausgewählt ist.

Besonders bevorzugte Kombinationen, die neben einer Verbindung der Formel **1** 2 weitere Wirkstoffe enthalten sind ausgewählt aus den nachstehend aufgeführten Wirkstoffkombinationen. Es sind dies Arzneimittelkombinationen, die beispielsweise enthalten können:
A) eine Verbindung der Formel **1,** ein Anticholinergikum (**2a**), ein PDEIV-Inhibitor (**2b**);
B) eine Verbindung der Formel **1,** ein Anticholinergikum (**2a**), ein Steroid (**2c**);
C) eine Verbindung der Formel **1,** ein Anticholinergikum **(2a),** ein LTD4-Antagonist **(2d);**
D) eine Verbindung der Formel **1,** ein Anticholinergikum **(2a),** ein EGFR-Hemmer **(2e);**
E) eine Verbindung der Formel **1,** ein PDEIV-Inhibitor **(2b),** ein Steroid **(2c);**
F) eine Verbindung der Formel **1,** ein PDEIV-Inhibitor **(2b),** ein LTD4-Antagonist **(2d);**
G) eine Verbindung der Formel **1,** ein PDEIV-Inhibitor **(2b),** ein EGFR-Hemmer **(2e);**
H) eine Verbindung der Formel **1,** ein Steroid **(2c),** ein LTD4-Antagonist **(2d);**
I) eine Verbindung der Formel **1,** ein Steroid **(2c),** ein EGFR-Hemmer **(2e);**
J) eine Verbindung der Formel **1,** ein LTD4-Antagonist **(2d),** ein EGFR-Hemmer (**2e**).

Besonders bevorzugte Beispiele für Arzneimittelkombinationen der o.g. Gruppe A, sind ausgewählt aus der Gruppe bestehend aus den folgenden Kombinationen:
Verbindungen **1.h** und **2a.1** und **2b.2; 1.h** und **2a.1** und **2b.4; 1.h** und **2a.1** und **2b.11; 1.h** und **2a.1** und **2b.19; 1.h** und **2a.9.1** und **2b.2; 1.h** und **2a.9.1** und **2b.4; 1.h** und **2a.9.1** und **2b.11; 1.h** und **2a.9.1** und **2b.19; 1.h** und **2a.9.2** und **2b.2; 1.h** und **2a.9.2** und **2b.4; 1.h** und **2a.9.2** und **2b.11; 1.h** und **2a.9.2** und **2b.19; 1.h** und **2a.10.1** und **2b.2; 1.h** und **2a.10.1** und **2b.4; 1.h** und **2a.10.1** und **2b.11; 1.h** und **2a.10.1** und **2b.19; 1.h** und **2a.10.2** und **2b.2; 1.h** und **2a.10.2** und **2b.4; 1.h** und **2a.10.2** und **2b.11; 1.h** und **2a.10.2** und **2b.19; 1.h** und **2a.11.1** und **2b.2; 1.h** und **2a.11.1** und **2b.4; 1.h** und **2a.11.1** und **2b.11; 1.h** und **2a.11.1** und **2b.19; 1.h** und **2a.11.6** und **2b.2; 1.h** und **2a.11.6** und **2b.4; 1.h** und **2a.11.6** und **2b.11; 1.h** und **2a.11.6** und **2b.19; 1.k** und **2a.1** und **2b.2; 1.k** und **2a.1** und **2b.4;_1.k** und **2a.1** und **2b.11; 1.k** und **2a.1** und **2b.19; 1.k** und **2a.9.1** und **2b.2; 1.k** und **2a.9.1** und **2b.4; 1.k** und **2a.9.1** und **2b.11; 1.k** und **2a.9.1** und **2b.19; 1.k** und **2a.9.2** und **2b.2; 1.k** und **2a.9.2** und **2b.4; 1.k** und **2a.9.2** und **2b.11; 1.k** und **2a.9.2** und **2b.19; 1.k** und **2a.10.1** und **2b.2; 1.k** und **2a.10.1** und **2b.4; 1.k** und **2a.10.1** und **2b.1.1; 1.k** und **2a.10.1** und **2b.19; 1.k** und **2a.10.2** und **2b.2; 1.k** und **2a.10.2** und **2b.4; 1.k** und **2a.10.2** und 2b.11; 1.k und **2a.10.2** und **2b.19; 1.k** und **2a.11.1** und **2b.2; 1.k** und **2a.11.1** und **2b.4; 1.k** und **2a.11.1** und **2b.11; 1.k** und **2a.11.1** und **2b.19; 1.k** und **2a.11.6** und **2b.2; 1.k** und **2a.11.6** und **2b.4; 1.k** und **2a.11.6** und **2b.11; 1.k** und **2a.11.6** und **2b.19,** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugte Beispiele für erfindungsgemäß besonders bevorzugte Arzneimittelkombinationen der o.g. Gruppe B, sind ausgewählt aus der Gruppe bestehend aus den folgenden Kombinationen:
Verbindungen **1.h** und **2a.1** und **2c.8; 1.h** und **2a.1** und **2c.9; 1.h** und **2a.1** und **2c.10; 1.h** und **2a.1** und **2c.11; 1.h** und **2a.1** und **2c.17; 1.h** und **2a.9.1** und **2c.8; 1.h** und **2a.9.1** und **2c.9; 1.h** und **2a.9.1** und **2c.10; 1.h** und **2a.9.1** und **2c.11; 1.h** und **2a.9.1** und **2c.17; 1.h** und **2a.9.2** und **2c.8; 1.h** und **2a.9.2** und **2c.9; 1.h** und **2a.9.2** und **2c.10; 1.h** und **2a.9.2** und **2c.11; 1.h** und **2a.9.2** und **2c.17; 1.h** und **2a.10.1** und **2c.8; 1.h** und **2a.10.1** und **2c.9; 1.h** und **2a.10.1** und **2c.10; 1.h** und **2a.10.1** und **2c.11; 1.h** und **2a.10.1** und **2c.17; 1.h** und **2a.10.2** und **2c.8; 1.h** und **2a.10.2** und **2c.9; 1.h** und **2a.10.2** und **2c.10; 1.h** und **2a.10.2** und **2c.11; 1.h** und **2a.10.2** und **2c.17; 1.h** und **2a.11.1** und **2c.8; 1.h** und **2a.11.1** und **2c.9; 1.h** und **2a.11.1** und **2c.10; 1.h** und **2a.11.1** und **2c.11; 1.h** und **2a.11.1** und **2c.17; 1.h** und **2a.11.6** und **2c.8; 1.h** und **2a.11.6** und **2c.9; 1.h** und **2a.11.6** und **2c.10; 1.h** und **2a.11.6** und **2c.11; 1.h** und **2a.11.6** und **2c.17; 1.k** und **2a.1** und **2c.8; 1.k** und **2a.1** und **2c.9; 1.k** und **2a.1** und **2c.10; 1.k** und 2a.1 und 2c.11; 1.k und 2a.1 und 2c.17; **1.k** und **2a.9.1** und **2c.8; 1.k** und **2a.9.1** und **2c.9; 1.k** und **2a.9.1** und **2c.10;_1.k** und **2a.9.1** und **2c.11; 1.k** und **2a.9.1** und **2c.17; 1.k** und **2a.9.2** und **2c.8; 1.k** und **2a.9.2** und **2c.9; 1.k** und **2a.9.2** und **2c.10; 1.k** und **2a.9.2** und **2c.11; 1.k** und **2a.9.2** und **2c.17; 1.k** und **2a.10.1** und **2c.8; 1.k** und **2a.10.1** und **2c.9; 1.k** und **2a,10,1** und **2c,10;** **1.k** und **2a.10.1** und **2c.11; 1.k** und **2a.10.1** und **2c.17; 1.k** und **2a.10.2** und **2c.8; 1.k** und **2a.10.2** und **2c.9; 1.k** und **2a,10.2** und **2c.10; 1.k** und **2a.10.2** und **2c.11; 1.k** und **2a.10.2** und **2c.17; 1.k** und **2a.11.1** und **2c.8; 1.k** und **2a.11.1** und **2c.9; 1.k** und **2a.11.1** und **2c.10;_1.k** und **2a.11.1** und **2c.11; 1.k** und **2a.11.1** und **2c.17; 1.k** und **2a.11.6** und **2c.8; 1.k** und **2a.11.6** und **2c.9; 1.k** und **2a.11.6** und **2c.10; 1.k** und **2a.11.6** und **2c.11; 1.k** und **2a.11.6** und **2c.17"** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.:

Besonders bevorzugte Beispiele für Arzneimittelkombinationen der o.g. Gruppe C, sind ausgewählt aus der Gruppe bestehend aus den folgenden Kombinationen:
Verbindungen **1.h** und **2a.1** und **2d.1; 1.h** und **2a.1** und **2d.4; 1.h** und **2a.1** und **2d.5; 1.h** und **2a.1** und **2d.8; 1.h** und **2a.9.1** und **2d.1; 1.h** und **2a.9.1** und **2d.4; 1.h** und **2a.9.1** und **2d.5; 1.h** und **2a.9.1** und **2d.8; 1.h** und **2a.9.2** und **2d.1; 1.h** und **2a.9.2** und **2d.4; 1.h** und **2a.9.2** und **2d.5; 1.h** und **2a.9.2** und **2d.8; 1.h** und **2a.10.1** und **2d.1; 1.h** und **2a.10.1** und **2d.4; 1.h** und **2a.10.1** und **2d.5; 1.h** und **2a.10.1** und **2d.8; 1.h** und **2a.10.2** und **2d.1; 1.h** und **2a.10.2** und **2d.4; 1.h** und **2a.10.2** und **2d.5; 1.h** und **2a.10.2** und **2d.8; 1.h** und **2a.11.1** und **2d.1; 1.h** und **2a.11.1** und **2d.4; 1.h** und **2a.11.1** und **2d.5; 1.h** und **2a.11.1** und **2d.8; 1.h** und **2a.11.6** und **2d.1; 1.h** und **2a.11.6** und **2d.4; 1.h** und **2a.11.6** und **2d.5; 1.h** und **2a.11.6** und **2d.8; 1.k** und **2a.1** und **2d.1; 1.k** und **2a.1** und **2d.4; 1.k** und **2a.1** und **2d.5; 1.k** und **2a.1** und **2d.8; 1.k** und **2a.9.1** und **2d.1; 1.k** und **2a.9.1** und **2d.4; 1.k** und **2a.9.1** und **2d.5; 1.k** und **2a.9.1** und **2d.8; 1.k** und **2a.9.2** und **2d.1; 1.k** und **2a.9.2** und **2d.4; 1.k** und **2a.9.2** und **2d.5; 1,k** und **2a.9.2** und **2d.8; 1.k** und **2a.10.1** und **2d.1; 1.k** und **2a.10.1** und **2d.4; 1.k** und **2a.10.1** und **2d.5; 1.k** und **2a.10.1** und **2d.8; 1.k** und **2a.10.2** und **2d.1; 1.k** und **2a.10.2** und **2d.4; 1.k** und **2a.10.2** und **2d.5; 1.k** und **2a.10.2** und **2d.8; 1.k** und **2a.11.1** und **2d.1; 1.k** und **2a.11.1** und **2d.4; 1.k** und **2a.11.1** und **2d.5; 1.k** und **2a.11.1** und **2d.8; 1.k** und **2a.11.6** und **2d.1; 1.k** und **2a.11.6** und **2d.4; 1.k** und **2a.11.6** und **2d.5; 1.k** und **2a.11.6** und **2d.8,** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.:

Besonders bevorzugte Beispiele für Arzneimittelkombinationen der o.g. Gruppe D, sind ausgewählt aus der Gruppe bestehend aus den folgenden Kombinationen:
Verbindungen **1.h** und **2a.1** und **2e.2; 1.h** und **2a.1** und **2e.3; 1.h** und **2a.1** und **2e.4; 1.h** und **2a.1** und **2e.10; 1.h** und **2a.9.1** und **2e.2; 1.h** und **2a.9.1** und **2e.3; 1.h** und **2a.9.1** und **2e.4; 1.h** und **2a.9.1** und **2e.10; 1.h** und **2a.9.2** und **2e.2; 1.h** und **2a.9.2** und **2e.3; 1.h** und **2a.9.2** und **2e.4; 1.h** und **2a.9.2** und **2e.10; 1.h** und **2a.10.1** und **2e.2; 1.h** und **2a.10.1** und **2e.3; 1.h** und **2a.10.1** und **2e.4; 1.h** und **2a.10.1** und **2e.10; 1.h** und **2a.10.2** und **2e.2; 1.h** und **2a.10.2** und **2e.3; 1.h** und **2a.10.2** und **2e.4; 1.h** und **2a.10.2** und **2e.10; 1.h** und **2a.11.1** und **2e.2; 1.h** und **2a.11.1** und **2e.3; 1.h** und **2a.11.1** und **2e.4; 1.h** und **2a.11.1** und **2e.10; 1.h** und **2a.11.6** und **2e.2; 1.h** und **2a.11.6** und **2e.3; 1.h** und **2a.11.6** und **2e.4; 1.h** und **2a.11.6** und **2e.10; 1.k** und **2a.1** und **2e.2; 1.k** und **2a.1** und **2e.3; 1.k** und **2a.1** und **2e.4;_1.k** und **2a.1** und **2e.10; 1.k** und **2a.9.1** und **2e.2; 1.k** und **2a.9.1** und **2e.3; 1.k** und **2a.9.1** und **2e.4;1.k** und **2a.9.1** und **2e.10; 1.k** und **2a.9.2** und **2e.2; 1.k** und **2a.9.2** und **2e.3; 1.k** und **2a.9.2** und **2e.4; 1.k** und **2a.9.2** und **2e.10; 1.k** und **2a.10.1** und **2e.2; 1.k** und **2a.10.1** und **2e.3; 1.k** und **2a.10.1** und **2e.4; 1.k** und **2a.10.1** und **2e,10; 1.k** und **2a.10.2** und **2e.2; 1.k** und **2a.10.2** und **2e.3;_1.k** und **2a.10.2** und **2e.4; 1.k** und **2a.10.2** und **2e.10; 1.k** und **2a.11.1** und **2e.2; 1.k** und **2a.11.1** und **2e.3; 1.k** und **2a.11.1** und **2e.4; 1.k** und **2a.11.1** und **2e.10; 1.k** und **2a.11.6** und **2e.2; 1.k** und **2a.11.6** und **2e.3; 1.k** und **2a.11.6** und **2e.4; 1.k** und **2a.11.6** und **2e.10**, jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.:

Besonders bevorzugte Beispiele für Arzneimittelkombinationen der o.g. Gruppe E, sind ausgewählt aus der Gruppe bestehend aus den folgenden Kombinationen:
Verbindungen **1.h** und **2c.8** und **2b.2; 1.h** und **2c.8** und **2b.4; 1.h** und **2c.8** und **2b.11; 1.h** und **2c.8** und **2b.19; 1.h** und **2c.9** und **2b.2; 1.h** und **2c.9** und **2b.4; 1.h** und **2c.9** und **2b.11; 1.h** und **2c.9** und **2b.19; 1.h** und **2c.10** und **2b.2; 1.h** und **2c.10** und **2b.4; 1.h** und **2c.10** und **2b.11; 1.h** und **2c.10** und **2b.19; 1.h** und **2c.11** und **2b.2; 1.h** und **2c.11** und **2b.4; 1.h** und **2c.11** und **2b.11; 1.h** und **2c.11** und **2b.19; 1.h** und **2c.17** und **2b.2; 1.h** und **2c.17** und **2b.4; 1.h** und **2c.17** und **2b.11; 1.h** und **2c.17** und **2b.19; 1.k** und **2c.8** und **2b.2; 1.k** und **2c.8** und **2b.4; 1.k** und **2c.8** und **2b.11; 1.k** und **2c.8** und **2b.19; 1.k** und **2c.9** und **2b.2; 1.k** und **2c.9** und **2b.4; 1.k** und **2c.9** und **2b.11; 1.k** und **2c.9** und **2b.19; 1.k** und **2c.10** und **2b.2; 1.k** und **2c.10** und **2b.4; 1.k** und **2c.10** und **2b.11; 1.k** und **2c.10** und **2b.19; 1.k** und **2c.11** und **2b.2; 1.k** und **2c.11** und **2b.4; 1.k** und **2c.11** und **2b.11; 1.k** und **2c.1** und **2b.19, 1.k** und **2c.17** und **2b.2; 1.k** und **2c.17** und **2b.4; 1.k** und **2c.17** und **2b.11; 1.k** und **2c.17** und **2b.19;** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugte Beispiele für Arzneimittelkombinationen der o.g. Gruppe F, sind ausgewählt aus der Gruppe bestehend den folgenden Kombinationen:
Verbindungen **1.h** und **2d.1** und **2b.2; 1.h** und **2d.1** und **2b.4; 1.h** und **2d.1** und **2b.11; 1.h** und **2d.1** und **2b.19; 1.h** und **2d.4** und **2b.2; 1.h** und **2d.4** und **2b.4; 1.h** und **2d.4** und **2b.11; 1.h** und **2d.4** und **2b.19; 1.h** und **2d.5** und **2b.2; 1.h** und **2d.5** und **2b.4; 1.h** und **2d.5** und **2b.11; 1.h** und **2d.5** und **2b.19; 1.h** und **2d.8** und **2b.2; 1.h** und **2d.8** und **2b.4; 1.h** und **2d.8** und **2b.11; 1.h** und **2d.8** und **2b.19; 1.k** und **2d.1** und **2b.2; 1.k** und **2d.1** und **2b.4; 1.k** und **2d.1** und **2b.11; 1.k** und **2d.1** und **2b.19; 1.k** und **2d.4** und **2b.2; 1.k** und **2d.4** und **2b.4; 1.k** und **2d.4** und **2b.11; 1.k** und **2d.4** und **2b.19; 1.k** und **2d.5** und **2b.2; 1.k** und **2d.5** und **2b.4; 1.k** und **2d.5** und **2b.11; 1.k** und **2d.5** und **2b.19; 1.k** und **2d.8** und **2b.2; 1.k** und **2d.8** und **2b.4; 1.k** und **2d.8** und **2b.11;_1.k** und **2d.8** und **2b.19,** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugte Beispiele für Arzneimittelkombinationen der o.g. Gruppe G, sind ausgewählt aus der Gruppe bestehend aus den folgenden Kombinationen:
Verbindungen **1.h** und **2e.2** und **2b.2; 1.h** und **2e.2** und **2b.4; 1.h** und **2e.2** und **2b.11; 1.h** und **2e.2** und **2b.19; 1.h** und **2e.3** und **2b.2; 1.h** und **2e.3** und **2b.4; 1.h** und **2e.3** und **2b.11; 1.h** und **2e.3** und **2b.19; 1.h** und **2e.4** und **2b.2; 1.h** und **2e.4** und **2b.4; 1.h** und **2e.4** und **2b.11; 1.h** und **2e.4** und **2b.19; 1.h** und **2e.10** und **2b.2; 1.h** und **2e.10** und **2b.4; 1.h** und **2e.10** und **2b.11; 1.h** und **2e.10** und **2b.19; 1.k** und **2e.2** und **2b.2; 1.k** und **2e.2** und **2b.4;** **1.k** und **2e.2** und **2b.11; 1.k** und **2e.2** und **2b.19; 1.k** und **2e.3** und **2b.2; 1.k** und **2e.3** und **2b.4; 1.k** und **2e.3** und **2b.11; 1.k** und **2e.3** und **2b.19; 1.k** und **2e.4** und **2b.2; 1.k** und **2e.4** und **2b.4; 1.k** und **2e.4** und **2b.11; 1.k** und 2e.4 und **2b.19; 1.k** und **2e.10** und **2b.2; 1.k** und **2e.10** und **2b.4; 1.k** und **2e.10** und **2b.11; 1.k** und **2e.10** und **2b.19,** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugte Beispiele für Arzneimittelkombinationen der o.g. Gruppe H, sind ausgewählt aus der Gruppe bestehend aus den folgenden Kombinationen:
Verbindungen **1.h** und **2c.8** und **2d.1; 1.h** und **2c.8** und **2d.4; 1.h** und **2c.8** und **2d.5; 1.h** und **2c.8** und **2d.8; 1.h** und **2c.9** und **2d.1; 1.h** und **2c.9** und **2d.4; 1.h** und **2c.9** und **2d.5; 1.h** und **2c.9** und **2d.8; 1.h** und **2c.10** und **2d.1; 1.h** und **2c.10** und **2d.4; 1.h** und **2c.10** und **2d.5; 1.h** und **2c.10** und **2d.8; 1.h** und **2c.11** und **2d.1; 1.h** und **2c.11** und **2d.4; 1.h** und **2c.11** und **2d.5; 1.h** und **2c.11** und **2d.8; 1.h** und **2c.17** und **2d.1; 1.h** und **2c.17** und **2d.4; 1.h** und **2c.17** und **2d.5; 1.h** und **2c.17** und **2d.8; 1.k** und **2c.8** und **2d.1; 1.k** und **2c.8** und **2d.4; 1.k** und **2c.8** und **2d.5; 1.k** und **2c.8** und **2d.8; 1.k** und **2c.9** und **2d.1; 1.k** und **2c.9** und **2d.4; 1.k** und **2c.9** und **2d.5; 1.k** und **2c.9** und **2d.8; 1.k** und **2c.10** und **2d.1; 1.k** und **2c,10** und **2d.4;** **1.k** und **2c.10** und **2d.5; 1.k** und **2c.10** und **2d.8; 1.k** und **2c.11** und **2d.1; 1.k** und **2c.11** und **2d.4; 1.k** und **2c.11** und **2d.5; 1.k** und **2c.11** und **2d.8, 1.k** und **2c.17** und **2d.1; 1.k** und **2c.17** und **2d.4; 1.k** und **2c.17** und **2d.5; 1.k** und **2c.17** und **2d.8;** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugte Beispiele für Arzneimittelkombinationen der o.g. Gruppe I, sind ausgewählt aus der Gruppe bestehend aus den folgenden Kombinationen:
Verbindungen **1.h** und **2c.8** und **2e.2; 1.h** und **2c.8** und **2e.3; 1.h** und **2c.8** und **2e.4; 1.h** und **2c.8** und **2e.10; 1.h** und **2c.9** und **2e.2; 1.h** und **2c.9** und **2e.3; 1.h** und **2c.9** und **2e.4; 1.h** und **2c.9** und **2e.10; 1.h** und **2c.10** und **2e.2; 1.h** und **2c.10** und **2e.3; 1.h** und **2c.10** und **2e.4; 1.h** und **2c.10** und **2e.10; 1.h** und **2c.11** und **2e.2; 1.h** und **2c.11** und **2e.3; 1.h** und **2c.11** und **2e.4; 1.h** und **2c.11** und **2e.10; 1.h** und **2c.17** und **2e.2; 1.h** und **2c.17** und **2e.3; 1.h** und **2c.17** und **2e.4; 1.h** und **2c.17** und **2e.10; 1.k** und **2c.8** und **2e.2; 1.k** und **2c.8** und **2e.3; 1.k** und **2c.8** und **2e.4; 1.k** und **2c.8** und **2e.10; 1.k** und **2c.9** und **2e.2; 1.k** und **2c.9** und **2e.3; 1.k** und **2c.9** und **2e.4; 1.k** und **2c.9** und **2e.10; 1.k** und **2c.10** und **2e.2; 1.k** und **2c.10** und **2e.3; 1.k** und **2c.10** und **2e.4; 1.k** und **2c.10** und **2e.10; 1.k** und **2c.11** und **2e.2; 1.k** und **2c.11** und **2e.3; 1.k** und **2c.11** und **2e.4; 1.k** und **2c.11** und **2e.10, 1.k** und **2c.17** und **2e.2; 1.k** und **2c.17** und **2e.3; 1.k** und **2c.17** und **2e.4; 1.k** und **2c.17** und **2e.10;** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Besonders bevorzugte Beispiele für Arzneimittelkombinationen der o.g. Gruppe J, sind ausgewählt aus der Gruppe bestehend aus den folgenden Kombinationen:
Verbindungen **1.h** und **2d.1** und **2e.2; 1.h** und **2d.1** und **2e.3; 1.h** und **2d.1** und **2e.4; 1.h** und **2d.1** und **2e.10; 1.h** und **2d.4** und **2e.2; 1.h** und **2d.4** und **2e.3; 1.h** und **2d.4** und **2e.4; 1.h** und **2d.4** und **2e.10; 1.h** und **2d.5** und **2e.2; 1.h** und **2d.5** und **2e.3; 1.h** und **2d.5** und **2e.4; 1.h** und **2d.5** und **2e.10; 1.h** und **2d.8** und **2e.2; 1.h** und **2d.8** und **2e.3; 1.h** und **2d.8** und **2e.4; 1.h** und **2d.8** und **2e.10; 1.k** und **2d.1** und **2e.2; 1.k** und **2d.1** und **2e.3; 1.k** und **2d.1** und **2e.4; 1.k** und **2d.1** und **2e.10; 1.k** und **2d.4** und **2e.2; 1.k** und **2d.4** und **2e.3; 1.k** und **2d.4** und **2e.4; 1.k** und **2d.4** und **2e.10; 1.k** und **2d.5** und **2e.2; 1.k** und **2d.5** und **2e.3; 1.k** und **2d.5** und **2e.4; 1.k** und **2d.5** und **2e.10; 1.k** und **2d.8** und **2e.2; 1.k** und **2d.8** und **2e.3; 1.k** und **2d.8** und **2e.4; 1.k** und **2d.8** und **2e.10,** jeweils gegebenenfalls in Form ihrer Racemate, Enantiomere oder Diastereomere und gegebenenfalls in Form ihrer pharmakologisch verträglichen Säureadditionssalze, Solvate und/oder Hydrate.

Erfindungsgemäß von herausragender Bedeutung sind all diejenigen im Rahmen der vorliegenden Erfindung offenbarten Arzneimittelkombinationen, die die Verbindungen der Formel 1 in Form Ihrer R-Enantiomere enthalten.

Halogen steht im Rahmen der vorliegenden Erfindung für Fluor, Chlor, Brom oder Jod. Sofern nicht gegenteilig angegeben, gelten Fluor und Brom als bevorzugte Halogene, wobei Fluor im Allgemeinen bevorzugt ist.

Als Alkylgruppen (Alkyl) werden, soweit nicht anders angegeben, verzweigte und unverzweigte Alkylgruppen mit 1 bis 6, bevorzugt 1 bis 4 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Als Alkylengruppen (Alkylen) werden, soweit nicht anders angegeben, verzweigte und unverzweigte Alkylengruppen mit 1 bis 6, bevorzugt 1 bis 4 Kohlenstoffatomen bezeichnet. Beispielsweise werden genannt: Methylen, Ethylen, Propylen oder Butylen. Sofern nicht anders beschrieben, umfassen die Definitionen Propylen und Butylen alle denkbaren isomeren Formen der jeweiligen Reste.

Als Cycloalkylgruppen (Cycloalkyl) werden, soweit nicht anders angegeben cyclische Alkylgruppen mit 3 bis 6 bezeichnet. Beispielsweise werden genannt: Cyclopropyl, Cyclobutanyl, Cyclopentyl oder Cyclohexyl.

Als Alkyloxygruppen (O-Alkyl) werden, soweit nicht anders angegeben, verzweigte und unverzweigte Alkylgruppen mit 1 bis 6, bevorzugt 1 bis 4 Kohlenstoffatomen bezeichnet, die über ein Sauerstoffatom verknüpft sind. Beispielsweise werden genannt: Methyloxy, Ethyloxy, Propyloxy oder Butyloxy. Zur Bezeichnung der Gruppen Methyloxy, Ethyloxy, Propyloxy oder auch Butyloxy werden gegebenenfalls auch die Abkürzungen -OMe, -OEt, -OProp oder -OBu verwendet. Sofern nicht anders beschrieben, umfassen die Definitionen Propyloxy und Butyloxy alle denkbaren isomeren Formen der jeweiligen Reste. So umfasst beispielsweise Propyloxy *n*-Propyloxy und *iso*-Propyloxy, Butyloxy umfasst *iso*-Butyloxy, *sec-*Butyloxy und *tert*-Butyloxy etc. Gegebenenfalls wird im Rahmen der vorliegenden Erfmdung statt der Bezeichnung Alkyloxy auch die Bezeichnung Alkoxy verwendet. Zur Bezeichnung der Gruppen Methyloxy, Ethyloxy, Propyloxy oder auch Butyloxy gelangen dementsprechend gegebenenfalls auch die Ausdrücke Methoxy, Ethoxy, Propoxy oder Butoxy zur Anwendung.

Als Halogenalkylengruppen werden, soweit nicht anders angegeben, verzweigte und unverzweigte Alkylgruppen mit 1 bis 6 Kohlenstoffatomen bezeichnet, bei denen ein oder mehrere Wasserstoffatome durch Halogenatome, bevorzugt durch Fluor ersetzt sind. Beispielsweise werden genannt: CHF₂, CF₃, CH₂CF₃, CF₂CF₃.

Als Arylgruppen werden, soweit nicht anders angegeben, aromatische Ringsysteme mit 6 bis 10 Kohlenstoffatomen bezeichnet. Bevorzugte Arylgruppen sind Phenyl und Naphthyl, wobei Phenyl erfindungsgemäß besonders bevorzugt ist.

Als Arylalkylengruppen werden, soweit nicht anders angegeben, vorstehend genannte Arylgruppen bezeichnet, die über verzweigte und unverzweigte Alkylgruppen mit 1 bis 4 Kohlenstoffatomen verknüpft sind. Beispielsweise werden genannt Benzyl, Phenylethyl, Naphthylmethyl, Naphthylethyl. Die verbrückenden Alkylgruppen werden im Rahmen der vorliegenden Erfindung auch als Alkylenbrücken bezeichnet.

Als Aryloxygruppen (O-Aryl) werden, soweit nicht anders angegeben, Arylgruppen mit 6 bis 10 Kohlenstoffatomen bezeichnet, die über eine Sauerstoffbrücke verknüpft sind. Bevorzugte Gruppen sind in diesem Zusammenhang beispielsweise Phenyloxy oder Naphthyloxy, die im Rahmen der vorliegenden Erfindung gegebenenfalls auch als Phenoxy oder Naphthoxy bezeichnet werden können.

Als Arylalkylenoxygruppen (Arylalkylen-O-) werden, soweit nicht anders angegeben, Arylgruppen bezeichnet, die über verzweigte und unverzweigte Alkyloxygruppen mit 1 bis 4 Kohlenstoffatomen verknüpft sind. Beispielsweise werden genannt Benzyloxy, Phenylethyloxy, Naphthylmethyloxy, Naphthylethyloxy.

Im Rahmen der vorliegenden Erfindung wird unter Arzneimittelkombination der Komponenten **1** und **2** die gemeinsame Applikation beider Wirkstoffe in einer einzigen Darreichungsform bzw. Formulierung oder die getrennte Applikationen der beiden Wirkstoffe in getrennten Darreichungsformen verstanden. Werden die Wirkstoffe **1** und **2** in getrennten Darreichungsformen appliziert, kann diese getrennte Applikation gleichzeitig oder zeitlich abgestuft, das heißt nacheinander erfolgen.

Ein Aspekt der vorliegenden Erfindung betrifft vorstehend genannte Arzneimittelkombinationen, welche neben therapeutisch wirksamen Mengen von **1** und **2** einen pharmazeutisch verträglichen Trägerstoff enthalten. Ein Aspekt der vorliegenden Erfindung betrifft vorstehend genannte Arzneimittel, welche neben therapeutisch wirksamen Mengen von **1** und **2** keinen pharmazeutisch verträglichen Trägerstoff enthalten.

Ein bevorzugter Aspekt der vorliegenden Erfindung betrifft die Verwendung therapeutisch wirksamer Mengen der Wirkstoffe **1** zur Herstellung eines einen oder mehrere, bevorzugt einen Wirkstoff **2** enthaltenden Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

Bevorzugt ist vorstehend genannte Verwendung der erfindungsgemäßen Arzneimittelkombinationen zur Herstellung eines Arzneimittels zur Behandlung von obstruktiven Lungenerkrankungen, die ausgewählt sind aus der Gruppe bestehend aus Asthma Bronchiale, pädiatrisches Asthma, schweres Asthma, akuter Asthma-Anfall, chronische Bronchitis und COPD (chronisch obstruktive pulmonale Erkrankung), wobei die Verwendung zur Herstellung eines Arzneimittels zur Behandlung von Asthma Bronchiale und COPD erfindungsgemäß besonders bevorzugt ist.

Bevorzugt ist ferner die vorstehend genannte Verwendung der erfindungsgemäßen Arzneimittelkombinationen zur Herstellung eines Arzneimittels zur Behandlung von Lungenemphysemen die ihren Ursprung haben in COPD oder α1-Proteinase-Inhibitor-Mangel.

Bevorzugt ist ferner die vorstehend genannte Verwendung der erfindungsgemäßen Arzneimittelkombinationen zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, bronchoalveoläres Karzinom und Lymphome.

Bevorzugt ist ferner die vorstehend genannte Verwendung der erfindungsgemäßen Arzneimittelkombinationen zur Herstellung eines Arzneimittels zur Behandlung von Interstitiellen Lungenerkrankungen, die ausgewählt sind aus der Gruppe bestehend aus infektiös bedingte Pneumonien, wie beispielsweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, sytemische Sklerodermie oder Sarkoidose, Granulomatosen, wie beispielsweise Morbus Boeck, idiopathische interstitielle Pneumonie oder idiopathische pulmonäre Fibrose (IPF).

Bevorzugt ist ferner die vorstehend genannte Verwendung der erfindungsgemäßen Arzneimittelkombinationen zur Herstellung eines Arzneimittels zur Behandlung von Zystischer Fibrose bzw. Mukoviszidose.

Bevorzugt ist ferner die vorstehend genannte Verwendung der erfindungsgemäßen Arzneimittelkombinationen zur Herstellung eines Arzneimittels zur Behandlung von Bronchitiden, wie beispielsweise Bronchitis aufgrund bakterieller oder viraler Infektion, Allergische Bronchitis und Toxische Bronchitis.

Bevorzugt ist ferner die vorstehend genannte Verwendung der erfindungsgemäßen Arzneimittelkombinationen zur Herstellung eines Arzneimittels zur Behandlung von Bronchiektasen.

Bevorzugt ist ferner die vorstehend genannte Verwendung der erfindungsgemäßen Arzneimittelkombinationen zur Herstellung eines Arzneimittels zur Behandlung von ARDS (adult respiratory distress syndrom).

Bevorzugt ist ferner die vorstehend genannte Verwendung der erfmdungsgemäßen Arzneimittelkombinationen zur Herstellung eines Arzneimittels zur Behandlung von Lungenödemen, beispielsweise toxischer Lungenödeme nach Aspiration oder Inhalation von toxischen Substanzen und Fremdstoffen.

Besonders bevorzugt betrifft die vorstehend genannte Verwendung der erfindungsgemäßen Arzneimittelkombinationen zur Herstellung eines Arzneimittels zur Behandlung von Asthma oder COPD. Von besonderer Bedeutung ist ferner die vorstehend genannte Verwendung der erfindungsgemäßen Arzneimittelkombinationen zur Herstellung eines Arzneimittels zur einmal täglichen Behandlung von entzündlichen und obstruktiven Atemwegserkrankungen, besonders bevorzugt zur einmal täglichen Behandlung von Asthma oder COPD.

Die vorliegende Erfindung betrifft ferner die Verwendung therapeutisch wirksamer Mengen eines Wirkstoffs der Formel **1** in Kombination mit therapeutisch wirksamen Mengen eines Wirkstoffs **2** zur Herstellung eines Arzneimittels zur Behandlung einer der vorstehend genannten Erkrankungen.

Die vorliegende Erfindung betrifft ferner ein Verfahren zur Behandlung einer der vorstehend genannten Erkrankungen, welches dadurch gekennzeichnet ist, dass therapeutisch wirksame Mengen eines Wirkstoffs der Formel **1** in Kombination mit therapeutisch wirksamen Mengen eines Wirkstoffs **2** appliziert werden.

Im Rahmen der erfindungsgemäßen Arzneimittelkombinationen können pro Einmalgabe beispielsweise 0,1 - 1000µg einer Verbindung der Formel **1** appliziert werden. Bevorzugt werden pro Einmalgabe 1 - 500 µg, besonders bevorzugt 3 - 100 µg der Verbindung der Formel **1** appliziert, wobei ein Dosierungsbereich von 5 - 75µg, bevorzugt von 7 - 50 µg erfindungsgemäß bevorzugt ist. Besonders bevorzugt werden die erfindungsgemäßen Arzneimittel in einer solchen Menge appliziert, dass pro Einmalgabe 9 - 40 µg, besonders bevorzugt 11 - 30µg, ferner bevorzugt 12 - 25 µg der Verbindung der Formel **1** verabreicht werden. Beispielsweise und ohne den Gegenstand der vorliegenden Erfindung darauf zu beschränken, können pro Einmalgabe 5µg, 7,5µg, 10µg, 12,5µg, 15µg, 17,5µg, 20ug, 22,5µg, 25µg, 27,5µg, 30µg, 32,5µg, 35µg, 37,5µg, 40µg, 42,5µg, 45µg, 47,5µg, 50µg, 52,5µg, 55µg, 57,5µg, 60µg, 62,5µg, 65µg, 67,5µg, 70µg, 72,5µg oder 75µg einer Verbindung der Formel **1** appliziert werden.

Die vorstehend genannten Dosierungen beziehen sich auf die Verbindungen der Formel **1** in Form ihrer freien Base. Werden die Verbindungen der Formel **1** in Form ihrer pharmazeutisch verträglichen Säureadditionssalze appliziert, sind aus vorstehend genannten Dosisbereichen die entsprechenden Dosisbereiche für die Säureadditionssalze unter Berücksichtigung des Molekulargewichts der verwendeten Säuren für den Fachmann leicht berechenbar. Besonders bevorzugt, werden die Verbindungen der Formel **1** bei o.g. Dosisbereichen in Form der enantiomerenreinen Verbindungen, besonders bevorzugt in Form ihrer R-Enantiomere appliziert.

Werden die Verbindungen der Formel **1** in Kombination mit einem Anticholinergikum **2a** appliziert, variiert die Menge des eingesetzten Anticholinergikums stark in Abhängigkeit von der Wahl des Wirkstoffs.

Ohne den Umfang der Erfindung darauf zu beschränken können im Falle des Tiotropiums **2a.1'** solche Mengen an Anticholinergikum **(2a.1')** appliziert werden, dass pro Einmalgabe 0,1 - 80µg, bevorzugt 0,5 - 60 µg, besonders bevorzugt etwa 1 - 50µg **2a.1'** enthalten sind. Beispielsweise und ohne den Gegenstand der vorliegenden Erfindung darauf zu beschränken, können pro Einmalgabe 2,5µg, 5µg, 10µg, 18µg, 20µg, 36µg oder 40µg **2a.1'** appliziert werden. Die jeweils entsprechende Menge des zum Einsatz gelangenden Salzes **2a.1** oder gegebenenfalls zum Einsatz gelangender Hydrate oder Solvate sind für den Fachmann je nach Wahl des Anions leicht berechenbar. Wird als erfindungsgemäß bevorzugtes Tiotropiumsalz **2a.1** beispielsweise Tiotropiumbromid verwendet, entsprechen die vorstehend beispielhaft genannten pro Einmalgabe applizierten Wirkstoffmengen von **2a.1'** den nachfolgenden pro Einmalgabe applizierten Mengen an **2a.1:** 3µg, 6µg, 12µg, 21,7µg, 24,1µg, 43,3µg und 48,1µg **2a,1.** Im Falle des Tiotropiums **2a.1'** erfolgt die Applikation der vorstehend genannten Dosierungen vorzugsweise ein- oder zweimal täglich, wobei die einmal tägliche Applikation erfindungsgemäß besonders bevorzugt ist.

Ohne den Umfang der Erfindung darauf zu beschränken, können im Falle des Kations **2a.2'** solche Mengen an Anticholinergikum **(2a.2')** appliziert werden, dass pro Einmalgabe 1 - 500µg, bevorzugt 5 - 300 µg, besonders bevorzugt 15-200 µg **2a.2'** enthalten sind. Beispielsweise und ohne den Gegenstand der vorliegenden Erfindung darauf zu beschränken, können pro Einmalgabe 15µg, 20µg, 25µg, 30µg, 35µg, 40µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg oder 200µg **2a.2'** appliziert werden. Die jeweils entsprechende Menge des zum Einsatz gelangenden Salzes **2a.2** oder gegebenenfalls zum Einsatz gelangender Hydrate oder Solvate sind für den Fachmann je nach Wahl des Anions leicht berechenbar. Im Falle des Oxitropiums **2a.2'** erfolgt die Applikation der vorstehend genannten Dosierungen vorzugsweise ein- bis viermal täglich, wobei die zwei- bis dreimal tägliche Applikation erfindungsgemäß besonders bevorzugt ist.

Ohne den Umfang der Erfindung darauf zu beschränken, können im Falle des Kations **2a.3'** solche Mengen an Anticholinergikum **(2a.3')** appliziert werden, dass pro Einmalgabe 1 - 500µg, bevorzugt 5 - 300 µg, besonders bevorzugt 15-200 µg **2a.3'** enthalten sind. Beispielsweise und ohne den Gegenstand der vorliegenden Erfindung darauf zu beschränken, können pro Einmalgabe 15µg, 20µg, 25µg, 30µg, 35µg, 40µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg oder 200µg **2a.3'** appliziert werden. Die jeweils entsprechende Menge des zum Einsatz gelangenden Salzes **2a.3** oder gegebenenfalls zum Einsatz gelangender Hydrate oder Solvate sind für den Fachmann je nach Wahl des Anions leicht berechenbar. Im Falle des Flutropiums **2a.3'** erfolgt die Applikation der vorstehend genannten Dosierungen vorzugsweise ein- bis viermal täglich, wobei die zwei- bis dreimal tägliche Applikation erfindungsgemäß besonders bevorzugt ist.

Ohne den Umfang der Erfindung darauf zu beschränken, können im Falle des Kations **2a.4'** solche Mengen an Anticholinergikum **(2a.4')** appliziert werden, dass pro Einmalgabe 1 - 500µg, bevorzugt 5 - 300 µg, besonders bevorzugt 20-200 µg **2a.4'** enthalten sind. Beispielsweise und ohne den Gegenstand der vorliegenden Erfindung darauf zu beschränken, können pro Einmalgabe 20µg, 25µg, 30µg, 35µg, 40µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg oder 200µg **2a.4'** appliziert werden. Die jeweils entsprechende Menge des zum Einsatz gelangenden Salzes **2a.4** oder gegebenenfalls zum Einsatz gelangender Hydrate oder Solvate sind für den Fachmann je nach Wahl des Anions leicht berechenbar. Im Falle des Ipratropiums **2a.4'** erfolgt die Applikation der vorstehend genannten Dosierungen vorzugsweise ein- bis viermal täglich, wobei die zwei- bis dreimal, besonders bevorzugt die dreimal tägliche Applikation erfindungsgemäß besonders bevorzugt ist.

Ohne den Umfang der Erfindung darauf zu beschränken, können im Falle des Kations **2a.5'** solche Mengen an Anticholinergikum **(2a.5')** appliziert werden, dass pro Einmalgabe 1 - 500µg, bevorzugt 5 - 300 µg, besonders bevorzugt 15-200 µg enthalten sind. Beispielsweise und ohne den Gegenstand der vorliegenden Erfindung darauf zu beschränken, können pro Einmalgabe 15µg, 20µg, 25µg, 30µg, 35µg, 40µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg oder 200µg **2a.5'** appliziert werden. Die jeweils entsprechende Menge des zum Einsatz gelangenden Salzes **2a.5** oder gegebenenfalls zum Einsatz gelangender Hydrate oder Solvate sind für den Fachmann je nach Wahl des Anions leicht berechenbar. Im Falle des Glycopyrroniums **2a.5'** erfolgt die Applikation der vorstehend genannten Dosierungen vorzugsweise ein- bis viermal täglich, wobei die zweibis dreimal tägliche Applikation erfindungsgemäß besonders bevorzugt ist.

Ohne den Umfang der Erfindung darauf zu beschränken, können im Falle des Kations **2a.6'** solche Mengen an Anticholinergikum **(2a.6')** appliziert werden, dass pro Einmalgabe 1000 - 6500µg, bevorzugt 2000 - 6000µg, besonders bevorzugt 3000 - 5500µg, besonders bevorzugt 4000 - 5000µg **2a.6'** enthalten sind. Beispielsweise und ohne den Gegenstand der vorliegenden Erfindung darauf zu beschränken, können pro Einmalgabe 3500µg, 3750µg, 4000µg, 4250µg, 4500µg, 4750µg, oder 5000µg **2a.6'** appliziert werden. Die jeweils entsprechende Menge des zum Einsatz gelangenden Salzes **2a.6** oder gegebenenfalls zum Einsatz gelangender Hydrate oder Solvate sind für den Fachmann je nach Wahl des Anions leicht berechenbar. Im Falle des Trospiums **2a.6'** erfolgt die Applikation der vorstehend genannten Dosierungen vorzugsweise ein- bis viermal täglich, wobei die zwei- bis dreimal tägliche Applikation erfindungsgemäß besonders bevorzugt ist.

Ohne den Umfang der Erfindung darauf zu beschränken, im Falle des Kations **2a.7'** solche Mengen an Anticholinergikum **(2a.7')** appliziert werden, dass pro Einmalgabe 50 - 1000µg, bevorzugt 100 - 800µg, besonders bevorzugt 200 - 700µg, besonders bevorzugt 300 - 600µg **2a.7'** enthalten sind. Beispielsweise und ohne den Gegenstand der vorliegenden Erfindung darauf zu beschränken, können pro Einmalgabe 300µg, 350µg, 400µg, 450µg, 500µg, 550µg, oder 600µg **2a.7'** appliziert werden. Die jeweils entsprechende Menge des zum Einsatz gelangenden Salzes **2a.7** oder gegebenenfalls zum Einsatz gelangender Hydrate oder Solvate sind für den Fachmann je nach Wahl des Anions leicht berechenbar. Im Falle des Kations **2a.7'** erfolgt die Applikation der vorstehend genannten Dosierungen vorzugsweise ein- bis dreimal täglich, wobei die ein- bis zweimal, besonders bevorzugt die einmal tägliche Applikation erfindungsgemäß besonders bevorzugt ist.

Ohne den Umfang der Erfindung darauf zu beschränken, können im Falle der Kationen **2a.9'** und **2a.10'** solche Mengen an Anticholinergikum (**2a.9'** oder **2a.10'** appliziert werden, dass pro Einmalgabe 1 - 500µg, bevorzugt 5 - 300 µg, besonders bevorzugt 15-200 µg **2a.9'** oder **2a.10'** enthalten sind. Beispielsweise und ohne den Gegenstand der vorliegenden Erfindung darauf zu beschränken, können pro Einmalgabe 15µg, 20µg, 25µg, 30µg, 35µg, 40µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg oder 200µg **2a.9'** oder **2a.10'** appliziert werden. Die jeweils entsprechende Menge des zum Einsatz gelangenden Salzes **2a.9'** oder **2a.10'oder** gegebenenfalls zum Einsatz gelangender Hydrate oder Solvate sind für den Fachmann je nach Wahl des Anions leicht berechenbar. Im Falle der Kationen **2a.9'** oder **2a.10'**erfolgt die Applikation der vorstehend genannten Dosierungen vorzugsweise ein- bis dreimal täglich, wobei die ein- bis zweimal, besonders bevorzugt die einmal tägliche Applikation erfindungsgemäß besonders bevorzugt ist.

Ohne den Umfang der Erfindung darauf zu beschränken, können im Falle der Kationen **2a.11'** bis **2a.13'** solche Mengen an Anticholinergikum **(2a.11', 2a.12'** oder **2a.13')** appliziert werden, dass pro Einmalgabe 1 - 500µg, bevorzugt 5 - 300 µg, besonders bevorzugt 10-200 µg **2a.11', 2a.12'** oder **2a.13'** enthalten sind. Beispielsweise und ohne den Gegenstand der vorliegenden Erfindung darauf zu beschränken, können pro Einmalgabe10µg, 15µg, 20µg, 25µg, 30µg, 35µg, 40µg, 45µg, 50µg, 55µg, 60µg, 65µg, 70µg, 75µg, 80µg, 85µg, 90µg, 95µg, 100µg, 105µg, 110µg, 115µg, 120µg, 125µg, 130µg, 135µg, 140µg, 145µg, 150µg, 155µg, 160µg, 165µg, 170µg, 175µg, 180µg, 185µg, 190µg, 195µg oder 200µg **2a.11', 2a.12'** oder **2a.13'** appliziert werden. Die jeweils entsprechende Menge des zum Einsatz gelangenden Salzes **2a.11, 2a.12** oder **2a.13** oder gegebenenfalls zum Einsatz gelangender Hydrate oder Solvate sind für den Fachmann je nach Wahl des Anions leicht berechenbar.

Im Falle der Kationen **2a.11, 2a.12** oder **2a.13** erfolgt die Applikation der vorstehend genannten Dosierungen vorzugsweise ein- bis dreimal täglich, wobei die ein- bis zweimal, besonders bevorzugt die einmal tägliche Applikation erfindungsgemäß besonders bevorzugt ist.

Werden die Verbindungen der Formel **1** in Kombination mit einem PDE IV-Inhibitor **2b** appliziert, werden vorzugsweise etwa 1 - 10000 µg **2b** pro Einmalgabe appliziert. Bevorzugt werden solche Mengen an **2b** appliziert, dass pro Einmalgabe 10 - 5000µg, bevorzugt 50 - 2500 µg, besonders bevorzugt 100-1000 µg **2b** enthalten sind. Beispielsweise und ohne den Gegenstand der vorliegenden Erfindung darauf zu beschränken, können pro Einmalgabe 100µg, 115µg, 120µg, 125µg, 130µg, 135µg, 140µg, 145µg, 150µg, 155µg, 160µg, 165µg, 170µg, 175µg, 180µg, 185µg, 190µg, 195µg, 200µg, 205µg, 210µg, 215µg, 220µg, 225µg, 230µg, 235µg, 240µg, 245µg, 250µg, 255µg, 260µg, 265µg, 270µg, 275µg, 280µ, 285µg, 290µg, 295µg, 300µg, 305µg, 310µg, 315µg, 320µg, 325µg, 330µg, 335µg, 340µg, 345µg, 350µg, 355µg, 360µg, 365µg, 370µg, 375µg, 380µg, 385µg, 390µg, 395µg, 400µg, 405µg, 410µg, 415µg, 420µg, 425µg, 430µg, 435µg, 440µg, 445µg, 450µg, 455µg, 460µg, 465µg, 470µg, 475µg, 480µg, 485µg, 490µg, 495µg, 500µg, 505µg, 510µg, 515µg, 520µg, 525µg, 530µg, 535µg, 540µg, 545µg, 550µg, 555µg, 560µg, 565µg, 570µg, 575µg, 580µg, 585µg, 590µg, 595µg, 600µg, 605µg, 610µg, 615µg, 620µg, 625µg, 630µg, 635µg, 640µg, 645µg, 650µg, 655µg, 660µg, 665µg, 670µg, 675µg, 680µg, 685µg, 690µg, 695µg, 700µg, 705µg, 710µg, 715µg, 720µg, 725µg, 730µg, 735µg, 740µg, 745µg, 750µg, 755µg, 760µg, 765µg, 770µg, 775µg, 780µg, 785µg, 790µg, 795µg, 800µg, 805µg, 810µg, 815µg, 820µg, 825µg, 830µg, 835µg, 840µg, 845µg, 850µg, 855µg, 860µg, 865µg, 870µg, 875µg, 880µg, 885µg, 890µg, 895µg, 900µg, 905µg, 910µg, 915µg, 920µg, 925µg, 930µg, 935µg, 940µg, 945µg, 950µg, 955µg, 960µg, 965µg, 970µg, 975µg, 980µg, 985µg, 990µg, 995µg oder 1000µg **2b** appliziert werden. Bei gegebenenfalls zum Einsatz gelangenden Säureadditionssalzen von **2b** ist die entsprechende Menge des zum Einsatz gelangenden Salzes für den Fachmann je nach Wahl der Säure aus vorstehenden Werten leicht berechenbar.

Werden die Verbindungen der Formel **1** in Kombination mit einem Steroid **2c** appliziert, werden vorzugsweise etwa 1 - 10000 µg **2c** pro Einmalgabe appliziert. Bevorzugt werden solche Mengen an **2c** appliziert, dass pro Einmalgabe 5 - 5000µg, bevorzugt 5 - 2500 µg, besonders bevorzugt 10-1000 µg **2c** enthalten sind. Beispielsweise und ohne den Gegenstand der vorliegenden Erfindung darauf zu beschränken, können pro Einmalgabe 10µg, 15µg, 20µg, 25µg, 30µg, 35µg, 40µg, 45µg, 50µg, 55µg, 60µg, 65µg, 70µg, 75µg, 80µg, 85µg, 90µg, 95µg, 100µg, 115µg, 120µg, 125µg, 130µg, 135µg, 140µg, 145µg, 150µg, 155µg, 160µg, 165µg, 170µg, 175µg, 180µg, 185µg, 190µg, 195µg, 200µg, 205µg, 210µg, 215µg, 220µg, 225µg, 230µg, 235µg, 240µg, 245µg, 250µg, 255µg, 260µg, 265µg, 270µg, 275µg, 280µg, 285µg, 290µg, 295µg, 300µg, 305µg, 310µg, 315µg, 320µg, 325µg, 330µg, 335µg, 340µg, 345µg, 350µg, 355µg, 360µg, 365µg, 370µg, 375µg, 380µg, 385µg, 390µg, 395µg, 400µg, 405µg, 410µg, 415µg, 420µg, 425µg, 430µg, 435µg, 440µg, 445µg, 450µg, 455µg, 460µg, 465µg, 470µg, 475µg, 480µg, 485µg, 490µg, 495µg, 500µg, 505µg, 510µg, 515µg, 520µg, 525µg, 530µg, 535µg, 540µg, 545µg, 550µg, 555µg, 560µg, 565µg, 570µg, 575µg, 580µg, 585µg, 590µg, 595µg, 600µg, 605µg, 610µg, 615µg, 620µg 625µg 630µg 635µ, 640µ, 645µg 650µg 655µg 660µg, 665µg, 670µg, 675µg, 680µg, 685µg, 690µg, 695µg, 700µg, 705µg, 710µg, 715µg, 720µg, 725µg, 730µg, 735µg, 740µg, 745µg, 750µg, 755µg, 760µg, 765µg, 770µg, 775µg, 780µg, 785µg, 790µg, 795µg, 800µg, 805µg, 810µg, 815µg, 820µg, 825µg, 830µg, 835µg, 840µg, 845µg, 850µg, 855µg, 860µg, 865µg, 870µg, 875µg, 880µg, 885µg, 890µg, 895µg, 900µg, 905µg, 910µg, 915µg, 920µg, 925µg, 930µg, 935µg, 940µg, 945µg, 950µg, 955µg, 960µg, 965µg, 970µg, 975µg, 980µg, 985µg, 990µg, 995µg oder 1000µg **2c** appliziert werden. Bei gegebenenfalls zum Einsatz gelangenden Salzen oder Derivaten von **2c** ist die entsprechende Menge des zum Einsatz gelangenden Salzes/Derivates für den Fachmann je nach,Wahl des Salzes/Derivates aus vorstehenden Werten leicht berechenbar.

Werden die Verbindungen der Formel **1** in Kombination mit einem LTD4-Antagonisten **2d** appliziert, werden vorzugsweise etwa 0,01 - 500 mg **2d** pro Einmalgabe appliziert. Bevorzugt werden solche Mengen an **2d** appliziert, dass pro Einmalgabe 0,1 - 250mg, bevorzugt 0,5 - 100 mg, besonders bevorzugt 1-50 mg **2d** enthalten sind. Beispielsweise und ohne den Gegenstand der vorliegenden Erfindung darauf zu beschränken, können pro Einmalgabe 1mg, 2,5mg, 5mg, 5,5mg, 7 mg, 7, 5mg, 10mg, 12,5mag, 15mg, 17,5mag, 20mg, 22,5mg, 25mg, 27,5mg, 30mg, 32,5mg, 35mg, 37,5mg, 40mg, 42,5mg, 45mg, 47,5mg öder 50mg **2d** appliziert werden. Bei gegebenenfalls zum Einsatz gelangenden Säureadditionssalzen, Salzen oder Derivaten von **2d** ist die entsprechende Menge des zum Einsatz gelangenden Salzes/Derivates für den Fachmann je nach Wahl des Salzes/Derivates aus vorstehenden Werten leicht berechenbar.

Werden die Verbindungen der Formel **1** in Kombination mit einem EGFR-Hemmer **2e** appliziert, werden vorzugsweise etwa 100 - 15000 µg **2e** pro Einmalgabe appliziert. Bevorzugt werden solche Mengen an **2e** appliziert, dass pro Einmalgabe 500 - 10000µg, bevorzugt 750 - 8000 µg, besonders bevorzugt 1000-7000 µg **2e** enthalten sind. Beispielsweise und ohne den Gegenstand der vorliegenden Erfindung darauf zu beschränken, können pro Einmalgabe 1000µg, 1150µg, 1200µg, 1250µg, 1300µg, 1350µg, 1400µg, 1450µg, 1500µg, 1550µg, 1600µg, 1650µg, 1700µg, 1750µg, 1800µg, 1850µg, 1900µg, 1950µg, 2000µg, 2050µg, 2100µg, 2150µg, 2200µg, 2250µg, 2300µg, 2350µg, 2400µg, 2450µg, 2500µg, 2550µg, 2600µg, 2650µg, 2700µg, 2750µg, 2800µg, 2850µg, 2900µg, 2950µg, 3000µg, 3050µg, 3100µg, 3150µg, 3200µg, 3250µg, 3300µg, 3350µg, 3400µg, 3450µg, 3500µg, 3550µg, 3600µg, 3650µg, 3700µg, 3750µg, 3800µg, 3850µg, 3900µg, 3950µg, 4000µg, 4050µg, 4100µg, 4150µg, 4200µg, 4250µg, 4300µg, 4350µg, 4400µg, 4450µg, 4500µg, 4550µg, 4600µg, 4650µg, 4700µg, 4750µg, 4800µg, 4850µg, 4900µg, 4950µg, 5000µg, 5050µg, 5100µg, 5150µg, 5200µg, 5250µg, 5300µg, 5350µg, 5400µg, 5450µg, 5500µg, 5550µg, 5600µg, 5650µg, 5700µg, 5750µg, 5800µg, 5850µg, 5900µg, 5950µg, 6000µg, 6050µg, 6100µg, 6150µg, 6200µg, 6250µg, 6300µg, 6350µg, 6400µg, 6450µg, 6500µg, 6550µg, 6600µg, 6650µg, 6700µg, 6750µg, 6800µg, 6850µg, 6900µg, 6950µg, oder 7000µg **2e** appliziert werden. Bei gegebenenfalls zum Einsatz gelangenden Säureadditionssalzen von **2e** ist die entsprechende Menge des zum Einsatz gelangenden Salzes für den Fachmann je nach Wahl der Säure aus vorstehenden Werten leicht berechenbar.

Die beiden Wirkstoffkomponenten **1** und **2** können - zusammen oder räumlich getrennt - jeweils in an sich bekannter Weise inhalativ, oral, parenteral oder auf anderem Wege in weitestgehend üblichen Formulierungen wie beispielsweise Tabletten, Dragees, Pillen, Granulate, Aerosole, Sirupe, Emulsionen, Suspensionen, Pulver und Lösungen, unter Verwendung inerter, nicht toxischer, pharmazeutisch geeigneter Trägerstoffe oder Lösungsmittel, appliziert werden.

Geeignete Anwendungsformen zur Applikation der Verbindungen der Formel **1** und **2** sind beispielsweise Tabletten, Kapseln, Zäpfchen, Lösungen, Pulver etc. Der Anteil der pharmazeutisch wirksamen Verbindung(en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können. Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösungsmittel als Lösevermittler bzw. Hilflösungsmittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösungsmittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide), synthetische Gesteinsmehle (z.B. hochdisperse Kieselsäure und Silikate), Zucker (z.B. Rohr-, Milch- und Traubenzucker), Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wäßriger Suspensionen können die Wirkstoffe außer den oben genannten Hilfsstoffen mit verschiedenen Geschmacksaufbesserem oder Farbstoffen versetzt werden.

Vorzugsweise wird auch bei getrennter Applikation der beiden Komponenten **1** und **2** zumindest die Komponente 1 inhalativ appliziert. Wird die Komponente **1** inhalativ appliziert, kann die Komponente **2** bei getrennter Gabe der beiden Wirkstoffe auch beispielsweise oral oder auch parenteral auf Basis im Stand der Technik üblicher Formulierungen wie Tabletten, Dragees, Pillen, Granulate, Aerosole, Sirupe, Emulsionen, Suspensionen, Pulver und Lösungen, unter Verwendung inerter, nicht toxischer, pharmazeutisch geeigneter Trägerstoffe oder Lösungsmittel, appliziert werden.

Vorzugsweise werden die erfindungsgemäßen Arzneimittelkombinationen allerdings inhalativ mittels einer einzigen, beide Wirkstoffe **1** und **2** enthaltenden oder mittels getrennter, jeweils nur einen der Wirkstoffe **1** und **2** enthaltenden, für die inhalative Anwendung geeigneter Darreichungsformen appliziert.

Als inhalierbare Darreichungsformen kommen Inhalationspulver, treibgashaltige Dosieraerosole oder treibgasfreie Inhalationslösungen in Betracht. Erfindungsgemäße Inhalationspulver enthaltend die Wirkstoffkombination aus **1** und **2** können allein aus den genannten Wirkstoffen oder aus einem Gemisch der genannten Wirkstoffe mit physiologisch verträglichen Hilfsstoffen bestehen. Im Rahmen der vorliegenden Erfindung sind von dem Begriff treibgasfreie Inhalationslösungen auch Konzentrate oder sterile, gebrauchsfertige Inhalationslösungen umfaßt. Die erfindungsgemäßen Darreichungsformen können die Wirkstoffkombination aus **1** und **2** entweder gemeinsam in einer oder in zwei getrennten Darreichungsformen enthalten. Diese im Rahmen der vorliegenden Erfindung einsetzbaren Darreichungsformen werden im nachfolgenden Teil der Beschreibung detailliert beschrieben.

### A) Inhalationspulver enthaltend die erfindungsgemäßen Wirkstoffkombinationen:

Die erfindungsgemäßen Inhalationspulver können 1 und 2 entweder allein oder im Gemisch mit geeigneten physiologisch unbedenkliche Hilfsstoffen enthalten. Sind die Wirkstoffe 1 und 2 im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose, Trehalose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose, Trehalose oder Glucose, insbesondere, aber nicht ausschließlich in Form ihrer Hydrate, bevorzugt ist.

Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfststoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhalationspulver mikronisierter Wirkstoff **1** und **2,** vorzugsweise mit einer mittleren Teilchengröße von 0,5 bis 10µm, besonders bevorzugt von 1 bis 6µm, dem Hilfsstoff oder der Hilfsstoffmischung beigemischt. Verfahren zur Herstellung der erfindungsgemäßen Inhalationspulver durch Mahlen und Mikronisieren sowie durch abschließendes Mischen der Bestandteile sind aus dem Stand der Technik bekannt. Die erfindungsgemäßen Inhalationspulver können entweder in Form einer einzigen Pulvermischung, die sowohl **1** als auch **2** enthält oder in Form von separaten Inhalationspulvern, die lediglich **1** und **2** enthalten bereitgestellt und appliziert werden.

Die erfindungsgemäßen Inhalationspulver können mittels aus dem Stand der Technik bekannten Inhalatoren appliziert werden.

Erfindungsgemäße Inhalationspulver, die neben **1** und **2** ferner einen physiologisch unbedenklichen Hilfsstoff enthalten, können beispielsweise mittels Inhalatoren appliziert werden, die eine einzelne Dosis aus einem Vorrat mittels einer Meßkammer, wie er in der US 4570630A beschrieben wird, oder über andere apparative Vorrichtungen, wie sie in der DE 36 25 685 A beschrieben werden, dosieren. Die erfindungsgemäßen Inhalationspulver, die **1** und **2** gegebenenfalls in Verbindung mit einem physiologisch verträglichen Hilfsstoff enthalten, können beispielsweise mittels des unter dem Namen Turbuhaler^{®} bekannten Inhalators beziehungsweise mit Inhalatoren wie sie beispielsweise in der EP 237507 A offenbart werden, appliziert werden. Vorzugsweise werden die erfindungsgemäßen Inhalationspulver, die neben **1** und **2** physiologisch unbedenkliche Hilfsstoff enthalten, allerdings in Kapseln abgefüllt (zu sogenannten Inhaletten), die in Inhalatoren wie beispielsweise in der WO 94/28958 beschrieben, zur Anwendung gelangen.

Ein zur Anwendung der erfindungsgemäßen Arzneimittelkombination in Inhaletten besonders bevorzugter Inhalator ist Figur 1 zu entnehmen.

Dieser Inhalator (Handihaler^{®}) für die Inhalation pulverförmiger Arzneimittel aus Kapseln ist gekennzeichnet durch ein Gehäuse 1, enthaltend zwei Fenster 2, ein Deck 3, in dem sich Lufteinlassöffnungen befinden und welches mit einem über ein Siebgehäuse 4 befestigten Sieb 5 versehen ist, eine mit Deck 3 verbundene Inhalationskammer 6, an der ein mit zwei geschliffenen Nadeln 7 versehener, gegen eine Feder 8 beweglicher Drücker 9 vorgesehen ist, sowie ein über eine Achse 10 klappbar mit dem Gehäuse 1, dem Deck 3 und einer Kappe 11 verbundenes Mundstück 12, sowie Luftdurchlasslöcher 13 zur Einstellung des Strömungswiderstandes.

Sollen die erfmdungsgemäßen Inhalationspulver im Sinne der vorstehend gennannten bevorzugten Anwendung in Kapseln abgefüllt werden, bieten sich Füllmengen von 1 bis 30mg pro Kapsel an. Diese enthalten erfindungsgemäß entweder gemeinsam oder jeweils die bereits vorstehend für **1** und **2** genannten Dosierungen pro Einmalgabe.

### B) Treibgashaltige Inhalationsaerosole enthaltend die erfindungsgemäßen Wirkstoffkombinationen:

Erfindungsgemäße treibgashaltige Inhalationsaerosole können **1** und **2** im Treibgas gelöst oder in dispergierter Form enthalten. Hierbei können **1** und **2** in getrennten Darreichungsformen oder in einer gemeinsamen Darreichungsform enthalten sein, wobei **1** und **2** entweder beide gelöst, beide dispergiert oder jeweils nur eine Komponente gelöst und die andere dispergiert enthalten sein können.

Die zur Herstellung der erfindungsgemäßen Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt chlorierten und fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind Halogenierte Alkanderivate ausgewählt aus TG11, TG12, TG134a (1,1,1,2-Tetrafluorethan), TG227 (1,1,1,2,3,3,3-Heptafluorpropan) und Mischungen derselben, wobei die Treibgase TG134a, TG227 und Gemische derselben bevorzugt sind.

Die erfindungsgemäßem treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die erfindungsgemäßen treibgashaltigen Inhalationsaerosole können bis zu 5 Gew-% an Wirkstoff **1** und/oder **2** enthalten. Erfindungsgemäße Aerosole enthalten beispielsweise 0,002 bis 5 Gew-%, 0,01 bis 3 Gew-%, 0,015 bis 2 Gew-%, 0,1 bis 2 Gew-%, 0,5 bis 2 Gew-% oder 0,5 bis 1 Gew-% an Wirkstoff **1** und/oder **2.**

Liegen die Wirkstoffe **1** und/oder **2** in dispergierter Form vor weisen die Wirkstoffteilchen bevorzugt eine mittlere Teilchengröße von bis zu 10 µm, bevorzugt von 0,1 bis 6 µm, besonders bevorzugt von 1 bis 5 µm auf.

Die vorstehend genannten erfindungsgemäßen treibgashaltigen Inhalationaerosole können mittels im Stand der Technik bekannten Inhalatoren (MDIs = metered dose inhalers) appliziert werden. Dementsprechend betrifft ein weiterer Aspekt der vorliegenden Erfindung Arzneimittel in Form von wie vorstehend beschriebenen treibgashaltigen Aerosolen in Verbindung mit einem oder mehreren zur Verabreichung dieser Aerosole geeigneten Inhalatoren. Ferner betrifft die vorliegende Erfindung Inhalatoren, dadurch gekennzeichnet, dass sie vorstehend beschriebene erfindungsgemäße treibgashaltige Aerosole enthalten.

Die vorliegende Erfindung betrifft ferner Kartuschen, die ausgestattet mit einem geeigneten Ventil in einem geeigneten Inhalator zur Anwendung gelangen können und die eine der vorstehend genannten erfindungsgemäßen treibgashaltigen Inhalationsaerosole enthalten. Geeignete Kartuschen und Verfahren zur Abfüllung dieser Kartuschen mit den erfindungsgemäßen treibgashaltigen Inhalationsaerosolen sind aus dem Stand der Technik bekannt.

### C) Treibgasfreie Inhalationslösungen oder Suspensionen enthaltend die erfindungsgemäßen Wirkstoffkombinationen:

Erfindungsgemäß treibgasfreie Inhalationslösungen enthalten beispielsweise wässrige oder alkoholische, bevorzugt ethanolische, gegebenenfalls ethanolische im Gemisch mit wässrigen Lösungsmitteln. Im Falle wässrig/ethanolischer Lösungsmittelgemische ist der relative Anteil an Ethanol gegenüber Wasser nicht begrenzt, bevorzugt liegt die maximale Grenze jedoch bei bis 70 Volumenprozent, insbesondere bei bis zu 60 Volumenprozent Ethanol. Die restlichen Volumenprozente werden von Wasser aufgefüllt. Die **1** und **2** getrennt oder gemeinsam enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Erfindungsgemäß kann in der vorliegenden Formulierung auf den Zusatz von Editinsäure (EDTA) oder einem der bekannten Salze davon, Natriumedetat, als Stabilisator oder Komplexbildner verzichtet werden.

Andere Ausführungsformen beinhalten diese Verbindung(en).

In einer solchen bevorzugten Ausführungsform liegt der Gehalt bezogen auf Natriumedetat unter 100 mg / 100 ml, bevorzugt unter 50 mg/100 ml, besonders bevorzugt unter 20 mg/ 100 ml. Generell sind solche Inhalationslösungen bevorzugt, in denen der Gehalt an Natriumedetat bei 0 bis 10mg/100ml liegt.

Den erfindungsgemäßen treibgasfreien Inhalationslösungen können Co-Solventien und/oder weitere Hilfsstoffe zugesetzt werden.

Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.

Unter Hilfs- und Zusatzstoffen wird in diesem Zusammenhang jeder pharmakologisch verträgliche Stoff verstanden, der kein Wirkstoff ist, aber zusammen mit dem (den) Wirkstoffen(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besonders bevorzugt zwischen 5 und 20 mg/100ml enthalten.

Bevorzugte Formulierungen enthalten außer dem Lösungsmittel Wasser und der Wirkstoffkombination aus **1** und **2** nur noch Benzalkoniumchlorid und Natriumedetat. In einer anderen bevorzugten Ausführungsform wird auf Natriumedetat verzichtet.

Zur Applikation der erfindungsgemäßen treibgasfreien Inhalationslösungen sind besonders solche Inhalatoren, die eine kleine Menge einer flüssigen Formulierung in der therapeutisch notwendigen Dosierung binnen weniger Sekunden in ein therapeutischinhalativ geeignetes Aerosol vernebeln können. Im Rahmen der vorliegenden Erfindung sind solche Vernebler bevorzugt, bei denen bereits eine Menge von weniger als 100 µL, bevorzugt weniger als 50 µL, besonders bevorzugt zwischen 10 und 30 µL Wirkstofflösung mit bevorzugt einem Hub zu einem Aerosol mit einer durchschnittlichen Teilchengröße von weniger als 20 µm, bevorzugt weniger als 10 µm, so vernebelt werden können, dass der inhalierbare Anteil des Aerosols bereits der therapeutisch wirksamen Menge entspricht.

Eine derartige Vorrichtung zur treibgasfreien Verabreichung einer dosierten Menge eines flüssigen Arzneimittels zur inhalativen Anwendung, wird beispielsweise in der internationalen Patentanmeldung WO 91/14468 als auch in der WO 97/12687 (dort insbesondere Figuren 6a und 6b) ausführlich beschrieben. Die dort beschriebenen Vernebler (Devices) sind auch unter der Bezeichnung Respimat^{®} bekannt.

Die vorstehend genannten Vertreter der Wirkstoffe **2** sind im Stand der Technik bekannt. Die Verbindungen der Formel **1** sind dagegen noch nicht im Stand der Technik bekannt. Die nachstehend beschriebenen Synthesebeispiele dienen der Illustration möglicher synthetischer Zugänge zu den neuen Verbindungen der Formel 1. Sie sind allerdings nur als exemplarische Vorgehensweisen zur weitergehenden Erläuterung der Erfindung zu verstehen, ohne selbige auf den nachfolgend exemplarisch beschriebenen Gegenstand zu beschränken.

### Beispiel 1: 1-{3-[2-Hydroxy-2-(6-hydroxy-3-oxo-3,4-dihydro-2H-benzo[1,4]oxazin-8-yl)-ethylamino]-3-methyl-butyl}-6-methoxy-4,4-dimethyl-1,4-dihydro-benzo[d][1,3]oxazin-2-on

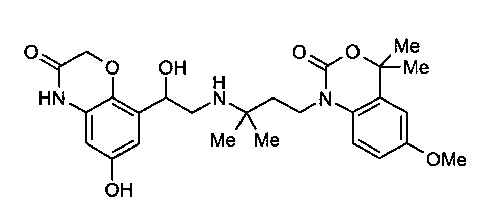

### a) (2-Acetyl-4-methoxy-phenyl)-carbaminsäureethylester

Zu einer Lösung von 82.5 g (0.5 mol) 2-Amino-5-methoxyacetophenon in 400 mL Pyridin werden 65.1 g (0.6 mol) Chlorameisensäureethylester unter Kühlung getropft, so dass die Temperatur nicht über 10-15°C steigt. Anschließend wird die Reaktionsmischung 2 h bei Raumtemperatur gerührt und dann auf Eis gegossen. Der ausfallende Niederschlag wird abgesaugt, mit Wasser gewaschen und aus Isopropanol umkristallisiert. Ausbeute: 102 g (86%); Smp. = 97-100°C.

### b) 6-Methoxy-4,4-dimethyl-1,4-dihydro-benzo[1,3]oxazin-2-on

Zu einer Lösung von 0.5 mol Methylmagnesiumiodid in 200 mL Diethylether werden 47.4 g (0.2 mol) (2-Acetyl-4-methoxy-phenyl)-carbaminsäureethylester, gelöst in 275 mL THF, unter Kühlung so zugetropft, dass die Temperatur nicht über 0°C steigt. Man lässt 30 Minuten bei Raumtemperatur und anschließend 2 Stunde unter Rückfluss rühren. Die Reaktionsmischung wird auf Eis gegossen und mit Ammoniumchlorid versetzt. Nach dem Abtrennen der organischen Phase wird wiederholt mit Ethylacetat extrahiert. Die organischen Phasen werden vereinigt, mit Wasser gewaschen, mit Natriumsulfat getrocknet und eingeengt. Der Rückstand wird in Methanol gelöst und die Lösung wird eingeengt und dann mit Wasser versetzt. Der dabei ausfallende Niederschlag wird abgetrennt, mit Wasser gewaschen und aus Toluol umkristallisiert. Ausbeute: 31.1 g (75%); Smp. = 178-180°C.

### c) 1-(3-Amino-3-methyl-butyl)-6-methoxy-4,4-dimethyl-1,4-dihydrobenzo[1,3]oxazin-2-on

Eine Lösung von 31 g (0.15 mol) 6-Methoxy-4,4-dimethyl-1,4-dihydro-benzo[1,3]oxazin-2-on in 120 mL HMPT wird bei 65-70°C zu 7.2 g Natriumhydrid (55-60 %ig) in 30 mL HMPT getropft. Nach beendeter Wasserstofffreisetzung wird noch 20 Minuten nachgerührt und dann auf Raumtemperatur abgekühlt. Bei dieser Temperatur werden 37.7 g (0.18 mol) (3-Chlor-1,1-dimethyl-propyl)-benzylidenamin, gelöst in 40 mL HMPT, zugegeben. Nach 3 Stunden Rühren bei 100°C wird die Reaktionsmischung auf Eis gegossen und mit Ethylacetat extrahiert. Die organischen Phasen werden mit Wasser gewaschen, mit Natrumsulfat getrocknet und eingeengt. Der Rückstand wird unter Erwärmung in 1 N Salzsäure gelöst und nach Abkühlung mit Diethylether extrahiert. Die wässrige Phase wird mit Natronlauge alkalisch gestellt und mit Ethylacetat ausgeschüttelt. Anschließend wird die organische Phase mit Natriumsulfat getrocknet und vom Lösungsmittel befreit. Aus dem Rückstand wird nach Lösen in Acetonitril und Zugabe von etherischer Salzsäure das Produkt in Form seines Hydrochlorids isoliert. Ausbeute: 34.3 g (70 %).

### d) 1-{3-[2-(6-Benzyloxy-3-oxo-3,4-dihydro-2H-benzo[1,4]oxazin-8-yl)-2-hydroxyethylaminol-3-methyl-butyl}-6-methoxy-4,4-dimethyl-1,4-dihydro-benzo[1,3]oxazin-2-on

357 mg (1 mmol) 6-Benzyloxy-8-(2-ethoxy-2-hydroxy-acetyl)-4H-benzo[1,4]oxazin-3-on und 292 mg (1 mmol) 1-(3-Amino-3-methyl-butyl)-6-methoxy-4,4-dimethyl-1,4-dihydrobenzo[1,3]oxazin-2-on werden in 5 mL Ethanol suspendiert und auf 70°C erwärmt. Die entstandene Lösung wird eine Stunde bei 70°C gerührt und dann auf Raumtemperatur abgekühlt. Nach Zugabe von 113 mg (3 mmol) Natriumborhydrid wird 3 Stunden bei Raumtemperatur gerührt, mit 0.7 mL gesättigter Kaliumcarbonatlösung versetzt und weitere 30 Minuten gerührt. Es wird über Aluminiumoxid (basisch) filtriert, wiederholt mit Methylenchlorid/Methanol 15:1 nachgewaschen und eingeengt. Das so erhaltene Rohprodukt wird chromatographisch (Methylenchlorid mit Methanol/Ammoniak-Gradient (9:1)) gereinigt. Beigefarbener Feststoff. Ausbeute: 340 mg (58%); Massenspektrometrie:
[M+H]⁺ = 590.

### e) 1-{3-[2-Hydroxy-2-(6-hydroxy-3-oxo-3,4-dihydro-2H-benzo[1,4]oxazin-8-yl)-ethylaminol-3-methyl-butyl}-6-methoxy-4,4-dimethyl-1,4-dihydro-benzo[d][1,3]oxazin-2-on

340 mg (0.58 mmol) 1-{3-[2-(6-Benzyloxy-3-oxo-3,4-dihydro-2H-benzo[1,4]oxazin-8-yl)-2-hydroxy-ethylamino]-3-methyl-butyl}-6-methoxy-4,4-dimethyl-1,4-dihydrobenzo[1,3]oxazin-2-on werden in 10 mL Methanol gelöst und mit Palladium auf Kohle als Katalysator bei 1 bar Wasserstoffdruck hydriert. Anschließend wird der Katalysator abfiltriert und das Filtrat eingeengt. Beigefarbener Feststoff. Ausbeute: 273 mg (95%); Massenspektrometrie: [M+H]⁺ = 500; Rf-Wert = 0.33 (Methylenchlorid : Methanol : Ammoniak = 9 : 1 : 0.1).

### Beispiel 2: 6-Hydroxy-1-{3-[2-hydroxy-2-(6-hydroxy-3-oxo-3,4-dihydro-2H-benzo[1,4]oxazin-8-yl)-ethylamino]-3-methyl-butyl}-4,4-dimethyl-1,4-dihydrobenzo[d][1,3]oxazin-2-on

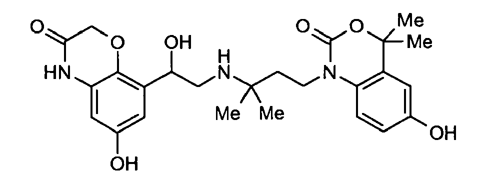

### a) 6-Benzyloxy-4,4-dimethyl-1,4-dihydro-benzo[1,3]oxazin-2-on

Die Herstellung erfolgt in Analogie zur Vorschrift für Beispiel 1b) aus 15.7 g (50 mmol) (2-Acetyl-4-benzyloxy-phenyl)-carbaminsäureethylester und 125 mmol Methylmagnesiumiodid. Ausbeute: 10.8 g (76%); Smp. = 134°C.

### b) 1-(3-Amino-3-methyl-butyl)-6-benzyloxy-4,4-dimethyl-1,4-dihydrobenzo[1,3]oxazin-2-on

In Analogie zur Vorschrift für Beispiel 1c) aus 10.5 g (37 mmol) 6-Benzyloxy-4,4-dimethyl-1,4-dihydro-benzo[1,3]oxazin-2-on und 9.3 g (44 mmol) (3-Chlor-1,1-dimethylpropyl)-benzylidenamin hergestellt. Ausbeute: 10.9 g (73%); Smp. = 233°C (Hydrochlorid).

### c) 6-Hydroxy-1-{3-[2-hydroxy-2-(6-hydroxy-3-oxo-3,4-dihydro-2H-benzo[1,4]oxazin-8-yl)-ethylamino]-3-methyl-butyl}-4,4-dimethyl-1,4-dihydrobenzo[1,3]oxazin-2-one

Die Umsetzung von 357 mg (1 mmol) 6-Benzyloxy-8-(2-ethoxy-2-hydroxy-acetyl)-4H-benzo[1,4]oxazin-3-on und 368 mg (1 mmol) 1-(3-Amino-3-methyl-butyl)-6-benzyloxy-4,4-dimethyl-1,4-dihydro-benzo[1,3]oxazin-2-on in Analogie zu den Vorschriften für Beispiel 1 liefert die Verbindung in Form eines beigefarbenen Feststoffes. Ausbeute 355 mg (73%); Massenspektrometrie: [M+H]⁺ = 486.

### Beispiel 3 : 4,4-Diethyl-1-{3-[2-hydroxy-2-(6-hydroxy-3-oxo-3 ,4-dihydro-2H-benzo[1,4]oxazin-8-yl)-ethylamino]-3-methyl}-1,4-dihydro-benzo[d][1,3]oxazin-2-on

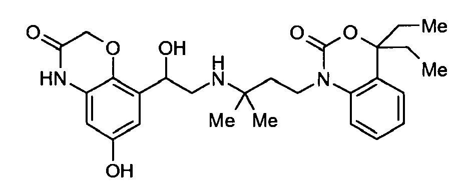

### a) 4,4-Diethyl-1,4-dihydro-benzo[1,3]oxazin-2-on

Erhalten aus der Reaktion von 67 g (0.3 mol) 2-Ethoxycarbonylaminobenzoesäuremethylester und 1.14 mol Ethylmagnesiumiodid in Analogie zur Vorschrift für Beispiel 1b). Ausbeute: 48.5 g (79%); Smp. = 160-162°C.

### b) 1-(3-Amino-3-methyl-butyl)-4,4-diethyl-1,4-dihydro-benzo[1,3]oxazin-2-on

Hergestellt aus 47.5 g (0.23 mol) 4,4-Diethyl-1,4-dihydro-benzo[1,3]oxazin-2-on und 57.5 g (0.27 mol) (3-Chlor-1,1-dimethyl-propyl)-benzylidenamin nach dem für Beispiel 1c) beschriebenen Verfahren. Ausbeute: 38.1 g (50%); Smp. = 208-210°C (Hydrochlorid).

### c) 4,4-Diethyl-1-{3-[2-hydrox-2-(6-hydroxy-3-oxo-3,4-dihydro-2H-benzo[1,4]oxazin-8-yl)-ethylamino]-3-methyl-butyl}-1,4-dihydro-benzo[d]1,3]oxazin-2-on

357 mg (1 mmol) 6-Benzyloxy-8-(2-ethoxy-2-hydroxy-acetyl)-4H-benzo[1,4]oxazin-3-on und 290 mg (1 mmol) 1-(3-Amino-3-methyl-butyl)-4,4-diethyl-1,4-dihydrobenzo[1,3]oxazin-2-on werden in Analogie zu den Vorschriften für Beispiel 1 umgesetzt. Nach anschließender Debenzylierung wird ein beigefarbener Feststoff erhalten. Ausbeute: 367 mg (74%); Massenspektrometrie: [M+H]⁺ = 498.

### Beispiel 4: 1-{3-[2-Hydroxy-2-(6-hydroxy-3-oxo-3,4-dihydro-2H-benzo[1,4]oxazin-8-yl)-ethylamino]-3-methyl-buty}-4,4-dimethyl-1,4-dihydro-benzo[d][1,3]oxazin-2-on

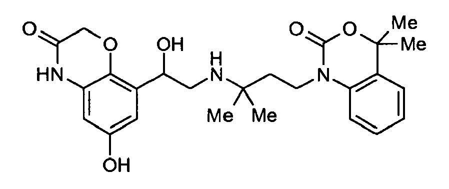

### a) 4,4-Dimethyl-1,4-dihydro-benzo[1,3]oxazin-2-on

112 g (1.13 mol) Phosgen werden in 500 mL THF eingeleitet. Anschließend wird eine Lösung von 52 g (0.34 mol) 2-(2-Amino-phenyl)-propan-2-ol, hergestellt aus 2-Aminoacetophenon und Methylmagnesiumiodid, in 300 mL THF zugegeben. Die Reaktionsmischung wird über Nacht stehen gelassen, eingeengt und mit 500 ml Pyridin versetzt. Nach dem Abdestillieren des Pyridins wird mit Wasser versetzt und mit Diethylether extrahiert. Die organischen Phasen werden nacheinander mit 2 N Salzsäure, Natriumhydroxid-Lösung und Wasser gewaschen, mit Natriumsulfat getrocknet und eingeengt. Der verbleibende Rückstand (46 g) wird ohne weitere Aufreinigung direkt weiter umgesetzt. Smp. (Toluol/Petrotether) = 109-110°C.

### b) 1-(3-Amino-3-methyl-butyl)-4,4-dimethyl-1,4-dihydro-benzo[1,3]oxazin-2-on

Erhalten aus 43 g (0.24 mol) 4,4-Dimethyl-1,4-dihydro-benzo[1,3]oxazin-2-on und 54 g (0.26 mol) (3-Chlor-1,1-dimethyl-propyl)-benzylidenamin in Analogie zu der für Beispiel 1c) beschriebenen Verfahren. Ausbeute 41 g (57%); Smp. (nach Umkristallisation aus Ethanol) = 262°C (Hydrochlorid).

### c) 1-{3-[2-Hydroxy-2-(6-hydroxy-3-oxo-3,4-dihydro-2H-benzo[1,4]oxazin-8-yl)-ethylamino]-3-methyl-butyl}-4,4-dimethyl-1,4-dihydro-benzo[d][1,3]oxazin-2-on

357 mg (1 mmol) 6-Benzyloxy-8-(2-ethoxy-2-hydroxy-acetyl)-4H-benzo[1,4]oxazin-3-on und 262 mg (1 mmol) 1-(3-Amino-3-methyl-butyl)-4,4-dimethyl-1,4-dihydrobenzo[1,3]oxazin-2-on werden in der für Beispiel 1 ausgeführten Weise umgesetzt. Nach anschließender Hydrierung wird ein beigefarbener Feststoff isoliert. Ausbeute: 285 mg (61 %); Massenspektrometrie [M+H]⁺ = 470.

### Beispiel 5: 1-{3-[2-Hydroxy-2-6-hydroxy-3-oxo-3,4-dihydro-2H-benzo[1,4]oxazin-8-yl)-ethylamino]-3-methyl-butyl}-1,4-dihydro-benzo[1,3]oxazin-2-on

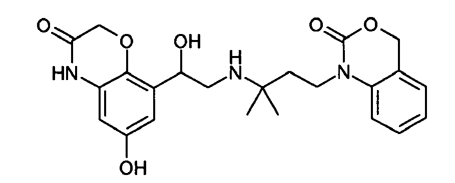

### a) 1-(3-Amino-3-methyl-butyl)-1,4-dihydro-benzo[1,3]oxazin-2-on

2.70 g (18 mmol) 1,4-Dihydro-benzo[1,3]oxazin-2-on und 4.35 g (21 mmol) (3-Chlor-1,1-dimethyl-propyl)-(1-phenyl-methyliden)-amin werden in der für Beispiel 6a) beschriebenen Weise umgesetzt. Zur Aufarbeitung wird das Reaktionsgemisch auf Eiswasser gegossen und mit Ethylacetat extrahiert. Die organischen Phasen werden mit Wasser gewaschen, mit Natriumsulfat getrocknet und eingeengt. Der Rückstand wird mit 25 mL 2 N Salzsäure versetzt und auf 70°C erwärmt. Nach Abkühlung auf Raumtemperatur wird mit Diethylether extrahiert. Die wässrige Phase wird eingeengt und mit Acetonitril versetzt. Der ausfallende Niederschlag wird abgesaugt und mit Acetonitril und Diethylether gewaschen. Ausbeute: 2.65 g (54%, Hydrochlorid); Schmelzbereich: 220°C (Zersetzung).

### b) 1-{3-[2-(6-Benzyloxy-3-oxo-3,4-dihydro-2H-benzo[1,4]oxazin-8-yl)-2-hydroxyethylamino]-3-methyl-butyl}-1,4-dihydro-benzo[1,3]oxazin-2-on

357 mg (1 mmol) 6-Benzyloxy-8-(2-ethoxy-2-hydroxy-acetyl)-4H-benzo[1,4]oxazin-3-on und 234 mg (1 mmol) 1-(3-Amino-3-methyl-butyl)-1,4-dihydro-benzo[1,3]oxazin-2-on in 5 mL Tetrahydrofuran werden 15 Minuten bei 60°C gerührt. Man kühlt auf 0°C ab und tropft unter Argonatmosphäre 1.5 mL einer 2 molaren Lösung von Lithiumborhydrid in Tetrahydrofuran zu. Es wird 15 min bei 0°C gerührt, mit 10 mL Dichlormethan und 3 mL Wasser versetzt, eine weitere Stunde gerührt und dann über Kieselgur filtriert. Man eluiert mit Dichlormethan und destilliert die Lösungsmittel ab. Der Rückstand wird mittels präparativer HPLC (Reverse Phase, Acetonitril/Wasser-Gradient mit 0.1% Trifluoressigsäure) gereinigt. Ausbeute: 196 mg (30%, Trifluoracetat); Massenspektroskopie: [M]⁺ = 532.

### c) 1-{3-[2-(6-Benzyloxy-3-oxo-3,4-dihydro-2H-benzo[1,4]oxazin-8-yl)-2-hydroxyethylamino]-3-methyl-butyl}-1,4-dihydro-benzo[1,3]oxazin-2-on

196 mg (0.3 mmol) 1-{3-[2-(6-Benzyloxy-3-oxo-3,4-dihydro-2H-benzo[1,4]oxazin-8-yl)-2-hydroxy-ethylamino]-3-methyl-butyl}-1,4-dihydro-benzo[1,3]oxazin-2-on werden in 5 Ethanol gelöst und mit Palladium auf Kohle (10%ig) als Katalysator bei 3 bar und Raumtemperatur hydriert. Der Katalysator wird abgetrennt und das Rohprodukt in Acetonitril/Diethylether auskristallisiert. Ausbeute: 48 mg (29%, Trifluorethylacetat); Massenspektroskopie: [M+H]⁺ = 442.

### Beispiel 6: 4-Ethyl-1-{3-[2-hydroxy-2-(6-hydroxy-3-oxo-3,4-dihydro-2H-benzo[1,4]oxazin-8-yl)-ethylamino]-3-methyl-butyl}-1,4-dihydro-benzo[1,3]oxazin-2-on

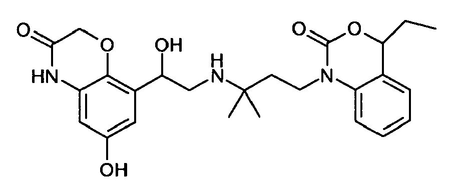

### a) 1-(3-Amino-3-methyl-butyl)-4-ethyl-1,4-dihydro-benzo[1,4]oxazin

Eine Lösung von 17.7 g (0.10 mol) 4-Ethyl-1,4-dihydro-benzo[1,3]oxazin-2-on in 85 mL HMPT wird mit 4.8 g Natriumhydrid (55-60%) versetzt und langsam auf 60°C erwärmt. Nach beendeter Wasserstoffentwicklung wird noch 30 min bei 80°C gerührt und dann auf Raumtemperatur abgekühlt. 25 g (0.12 mol) (3-Chlor-1,1-dimethyl-propyl)-(1-phenylmethyliden)-amin, gelöst in 25 mL HMPT, werden zugegeben und man läßt drei Stunden bei 100°C rühren. Die Reaktionsmischung wird abgekühlt, auf Eiswasser gegossen und mit Ethylacetat extrahiert. Die vereinigten organischen Phasen werden mit Wasser gewaschen, mit Natriumsulfat getrocknet und eingeengt. Der Rückstand wird zusammen mit 240 mL 1N Salzsäure auf 60°C erwärmt und nach Abkühlung mit Diethylether extrahiert. Die wässrige Phase wird mit konz. Natronlauge alkalisch gestellt und mit Ethylacetat extrahiert. Die vereinigten organischen Phasen werden mit Natriumsulfat getrocknet und eingeengt. Der Rückstand wird unter Erwärmung in Ethylacetat gelöst, mit einer äquimolaren Mengen Maleinsäure versetzt und langsam abgekühlt. Der ausfallende Niederschlag wird abgesaugt, mit Ethylacetat gewaschen und getrocknet. Ausbeute: 26.1 g (69%, Maleat); Schmelzbereich: 134°C.

### b) 1-{3-[2-(6-Benzyloxy-3-oxo-3,4-dihydro-2H-benzo[1,4]oxazin-8-yl)-2-hydroxyethylamino]-3-methyl-butyl}-4-ethyl-1,4-dihydro-benzo[1,3]oxazin-2-on

357 mg (1 mmol) 6-Benzyloxy-8-(2-ethoxy-2-hydroxy-acetyl)-4H-benzo[1,4]oxazin-3-on und 530 mg (1 mmol) 1-(3-Amino-3-methyl-butyl)-4-ethyl-1,4-dihydro-benzo[1,4]oxazin werden in Analogie zur Vorschrift 5b) umgesetzt und aufgearbeitet.
Ausbeute: 308 mg (46%, Trifluoracetat); Massenspektroskopie: [M]⁺ = 560.

### c) 4-Ethyl-1-{3-[2-hydroxy-2-(6-hydroxy-3-oxo-3,4-dihydro-2H-benzo[1,4]oxazin-8-yl)-ethylamino]-3-methyl-butyl}-1,4-dihydro-benzo[1,3]oxazin-2-on

308 mg (0.46 mmol) 1-{3-[2-(6-Benzyloxy-3-oxo-3,4-dihydro-2H-benzo[1,4]oxazin-8-yl)-2-hydroxy-ethylamino]-3-methyl-butyl}-4-ethyl-1,4-dihydro-benzo[1,3]oxazin-2-on werden mit Palladium auf Kohle (10%ig) als Katalysator bei Raumtemperatur und 3 bar Wasserstoffdruck hydriert. Der Katalysator wird abgetrennt, das Filtrat eingeengt und der Rückstand chromatographiert (Reverse Phase; Acetonitril/Wasser-Gradient). Ausbeute: 14 mg (5%, Trifluoracetat); Massenspektroskopie: [M]⁺ = 470.

### Beispiel 7: 8-{2-[1,1-Dimethyl-3-(2-oxo-3,4-dihydro-2H-chinolin-1-yl)-propylamino]1-1-hydroxy-ethyl}-6-hydroxy-4H-benzo[1,4]oxazin-3-on

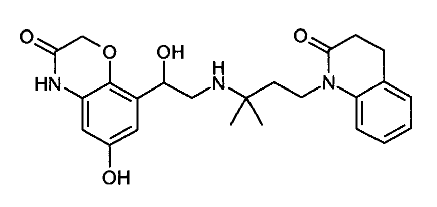

### a) 1-(3-Amino-3-methyl-butyl)-3,4-dihydro-chinolin-2-on

Die Herstellung erfolgt in Analogie zu dem für Beispiel 6a) beschriebenen Verfahren aus 15.7 g (107 mmol) 3,4-Dihydro-chinolin-2-on und 24.9 g (119 mmol) (3-Chlor-1,1-dimethyl-propyl)-(1-phenyl-methyliden)-amin. In Abweichung zur vorstehend genannten Vorschrift wird das Produkt nicht als Maleat, sondern als Hydrochlorid ausgefällt. Ausbeute: 6.9 g (24%, Hydrochlorid); Schmelzbereich: 200-203°C.

### b) 8-{2-[1,1-Dimethyl-3-(2-oxo-3,4-dihydro-2H-chinolin-1-yl)-Propylamino]-1-hydroxyethyl}-6-hydroxy-4H-benzo[1,4]oxazin-3-on

Herstellung aus 357 mg (1 mmol) 6-Benzyloxy-8-(2-ethoxy-2-hydroxy-acetyl)-4H-benzo[1,4]oxazin-3-on und 232 mg (1 mmol) 1-(3-Amino-3-methyl-butyl)-3,4-dihydrochinolin-2-on in Analogie zu dem für Beispiel 5c) und beschriebenen Verfahren. Die abschließende Reinigung des Produkts erfolgt mittels präparativer HPLC (Reverse Phase, Acetonitril/Wasser-Gradient mit 0.1% Trifluoressigsäure). Ausbeute: 94 mg (17%, Trifluoracetat); Massenspektroskopie: [M]⁺ = 440.

### Beispiel 8: 4,4-Diethyl-1-{3-[2-hydroxy-(8-hydroxy-2-oxo-1,2-dihydro-chinolin-5-yl)-ethylamino]-3-methyl-butyl}-1,4-dihydro-benzo[1,3]oxazin-2-on

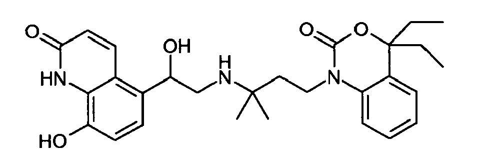

### a) 1-{3-[2-(8-Benzyloxy-2-oxo-1,2-dihydro-chinolin-5-yl)-2-hydroxy-ethylamino]-3-methyl-butyl}-4,4-diethyl-1,4-dihydro-benzo[1,3]oxazin-2-on

400 mg (1.4 mmol) 8-Benzyloxy-5-oxiranyl-chinolin-2-on und 436 mg (1.5 mmol) 1-(3-Amino-3-methyl-butyl)-4,4-diethyl-1,4-dihydro-benzo[1,3]oxazin-2-on in 5 mL n-Butanol werden 6 Stunden bei 140°C gerührt. Das Lösungsmittel wird abdestilliert und der Rückstand chromatographisch (Reverse Phase; Acetonitril/Wasser-Gradient) gereinigt. Beigefarbener Feststoff. Ausbeute: 160 mg (20%); Massenspektroskopie: [M]⁺ = 584.

### b) 4,4-Diethyl-1-{(3-[2-hydroxy-2-(8-hydroxy-2-oxo-1,2-dihydro-chinolin-5-yl)-ethylamino]-3-methyl-butyl}-1,4-dihydro-benzo[1,3]oxazin-2-on

160 mg (0.3 mmol) 1-{3-[2-(8-Benzyloxy-2-oxo-1,2-dihydro-chinolin-5-yl)-2-hydroxyethylamino]-3-methyl-butyl}-4,4-diethyl-1,4-dihydro-benzo[1,3]oxazin-2-on werden in 5 mL Methanol gelöst und in Gegenwart von Palladium auf Kohle (10%) hydriert. Ausbeute: 49 mg (34%); Massenspektroskopie: [M]⁺ = 494.

### Beispiel 9: 4,4-Diethyl-1-{3-[2-hydroxy-2-(6-hydroxy-2,2-dimethyl-3-oxo-3,4-dihydro-2H-benzo[1,4]oxazin-8-yl)-ethylamino]-3-methyl-butyl}-1,4-dihydro-benzo[d][1,3]oxazin-2-on

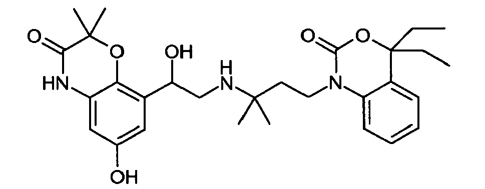

### a) N-(3-Acetyl-5-benzyloxy-2-hydroxy-phenyl)-2-brom-2-methyl-propionamid

Zu einer Lösung von 5.15 g (20 mmol) 1-(3-Amino-5-benzyloxy-2-hydroxy-phenyl)-ethanon in 20 mL Pyridin werden bei 5-20°C 4.64 g (25 mmol) 2-Brom-2-methylpropionylchlorid getropft. Nach beendeter Zugabe wird 15 Minuten gerührt, mit Eiswasser und 100 mL Ethylacetat versetzt und mit konz. Salzsäure angesäuert. Die organische Phase wird abgetrennt, mit Wasser gewaschen und mit Natriumsulfat getrocknet. Nach dem Abdestillieren des Lösungsmittels wird der Rückstand aus einem Diethylether/Petrolether-Gemisch kristallisiert. Ausbeute: 6.8 g (84%); Schmelzbereich: 88-90°C.

### b) 8-Acetyl-6-benzyloxy-2,2-dimethyl-4H-benzo[1,4]oxazin-3-on

6.60 g (16.2 mmol) N-(3-Acetyl-5-benzyloxy-2-hydroxy-phenyl)-2-brom-2-methylpropionamid und 2.76 g (20 mmol) Kaliumcarbonat werden 1 Stunde in 70 mL Acetonitril unter Rückfluß gerührt. Der Feststoff wird abgesaugt, das Filtrat eingeengt und der Rückstand mit 30 mL Ethylacetat versetzt. Nach erneuter Filtration und dem Abdestillieren des Lösungsmittels wird das Rohprodukt aus wenig Methanol kristallisiert. Ausbeute: 1.00 g (19%); Massenspektroskopie [M+H]⁺ = 326; Schmelzbereich = 148-150°C.

### c) 6-Benzyloxy-8-(2-ethoxy-2-hydroxy-acetyl)-2,2-dimethyl-benzo[1,4]oxazin-3-on

50.12 g (154 mmol) 8-Acetyl-6-benzyloxy-2,2-dimethyl-benzo[1,4]oxazin-3-on werden mit Selendioxid als Oxidationsmittel und Aktivkohle in refluxierendem Dioxan und etwas Wasser umgesetzt. Nach Abkühlung wird der Feststoff abfiltriert und mit Dioxan gewaschen. Das Filtrat wird eingeengt und der Rückstand in 550 mL Ethanol gelöst und für 30 Minuten unter Rückfluß erhitzt. Es wird filtriert und die Mutterlauge wird auf -18°C gekühlt, wobei ein Feststoff ausfällt, der abgesaugt wird. Nach Umkristallisation aus Ethanol liegt das Produkt in Form eines beigefarbenen Feststoffs vor. Ausbeute: 8.95 g (15%).

### d) 1-{3-[2-(6-Benzyloxy-2,2-dimethyl-3-oxo-3,4-dihydro-2H-benzo[1,4]oxazin-8-yl)-2-hydroxy-ethylamino] 3-methyl-butyl}-4,4-diethyl-1,4-dihydro-benzo[d][1,3]oxazin-2-on

Herstellung aus 406 mg (1 mmol) 6-Benzyloxy-8-(2-ethoxy-2-hydroxy-acetyl)-2,2-dimethyl-benzo[1,4]oxazin-3-on und 290 mg (1 mmol) 1-(3-Amino-3-methyl-butyl)-4,4-diethyl-1,4-dihydro-benzo[1,3]oxazin-2-on analog zu dem für Beispiel 5b) beschriebenen Verfahren. Die Zielverbindung wird mittels Chromatographie an einer kurzen Säule mit Kieselgel (Dichlormethan/Methanol-Gradient) gereinigt. Weißer Feststoff Ausbeute: 145 mg (24%); Massenspektroskopie [M+H]⁺ = 616.

### e) 4,4-Diethyl-1-{3-[2-hydroxy-2-(6-hydroxy-2,2-dimethyl-3-oxo-3,4-dihydro-2H-benzo[1,4]oxazin-8-yl)-ethylamino]-3-methyl-butyl}-1,4-dihydro-benzo[d][1,3]oxazin-2-on

130 mg (0.21 mmol) 1-{3-[2-(6-Benzyloxy-2,2-dimethyl-3-oxo-3,4-dihydro-2H-benzo[1,4]oxazin-8-yl)-2-hydroxy-ethylamino]-3-methyl-butyl}-4,4-diethyl-1,4-dihydrobenzo[d][1,3]oxazin-2-on werden in 5 mL Methanol gelöst und in Gegenwart von Palladium auf Kohle (10%ig) bei Raumtemperatur hydriert. Der Katalysator wird abgesaugt, das Filtrat eingeengt und der Rückstand chromatographisch gereinigt (Reverse Phase; Acetonitril/Wasser-Gradient). Weißer Feststoff. Ausbeute: 41 mg (37%); Massenspektroskopie [M+H]⁺ = 526.

### Beispiel 10: 4,4-Diethyl-1-{3-[2-hydroxy-2-(5-hydroxy-2-oxo-2,3-dihydro-benzooxazol-7-yl)-ethylamino]-3-methyl-butyl}-1,4-dihydro-benzo[d][1,3]oxazin-2-on

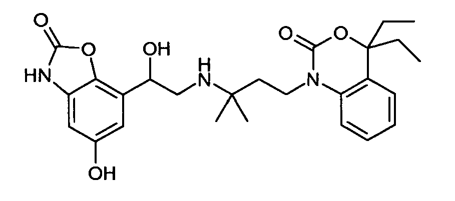

### a) 7-Acetyl-5-benzyloxy-3H-benzooxazol-2-on

Zu einer Lösung von 22.7 g (88.22 mmol) 1-(3-Amino-5-benzyloxy-2-hydroxy-phenyl)-ethanon in Toluol (200 mL) werden bei 0 °C 51.1 mL (97.04 mmol) einer Phosgen-Lösung (20 gew.% in Toluol) zugegeben. Anschließend werden 30.7 mL (220.6 mmol) Triethylamin so zugetropft, dass die Temperatur 5 °C nicht überschreitet. Nach 1 h Rühren bei Raumtemperatur werden weitere 4.6 mL Phosgen-Lösung und 12 mL Triethylamin bei 0 °C zugegeben. Es wird 1 h bei Raumtemperatur gerührt, mit Dichlormethan verdünnt und mit gesättigter wässriger Ammoniumchlorid-Lösung (500 mL) und 2 N wässriger Salzsäure (10 mL) versetzt. Nach dem Abtrennen der wässrigen Phase wird diese erschöpfend mit Dichlormethan extrahiert. Die vereinigten organischen Phasen werden mit Wasser und gesättigter wässriger Natriumchlorid-Lösung gewaschen, mit Natriumsulfat getrocknet und i. vac. eingeengt, wobei sich ein beiger Feststoff ausfällt. Der Niederschlag wird abfiltriert, mit wenig Toluol gewaschen und bei 50 °C i. vac. getrocknet.

Ausbeute: 18.5 g (74%); R_{f} = 0.19 (Kieselgel, Toluol/Aceton 95:10); ESI-MS: [M+H]⁺ = 284.

### b) 5-Benzyloxy-7-(2-ethoxy-2-hydroxy-acetyl)-3H-benzooxazol-2-on

Zu einer Lösung von 12.0 g (42.4 mmol) 7-Acetyl-5-benzyloxy-3*H*-benzooxazol-2-on in DMSO (60 mL) werden 14.4 mL (127.1 mmol) HBr (48% in Wasser) zugegeben. Das Gemisch wird unter einem leichten Stickstoffstrom 6 h bei 60 °C gerührt, auf 600 mL Eiswasser gegossen und 20 Min. gerührt. Der gebildete Niederschlag wird abfiltriert und mit Eiswasser und kalter Wasser/Ethylacetat-Lösung (1:1) gewaschen. Der Niederschlag wird in 300 mL Ethanol und 100 mL Ethylacetat gelöst und i. vac. eingeengt. Die Prozedur wird mit 500 mL Toluol und dann mit 500 mL Ethanol wiederholt. Der Rückstand wird anschließend in 250 mL Ethanol gelöst und 1 h unter Rückfluß erhitzt. Nach dem Abdestillieren von 30 mL Ethanol wird das Gemisch auf Raumtemperatur und dann auf 0°C gekühlt. Der gebildete Niederschlag wird abfiltriert, mit 80 mL eiskaltem Ethanol und 200 mL Ether gewaschen und bei 50 °C i. vac. getrocknet. Ausbeute: 6.5 g (45%); R_{f} = 0.23 (Kieselgel, Dichlormethan/MeOH 25:2); ESI-MS: [M+H-CO₂Et]⁺ = 270.

### c) 1-{3-[2-(5-Benzyloxy-2-oxo-2,3-dihydro-benzooxazol-7-yl)-2-hydroxy-ethylamino]-3-methyl-buty}-4,4-diethyl-1,4-dihydro-benzo[d][1,3]oxazin-2-on

Erhalten aus 343 mg (1 mmol) 5-Benzyloxy-7-(2-ethoxy-2-hydroxy-acetyl)-3*H-*benzooxazol-2-on und 290 mg (1 mmol) 1-(3-Amino-3-methyl-butyl)-4,4-diethyl-1,4-dihydro-benzo[1,3]oxazin-2-on nach dem für Beispiel 5b) beschriebenen Verfahren. Weißer Feststoff. Ausbeute: 160 mg (28%); Massenspektroskopie [M-H]⁺ = 572.

### d) 4,4-Diethyl-1-{3-[2-hydroxy-2-(5-hydroxy-2-oxo-2,3-dihydro-benzooxazol-7-yl)-ethylamino]-3-methyl-butyl}-1,4-dihydro-benzo[d][1,3]oxazin-2-on

150 mg (0.26 mmol) 1-{3-[2-(5-Benzyloxy-2-oxo-2,3-dihydro-benzooxazol-7-yl)-2-hydroxy-ethylamino]-3-methyl-butyl}-4,4-diethyl-1,4-dihydro-benzo[d][1,3]oxazin-2-on werden in 5 mL Methanol gelöst und mit Palladium auf Kohle als Katalysator 3 Stunde bei Raumtemperatur hydriert. Der Katalysator wird abgetrennt und das Filtrat eingeengt. Beigefarbener Feststoff. Ausbeute: 116 mg (92%); Massenspektroskopie [M-H]⁺ = 484.

HPLC-Methode (Methode A): Symmetry C18 (Waters); 3.5 µm; 4.6 x 150 mm; Säulentemperatur: 20°C; Gradient Acetonitril/Phosphat-Puffer (pH 7) 20:80 → 80:20 in 30 Min., Fluss: 1.0 mL / min; Detektion bei 220 und 254 nm.

### Beispiel 11:4,4-Diethyl-1-{3-[2-hydroxy-2-(7-hydroxy-2-oxo-1,2-dihydro-quinolin-5-yl)-ethylamino]-3-methyl-butyl}-1,4-dihydro-benzo[d][1,3]oxazin-2-on

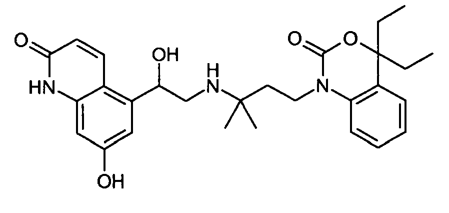

### a) Trifluormethansulfonsäure-2-acetyl-4-benzyloxy-6-nitro-phenylester

Zu einer Lösung von 1-(5-Benzyloxy-2-hydroxy-3-nitro-phenyl)-ethanon (90.0 g, 0.313 mol) in abs. Dichlormethan (940 mL) wird bei -10 °C innerhalb von 10 Min. Triethylamin (92.7 mL, 0.660 mol) zugegeben. Zu dieser roten Lösung wird innerhalb von 15 Min. eine Lösung von Trifuormethansulfonsäureanhydrid (65 mL, 0.394 mol) in abs. Dichlormethan (40 mL) zugegeben und es wird weitere 5 Min. bei -5 °C gerührt. Die braune Lösung wird mit ges. wässr. Ammoniumchlorid (400mL) und ges. wässr. NaCl (400mL) gewaschen und die Phasen werden getrennt. Trocknen mit Natriumsulfat und Einengen i. vac. liefert das Rohprodukt als Öl, das beim Stehen fest wird. Das Rohprodukt wird in Ether (150 mL) gelöst, die Lösung mit Hexan (800 mL) versetzt und der gebildete Niederschlag abfiltriert. Der Feststoff wird mit Ether/Hexan (80/20, 100 mL) verrührt, abfiltriert und im Ofen bei 40 °C getrocknet. Ausbeute: 118 g (90%); ESI-MS: [M+H]⁺ = 420.

### b) 3-(2-Acetyl-4-benzyloxy-6-nitrophenyl)-acrylsäuremethylester

Zu einer Lösung von Trifluormethansulfonsäure-2-acetyl-4-benzyloxy-6-nitro-phenylester (100.0 g, 0.238 mol) in Dioxan (360 mL) werden unter einer Stickstoffatmosphäre 100 g Molekularsieb (4 Å), Tris(dibenzylidenaceton)dipalladium (5.88 g, 6.42 mmol), Tri-*tert-*butylphophonium-tetrafluorborat (3.50 g, 12.06 mmol), Dicyclohexylmethylamin (81.2 mL, 0.371 mol), getrocknetes Tetrabutylammoniumiodid (105.8 g, 0.286 mol) und Methylacrylat (32.6 mL, 0.362 mol) zugegeben. Das Reaktionsgemisch wird 2 Stunden bei 80 °C gerührt, mit Ether (2 L) verdünnt und mit 500 g Kieselgel versetzt. Die Suspension

wird 10 Min. gerührt, filtriert und das Kieselgel mehrmals mit Ether gewaschen (4 × 600 mL). Die vereinigten organischen Phasen werden mit 1 M wässriger Salzsäure (300 mL), Natriumbicarbonat-Lösung und Natriumchlorid-Lösung gewaschen, mit Natriumsulfat getrocknet und eingeengt. Das ölige Rohprodukt wird aus heißem Ethanol (0.75 L) umkristallisiert. Der Niederschlag wird abfiltriert, mit Ethanol gewaschen (2 × 50 mL) und bei 40 °C getrocknet. Ausbeute: 32.2 g (38%); Massenspektroskopie: [M+H]⁺ = 356.

### c) 5-Acetyl-7-benzyloxy-3,4-dihydro-1H-chinolin-2-on

Eine Suspension von 3-(2-Acetyl-4-benzyloxy-6-nitrophenyl)-acrylsäure-methylester (5.0 g, 14.07 mmol) in Ethanol (100 mL) wird mit Raney-Nickel (3 g) bei Raumtemperatur und 4 bar Wasserstoffdruck hydriert. Nach 6 Stunden wird erneut Raney-Nickel (2 g) zugegeben und das Gemisch weitere 2 Stunden hydriert. Der Katalysator wird abgetrennt und das Filtrat mit 2 M wässriger Salzsäure (15 mL) versetzt. Das auskristallisierende Produkt wird abfiltriert und getrocknet. Ausbeute: 1.0 g (24%); Massenspektroskopie:
[M+H]⁺ = 296.

### d) 5-Acetyl-7-benzyloxy-1H-chinolin-2-on

Zu einer Suspension von 5-Acetyl-7-benzyloxy-3,4-dihydro-1H-chinolin-2-on (13.0 g, 44.02 mmol) in Dioxan (130 mL) wird DDQ (15.0 g, 66.08 mmol) zugegeben und es wird 30 Minuten unter Rückfluß erhitzt. Das Reaktionsgemisch wird auf Raumtemperatur gekühlt und weitere 2 Stunden gerührt. Der gebildete Niederschlag wird abfiltriert, mit Dioxan (2 × 20 mL) gewaschen und in Dichlormethan/Methanol (9:1, 600 mL) gelöst. Die organische Phase wird mit Natriumbicarbonat-Lösung (2 × 100 mL) gewaschen, mit Natriumsulfat getrocknet und eingeengt. Der Rückstand wird mit Methanol verrührt, der gebildete Niederschlag abfiltriert und getrocknet. Ausbeute: 8.3 g (64%); Massenspektroskopie: [M+H]⁺ = 294.

### e) 7-Benzyloxy-5-(2-chloracetyl)-1H-chinolin-2-on

5-Acetyl-7-benzyloxy-1H-chinolin-2-on (7.0 g, 23.86 mmol) wird in einem Gemisch aus 1,2-Dichloroethan (147 mL), Eisessig (43 mL) und Wasser (7 mL) gelöst und mit N-Benzyl-trimethylammonium-dichloriodat (19.0 g, 54.58 mmol) versetzt. Das Gemisch wird 4.5 Stunden bei 65 °C gerührt, anschließend mit Natriumbicarbonat-Lösung und 5%iger. Natriumbisulfit-Lösung verdünnt und 5 Minuten gerührt. Der gebildete Niederschlag wird abfiltriert, mit Wasser (2 × 20 mL) gewaschen und im Ofen getrocknet. Ausbeute: 6.0 g (77%); Massenspektroskopie: [M+H]⁺ = 328.

### f) 7-Benzyloxy-5-oxiranyl-1H-chinolin-2-on

Zu einer Suspension von 7-Benzyloxy-5-(2-chloroacetyl)-1H-chinolin-2-on (6 g, 18.31 mmol) in THF (150 mL) wird bei 0-5 °C Lithiumborhydrid (434 mg, 19.93 mmol) zugegeben und es wird 30 Minuten gerührt. 2.5 N Natriumhydroxid-Lösung (43 mL, 107.50 mmol) wird zugegeben und man rührt 2 Stunden bei 5-10 °C und 2.5 Stunden bei Raumtemperatur. Anschließend wird das Reaktionsgemisch langsam mit Eisessig (6.5 mL) gefolgt von halbgesättigter Natriumchlorid-Lösung (100 mL) versetzt und weitere 5 Minuten gerührt. Der gebildete Niederschlag wird abfiltriert und die wässrige Phase mit Ethylacetat/THF (1/1, 5 x 100 mL) extrahiert. Der abfiltrierte Feststoff und die organischen Phasen werden vereinigt, mit Natriumsulfat getrocknet und eingeengt. Das Rohprodukt wird mit Methanol (30 mL) verrührt und der Niederschlag abfiltriert und bei Raumtemperatur getrocknet. Ausbeute: 4.8 g (89%); Massenspektroskopie: [M+H]⁺ = 294.

### g)1-{3-[2-(7-Benzyloxy-2-oxo-1,2-dihydro-quinolin-5-yl)-2-hydroxy-ethylamino]-3-methyl-butyl}-4,4-diethyl-1,4-dihydro-benzo[d][1,3]oxazin-2-on

Eine Suspension von 7-Benzyloxy-5-oxiranyl-1H-chinolin-2-on (112 mg, 0.382 mmol) und 1-(3-Amino-3-methyl-butyl)-4,4-diethyl-1,4-dihydro-benzo[d][1,3]oxazin-2-on (220 mg, 0.758 mmol) in Isopropanol (1.0 mL) wird in der Mikrowelle 1 h auf 135 °C erhitzt. Das Gemisch wird mit EtOAc (10 mL) verdünnt und mit 0.5 M wässr. Weinsäure-Lösung gewaschen, wobei ein Teil des Produkts ausfällt. Die Phasen werden getrennt und die wässr. Suspension wird mit soviel MeOH versetzt, bis wieder eine klare Lösung vorliegt. Die wässr. Phase wird mit Dichlormethan extrahiert und die vereinigten org. Phasen werden über Natriumsulfat getrocknet und i. vac. eingeengt. Der Rückstand wird mit EtOAc verrührt und der Niederschlag abfiltriert und i. vac. getrocknet. Ausbeute: 152 mg (68%); HPLC-MS: Rₜ = 14.8 Min. (Methode A).

### h) 4,4-Diethyl-1-{3-[2-hydroxy-2-(7-hydroxy-2-oxo-1,2-dihydro-quinolin-5-yl)-ethylamino]-3-methyl-butyl}-1,4-dihydro-benzo[d][1,3]oxazin-2-on

Eine Suspension von 1-{3-[2-(7-Benzyloxy-2-oxo-1,2-dihydro-quinolin-5-yl)-2-hydroxyethylamino]-3-methyl-butyl}-4,4-diethyl-1,4-dihydro-benzo[*d*][1,3]oxazin-2-on (152 mg, 0.338 mmol) und Pd/C (10%) (40 mg) in MeOH (12 mL) wird bei RT und 1 bar Wasserstoffdruck 4 h hydriert. Der Katalysator wird über Celite abfiltriert und mit MeOH (5 mL) gewaschen. Die org. Phase wird eingeengt, der Rückstand mit EtOAc verrieben und der gebildete Niederschlag abfiltriert und i. vac. getrocknet. Ausbeute: 76 mg (46%); R_{f} = 0.3 (Kieselgel, Dichlormethan / MeOH / ges. wässr. Ammoniak 90:10:0.5); ESI-MS:
[M+H]⁺ = 494.

Für die nachfolgenden Synthesebeispiele sind spezifische Ausgangsverbindungen erforderlich, deren Darstellung nachfolgend beschrieben wird.

### Zwischenprodukt 1: 1-(3-Amino-3-methyl-butyl)-4,4-dipropyl-1,4-dihydrobenzo[d][1,3]oxazin-2-on hydrochlorid

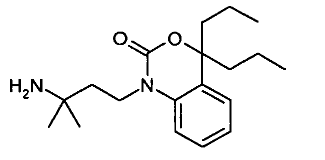

### a) 4-(2-Amino-phenyl)-heptan-4-ol

Zu einer Lösung von 7.00 mL (54.04 mmol) Anthranilsäuremethylester in abs. THF (70 mL) werden bei 0°C innerhalb von 30 Min. 90.0 mL (180.00 mmol) Propylmagnesiumchlorid (2 M in Ether) zugetropft. Das Gemisch wird 1 h bei RT gerührt und dann mit 100 mL 3 M wässr. Ammoniumchlorid-Lösung und EtOAc versetzt. Die Phasen werden getrennt und die wässr. Phase wird erschöpfend mit EtOAc extrahiert. Die vereinigten org. Phasen werden mit wässr. KHCO₃ und ges. wässr. NaCl gewaschen und mitr Natriumsulfat getrocknet. Das Rohprodukt wird ohne weitere Reinigung in dem nachfolgenden Reaktionsschritt eingesetzt. Ausbeute: 6.70 g (60%).

### b) {3-[2-(1-Hydroxy-1-propyl-butyl)-phenylamino]-1,1-dimethyl-propyl}-carbaminsäure-tert-butylester

Zu einer Lösung von 3.10 g (14.05 mmol) 4-(2-Amino-phenyl)-heptan-4-ol und 3.60 g (17.88 mmol) (1,1-Dimethyl-3-oxo-propyl)-carbaminsäure-tert-butylester in MeOH (40 mL) und AcOH (6 mL) werden 1.40 g (22.27 mmol) Natriumcyanoborhydrid zugegeben. Das Gemisch wird 16 h bei RT gerührt, mit EtOAc verdünnt und mit 0.5 M wässr. KHSO₃ und ges. wässr. NaCl gewaschen, mit Natriumsulfat getrocknet und i. vac. eingeengt. Das Rohprodukt wird ohne weitere Reinigung in dem nachfolgenden Reaktionsschritt eingesetzt. Ausbeute: 6.00 g (quantitative Ausbeute).

### c) [1,1-Dimethyl-3-(2-oxo-4,4-dipropyl-4H-benzo[d][1,3]oxazin-1-yl)-propyl]-carbaminsäure-tert-butylester

Zu einer Lösung von 6.00 g (15.28 mmol) {3-[2-(1-Hydroxy-1-propyl-butyl)-phenylamino]-1,1-dimethyl-propyl}-carbaminsäure-tert-butylesterund 5.32 mL (38.21 mmol) Triethylamin in abs. THF (80 mL) werden bei 0 °C 8.85 mL (16.81 mmol) Phosgen-Lösung (20 gew.% in Toluol) langsam zugetropft. Das Gemisch wird 2 h bei RT gerührt, mit EtOAc verdünnt, mit Eis versetzt und mit ges. wässr. Ammoniak-Lösung basisch gestellt. Die wässr. Phase wird mit EtOAc erschöpfend extrahiert und die vereinigten org. Phasen werden mit ges. wässr. NaCl gewaschen, mit Natriumsulfat getrocknet und i. vac. eingeengt. Nach Säulenchromatographie (Kieselgel, Cyclohexan/EtOAc 6:1) wird das Produkt als gelbes Öl erhalten. Ausbeute: 4.57 g (71%).

### d) 1-(3-Amino-3-methyl-butyl)-4,4-dipropyl-1,4-dihydro-benzo[d][1,3]oxazin-2-on hydrochlorid

Eine Lösung von 4.20 g (10.03 mmol) [1,1-Dimethyl-3-(2-oxo-4,4-dipropyl-4*H-*benzo[*d*][1,3]oxazin-1-yl)-propyl]-carbaminsäure-tert-butylester in 35 mL Ameisensäure wird 24 h bei RT gerührt und anschließend auf Eis gegossen. Die wässr. Phase wird mit ges. wässr. Ammoniak-Lösung basisch gestellt und mit EtOAc erschöpfend extrahiert. Die vereinigten org. Extrakte werden mit ges. wässr. NaCl gewaschen, über Natriumsulfat getrocknet und i. vac. eingeengt. Der Rückstand wird in EtOAc (50 mL) aufgenommen und mit 4 mL HCl-Lösung (ges. in EtOAc) versetzt. Die Lösung wird eingedampft und zweimal mit wenig EtOH versetzt und i. vac. eingeengt. Verreiben des Rückstands mit Diisopropylether ergibt das Produkt als hygroskopisches Hydrochloridsalz. Ausbeute: 2.60 g (73%).

### Zwischenprodukt 2: 1-(3-Amino-3-methyl-butyl)-4,4-diethyl-7-fluor-1,4-dihydrobenzo[d][1,3]oxazin-2-on

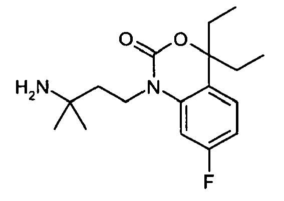

### a) 3-(2-Amino-4-fluor-phenyl)-pentan-3-ol

Das Produkt wird analog Zwischenprodukt 1a durch Reaktion von 2-Amino-4-fluorbenzoesäuremethylester und Ethylmagnesiumbromid in Dichlormethan bei - 78 °C → RT erhalten. Ausbeute: 4.1 g (99%).

### b) {3-[2-(1-Ethyl-1-hydroxy-propyl)-5-fluor-phenylamino]-1,1-dimethyl-propyl}-carbaminsäure-tert-butylester

Das Produkt wird analog Zwischenprodukt 1b ausgehend von 3-(2-Amino-4-fluor-phenyl)-pentan-3-ol und (1,1-Dimethyl-3-oxo-propyl)-carbaminsäure-tert-butylester erhalten. Das Rohprodukt wird mittels Säulenchromatographie (Kieselgel, Dichlormethan/MeOH 100:0 → 98:2) gereinigt. Ausbeute: 7.70 g (99%).

### c) [3-(4,4-Diethyl-7-fluor-2-oxo-4H-benzo[d][1,3]oxazin-1-yl)-1,1-dimethyl-propyl]-carbaminsäure-tert-butylester

Das Produkt wird analog Zwischenprodukt 1c ausgehend von {3-[2-(1-Ethyl-1-hydroxypropyl)-5-fluor-phenylamino]-1,1-dimethyl-propyl}-carbaminsäure-tert-butylester erhalten. Ausbeute: 4.20 g (51%).

### d) 1-(3-Amino-3-methyl-butyl)-4,4-diethyl-7-fluor-1,4-dihydro-benzo[d][1,3]oxazin-2-on

Das Produkt wird analog Zwischenprodukt 1d ausgehend von [3-(4,4-Diethyl-7-fluor-2-oxo-4*H*-benzo[*d*][1,3]oxazin-1-yl)-1,1-dimethyl-propyl]-carbaminsäure-tert-butylester als freie Base hergestellt. Ausbeute: 2.90 g (96%); ESI-MS: [M+H]⁺ = 309.

### Zwischenprodukt 3: 1-(3-Amino-3-methyl-butyl)-4,4-diethyl-8-methoxy-1,4-dihydrobenzo[d][1,3]oxazin-2-on

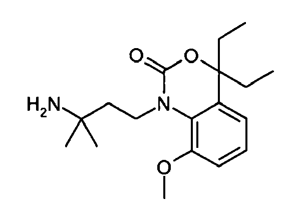

### a) 3-(2-Amino-3-methoxy-phenyl)-pentan-3-ol

Das Produkt wird analog Zwischenprodukt 1a durch Reaktion von 2-Amino-3-methoxybenzoesäuremethylester und Ethylmagnesiumbromid in Dichlormethan bei - 78 °C → RT erhalten. Ausbeute: 5.20 g (92%); HPLC-MS: Rₜ = 12.85 Min. (Methode A); ESI-MS:
[M+H]⁺ = 210.

### b) {3-[2-(1-Ethyl-1-hydrox-propyl)-6-methoxy-phenylamino]-1,1-dimethyl-propyl}-carbaminsäure-tert-butylester

Das Produkt wird analog Zwischenprodukt 1b ausgehend von 3-(2-Amino-3-methoxyphenyl)-pentan-3-ol und (1,1-Dimethyl-3-oxo-propyl)-carbaminsäure-tert-butylester erhalten. Das Rohprodukt wird mittels Säulenchromatographie (Kieselgel, Cyclohexan/EtOAc 4:1) gereinigt. Ausbeute: 4.60 g (47%).

### c) [3-(4,4-Diethyl-8-methoxy-2-oxo-4H-benzo[d][1,3]oxazin-1-yl)-1,1-dimethyl-propyl]-carbaminsäure-tert-butylester

Das Produkt wird analog Zwischenprodukt 1c ausgehend von {3-[2-(1-Ethyl-1-hydroxypropyl)-6-methoxy-phenylamino]-1,1-dimethyl-propyl}-carbaminsäure-tert-butylester erhalten. Ausbeute: 4.60 g (94%).

### d) 1-(3-Amino-3-methyl-butyl)-4,4-diethyl-8-methoxy-1,4-dihydro-benzo[d][1,3]oxazin-2-on

Das Produkt wird analog Zwischenprodukt 1d ausgehend von [3-(4,4-Diethyl-8-methoxy-2-oxo-4*H*-benzo[*d*][1,3]oxazin-1-yl)-1,1-dimethyl-propyl]-carbaminsäure-tert-butylester als freie Base hergestellt. Ausbeute: 3.00 g (93%); ESI-MS: [M+H]⁺ = 321.

### Zwischenprodukt 4: 1-(3-Amino-3-methyl-butyl)-spiro(cyclohexan-1,4'-2H-3',1'-benzoxazin)-2'-on

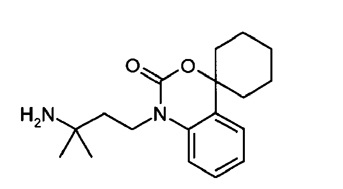

### a) 1-(2-Nitro-phenyl)-cyclohexanol

Zu einer Lösung von 20.0 g (80.32 mmol) 2-Nitro-iodbenzol in abs. THF (150 mL) werden bei -50 °C unter Stickstoffatmosphäre 40.16 mL (80.32 mmol) Phenylmagnesiumchlorid (2 M in THF) zugetropft. Nach 15 Min. Rühren werden 9.98 mL (96.30 mmol) Cyclohexanon schnell zugegeben. Das Gemisch wird auf RT erwärmt und weitere 2 h gerührt. Ges. wässr. Ammoniumchlorid-Lösung wird zugegeben und die wässr. Phase wird mit EtOAc erschöpfend extrahiert. Die vereinigten org. Extrakte werden mit ges. wässr. NaCl-Lösung gewaschen, mit Natriumsulfat getrocknet und i. vac. eingeengt. Nach Säulenchromatographie (Kieselgel, Hexan/EtOAc 20:1) wird das Produkt als bräunliches Öl erhalten. Ausbeute: 5.20 g (29%); R_{f} = 0.26 (Kieselgel, Hexan/EtOAc 10:1); ESI-MS:
[M+H-H₂O]⁺ = 204.

### b) 1-(2-Amino-phenyl)-cyclohexanol

Eine Suspension von 5.20 g (16.45 mmol) 1-(2-Nitro-phenyl)-cyclohexanol und 500 mg Raney-Nickel in EtOH (70 mL) wird bei RT und 3 bar Wasserstoffdruck 4 h hydriert. Der Katalysator wird über Celite abfiltriert und das Filtrat i. vac. eingeengt. Der Rückstand wird aus Hexan umkristallisiert. Ausbeute: 1.53 g (49%); R_{f} = 0.38 (Kieselgel, Hexan/EtOAc 4:1); ESI-MS: [M+H-H₂O]⁺ = 174.

### c) {3-[2-(1-Hydroxy-cyclohexyl-phenylamino]-1,1-dimethyl-propyl}-carbaminsäure-tert-butylester

Das Produkt wird analog Zwischenprodukt 1b ausgehend von 1-(2-Amino-phenyl)-cyclohexanol und (1,1-Dimethyl-3-oxo-propyl)-carbaminsäure-tert-butylester hergestellt. Nach Säulenchromatographie (Kieselgel, Hexan/EtOAc 7:1) wird das Produkt als farbloses Öl erhalten. Ausbeute: 2.65 g (66%); R_{f} = 0.50 (Kieselgel, Hexan/EtOAc 4:1).

### d) [3-Spiro(cyclohexan-1,4'-2H-3',1'-benzoxazin)-2'-oxo-1-yl)-1,1-dimethyl-propyl]-carbaminsäure-tert-butylester

Das Produkt wird analog Zwischenprodukt 1c ausgehend von {3-[2-(1-Hydroxycyclohexyl)-phenylamino]-1,1-dimethyl-propyl}-carbaminsäure-tert-butylester hergestellt. Ausbeute: 2.60 g (92%); R_{f} = 0.38 (Kieselgel, Hexan/EtOAc 4:1).

### e) 1-(3-Amino-3-methyl-butyl)-spiro(cyclohexan-1,4'-2H-3',1'-benzoxazin)-2'-on

Das Produkt wird analog Zwischenprodukt 1d ausgehend von [3-(Spiro(cyclohexan-1,4'-2H-3',1'-benzoxazin)-2'-oxo-1-yl)-1,1-dimethyl-propyl]-carbaminsäure-tert-butylester hergestellt. Ausbeute: 1.80 g (92%); R_{f} = 0.10 (Kieselgel, Dichlormethan/MeOH/ges. wässr. Ammoniak 95:5:0.5); ESI-MS: [M+H]⁺ = 303.

### Beispiel 12: 1-{3-[2-Hydroxy-2-(6-hydroxy-3-oxo-3,4-dihydro-2H-benzo[1,4]oxazin-8-yl)-ethylamino]-3-methyl-butyl}-4,4-dipropyl-1,4-dihydro-benzo[d][1,3]oxazin-2-on

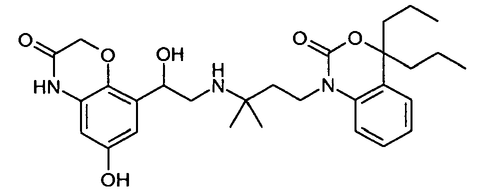

### a) 1-{3-[2-(6-Benzyloxy-3-oxo-3,4-dihydro-2H-benzo[1,4]oxazin-8-yl)-2-hydroxyethylamino]-3-methyl-butyl}-4,4-dipropyl-1,4-dihydro-benzo[d][1,3]oxazin-2-on

Zu einer Lösung von 200 mg (0.564 mmol) 1-(3-Amino-3-methyl-butyl)-4,4-dipropyl-1,4-dihydro-benzo[*d*][1,3]oxazin-2-on hydrochlorid in abs. THF (5 mL) werden bei RT unter einer Stickstoffatmosphäre 86 µL (0.619 mmol) Triethylamin zugegeben und es wird 30 Min. gerührt. 200 mg (0.560 mmol) 6-Benzyloxy-8-(2-ethoxy-2-hydroxy-acetyl)-4*H-*benzo[1,4]oxazin-3-on werden zugegeben und es wird weitere 2 h bei RT gerührt. Das Gemisch wird auf 10 °C gekühlt, mit 51 mg (2.34 mmol) Lithiumborhydrid versetzt, auf RT erwärmt und eine 1 h bei RT gerührt. Es wird erneut auf 10 °C gekühlt und langsam mit 15 mL Wasser und 20 mL Dichlormethan versetzt. Die Phasen werden getrennt und man extrahiert die wässr. Phase mit Dichlormethan. Die vereinigten org. Phasen werden mit Natriumsulfat getrocknet und i. vac. eingeengt. Der Rückstand wird in EtOAc (8 mL) gelöst und durch Zugabe von HCl-Lösung (ges. in EtOAc) auf pH 2 angesäuert. Der gebildete Niederschlag wird abfiltriert, mit EtOAc gewaschen und i. vac. getrocknet. Ausbeute: 270 mg (74%; Hydrochlorid), HPLC-MS: Rₜ = 18.7 Min. (Methode A).

### b) 1-{3-[2-Hydroxy-2-(6-hydroxy-3-oxo-3,4-dihydro-2H-benzo[1,4]oxazin-8-yl)-ethylamino]-3-methyl-butyl}-4,4-dipropyl-1,4-dihydro-benzo[d][1,3]oxazin-2-on

Eine Suspension von 270 mg (0.438 mmol) 1-{3-[2-(6-Benzyloxy-3-oxo-3,4-dihydro-2H-benzo[1,4]oxazin-8-yl)-2-hydroxy-ethylamino]-3-methyl-butyl}-4,4-dipropyl-1,4-dihydrobenzo[d][1,3]oxazin-2-on und 27 mg Pd/C (10%) in MeOH (8 mL) wird bei RT und 1 bar Wasserstoffdruck 3 h hydriert. Der Katalysator wird über Celite abfiltriert und mit MeOH (5 mL) gewaschen und das Filtrat i. vac. eingedampft. Der Rückstand wird in EtOAc/Dichlormethan (1:1, 10 mL) gelöst, durch Zugabe von HCl-Lösung (ges. in EtOAc) auf pH 2 angesäuert und i. vac. eingedampft. Der Rückstand wird mit Ether verrieben, filtriert und i. vac. getrocknet. Ausbeute: 80 mg (33%; Hydrochlorid), HPLC-MS: Rₜ = 12.8 Min. (Methode A), ESI-MS: [M+H]⁺ = 526.

### Beispiel 13: 1-{3-[2-Hydroxy-2-(5-hydroxy-3-oxo-3,4-dihydro-2H-benzo[1,4]oxazin-8-yl)-ethylamino]-3-methyl}-butyl-4,4-dipropyl-1,4-dihydro-benzo[d][1,3]oxazin-2-on

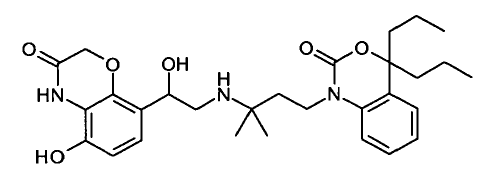

### a) 1-{3-[2-(5-Benzyloxy-3-oxo-3,4-dihydro-2H-benzo[1,4]oxazin-8-yl)-2-hydroxyethylamino]-3-methyl-butyl}-4,4-dipropyl-1,4-dihydro-benzo[d][1,3]oxazin-2-on

Das Produkt wird analog Beispiel 12a ausgehend von 5-Benzyloxy-8-(2,2-dihydroxyacetyl)-4*H*-benzo[1,4]oxazin-3-on und 1-(3-Amino-3-methyl-butyl)-4,4-dipropyl-1,4-dihydro-benzo[*d*][1,3]oxazin-2-on hydrochlorid hergestellt. Das Rohprodukt wird in EtOAc gelöst, mit 5%-iger wässr. NaOH-Lösung gewaschen und mittels Säulenchromatographie (Kieselgel, Dichlormethan/MeOH 98:2 → 90:10) gereinigt. Ausbeute: 170 mg (49%); HPLC-MS: Rₜ = 18.9 Min. (Methode A).

### b) 1-{3-[2-Hydroxy-2-(5-hydroxy-3-oxo-3,4-dihydro-2H-benzo[1,4]oxazin-8-yl)-ethylamino]-3-methyl-butyl}-4,4-dipropyl-1,4-dihydro-benzo[d][1,3]oxazin-2-on

Das Produkt wird analog Beispiel 12b ausgehend von 1-{3-[2-(5-Benzyloxy-3-oxo-3,4-dihydro-2H-benzo[1,4]oxazin-8-yl)-2-hydroxy-ethylamino]-3-methyl-butyl}-4,4-dipropyl-1,4-dihydro-benzo[d][1,3]oxazin-2-on hergestellt. Ausbeute: 30 mg (19%, Hydrochlorid); HPLC-MS: Rₜ = 13.0 Min. (Methode A); ESI-MS: [M+H]⁺ = 526.

### Beispiel 14: 4,4-Diethyl-7-fluor-1-{3-[2-hydroxy-2-(6-hydroxy-3-oxo-3,4-dihydro-2H-benzo [1,4]oxazin-8-yl)-ethylamino]-3-methyl-butyl}-1,4-dihydro-benzo[d][1,3]oxazin-2-on

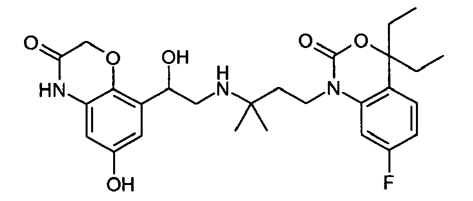

### a) 1-{3-[2-(6-Benzyloxy-3-oxo-3,4-dihydro-2H-benzo[1,4]oxazin-8-yl)-2-hydroxyethylamino]-3-methyl-butyl}-4,4-diethyl-7-fluor-1,4-dihydro-benzo[d][1,3]oxazin-2-on

Eine Lösung von 232 mg (0.649 mmol) 6-Benzyloxy-8-(2-ethoxy-2-hydroxy-acetyl)-4*H-*benzo[1,4]oxazin-3-on und 200 mg (0.649 mmol) 1-(3-Amino-3-methyl-butyl)-4,4-diethyl-7-fluor-1,4-dihydro-benzo[d][1,3]oxazin-2-on in abs. THF (5 mL) wird 2.5 h bei RT gerührt. Das Gemisch wird auf 5 °C gekühlt, mit 60 mg (2.755 mmol) Lithiumborhydrid versetzt, auf RT erwärmt und 1 h gerührt. Es wird erneut auf 5 °C gekühlt und langsam mit 15 ml Wasser und 20 mL Dichlormethan verdünnt. Die Phasen werden getrennt und die wässr. Phase wird mit Dichlormethan extrahiert. Die vereinigten org. Phasen werden mit Natriumsulfat getrocknet und i. vac. eingedampft. Der Rückstand wird mittels Säulenchromatographie (Kieselgel, Dichlormethan/MeOH 95:5) gereinigt. Ausbeute: 257 mg (65%); HPLC-MS: Rₜ = 16.5 Min. (Methode A).

### b) 4,4-Diethyl-7-fluor-1-{3-[2-hydroy-2-(6-hydroxy-3-oxo-3,4-dihydro-2H-benzo[1,4]oxazin-8-yl)-ethylamino]-3-methyl}-1,4-dihydro-benzo[d][1,3]oxazin-2-on

Das Produkt wird analog Beispiel 12b ausgehend von 1-{3-[2-(6-Benzyloxy-3-oxo-3,4-dihydro-2*H*-benzo[1,4]oxazin-8-yl)-2-hydroxy-ethylamino]-3-methyl-butyl}-4,4-diethyl-7-fluor-1,4-dihydro-benzo[*d*][1,3]oxazin-2-on hergestellt. Ausbeute: 170 mg (78%; Hydrochlorid); HPLC-MS: Rₜ = 10.6 Min. (Methode A); ESI-MS: [M+H]⁺ = 516.

### Beispiel 15: 4,4-Diethyl-1-{3-[2-hydroxy-2-(5-hydroxy-3-oxo-3,4-dihydro-2H-benzo[1,4]oxazin-8-yl)-ethylamino]-3-methyl-butyl}-8-methoxy-1,4-dihydrobenzo[d][1,3]oxazin-2-on

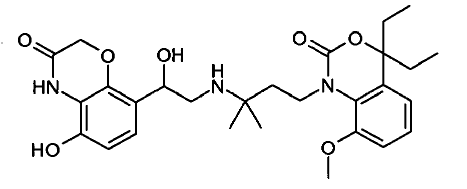

### a) 1-{3-[2-(5-Benzyloxy-3-oxo-3,4-dihydro-2H-benzo[1,4]oxazin-8-yl)-2-hydroxyethylaminol-3-methyl-butyl}-4,4-diethyl-8-methoxy-1,4-dihydro-benzo[d][1,3]oxazin-2-on

Das Produkt wird analog Beispiel 14a ausgehend von 5-Benzyloxy-8-(2,2-dihydroxyacetyl)-4*H*-benzo[1,4]oxazin-3-on und 1-(3-Amino-3-methyl-butyl)-4,4-diethyl-8-methoxy-1,4-dihydro-benzo[d][1,3]oxazin-2-on hergestellt. Das Rohprodukt wird mittels Säulenchromatographie (Kieselgel, Dichlormethan/MeOH 95:5) gereinigt. Ausbeute: 70 mg (18%); HPLC-MS: Rₜ = 16.5 Min. (Methode A).

### b) 4,4-Diethyl-1-{3-[2-hydroxy-2-(5-hydroxy-3-oxo-3,4-dihydro-2H-benzo[1,4]oxazin-8-yl)-ethylamino1-3-methyl-butyl}-8-methoxy-1,4-dihydro-benzo[d][1,3]oxazin-2-on

Das Produkt wird analog Beispiel 12b ausgehend von 1-{3-[2-(5-Benzyloxy-3-oxo-3,4-dihydro-2*H*-benzo[1,4]oxazin-8-yl)-2-hydroxy-ethylamino]-3-methyl-butyl}-4,4-diethyl-8-methoxy-1,4-dihydro-benzo[*d*][1,3]oxazin-2-on erhalten. Ausbeute: 40 mg (62%); HPLC-MS: Rₜ = 13.3 Min. (Methode A); ESI-MS: [M+H]⁺ = 528.

### Beispiel 16:1-(2-{1-[2-Hydroxy-2-(5-hydroxy-3-oxo-3,4-dihydro-2H-benzo[1,4]oxazin-8-vl)-ethylamino]-cyclopropyl}-ethyl)-4,4-dimethyl-1,4-dihydro-benzo[d][1,3]oxazin-2-on

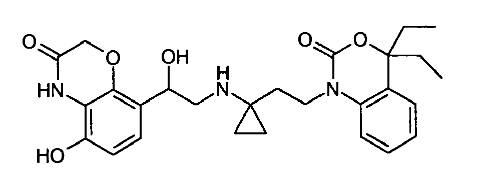

### a) 3-(4,4-Dimethyl-2-oxo-4H-benzo[d][1,3]oxazin-1-yl]-propionitril

Zu einer Lösung von 20.0 g (112 mmol) 4,4-Dimethyl-1,4-dihydro-benzo[d][1,3]oxazin-2-on und 17.4 g (126 mmol) Kaliumcarbonat in 250 mL Acetonitril werden 10.2 mL (123 mmol) Brompropionitril getropft und es wird über Nacht unter Rückfluß gerührt. Weitere 4 mL (48 mmol) Brompropionitril werden zugesetzt und es wird noch 2 Stunden unter Rückfluß gerührt. Der Feststoff wird abgesaugt, das Filtrat eingeengt und der Rückstand in Diisopropylether umkristallisiert. Weißer Feststoff. Ausbeute: 22.8 g (88%); Massenspektroskopie: [M+H]⁺ = 231.

### b) 1-[2-(1-Amino-cyclopropyl)-ethyl]-4,4-dimethyl-1,4-dihydro-benzo[d][1,3]oxazin-2-on

Eine Suspension von 6.0 g (26 mmol) 3-(4,4-Dimethyl-2-oxo-4H-benzo[d][1,3]oxazin-1-yl]-propionitril in 120 mL Diethylether wird unter Kühlung mit einem Eisbad mit 16.5 mL (56 mmol) Titan-tetra-isopropylat versetzt. Anschließend werden 18.5 mL einer 3 molaren Lösung von Ethylmagnesiumbromid in Diethylether so zugetropft, dass die Temperatur nicht über 20°C steigt. Man läßt 30 Minuten bei Raumtemperatur Rühren und gibt 7.0 mL (55 mmol) Bortrifluorid-Diethylether portionsweise unter Kühlung mit einem Eisbad zu. Es wird eine Stunde bei Raumtemperatur gerührt und unter Kühlung werden 150 mL 1 molare Natriumhydroxid-Lösung zugetropft. Das Reaktionsgemisch wird mit Diethylether verdünnt und die Phasen werden getrennt. Die wässrige Phase wird mit Diethylether extrahiert und die vereinigten organischen Phasen werden mit Natriumsulfit-Lösung und wiederholt mit 1 molarer Salzsäure ausgeschüttlet. Die Salzsäure-Phasen werden vereinigt, mit Diethylether ausgeschüttelt, mit Natriumhydroxid-Lösung alkalisch gestellt und erschöpfend mit Dichlormethan extrahiert. Die Dichlormethan-Phasen werden mit Natriumsulfat getrocknet und eingeengt. Leicht gelbes Öl. Ausbeute: 1.5 g (22%); Massenspektroskopie: [M+H]⁺ = 261.

### c) 1-(2-{1-[2-(5-Benzyloxy-3-oxo-3,4-dihydro-2H-benzo[1,4]oxazin-8-yl)-2-hydroxyethylamino]-cyclopropyl}-ethyl)-4,4-dimethyl-1,4-dihydro-benzo[d][1,3]oxazin-2-on

900 mg (2.5 mmol) 5-Benzyloxy-8-(2-ethoxy-2-hydroxy-acetyl)-4*H*-benzo[1,4]oxazin-3-on und 700 mg (2.7 mmol) 1-[2-(1-Amino-cyclopropyl)-ethyl]-4,4-dimethyl-1,4-dihydrobenzo[d][1,3]oxazin-2-on werden in 20 mL Ethanol gelöst und jeweils 30 Minuten bei 80°C und 50°C gerührt. Die Reaktionsmischung wird abgekühlt, mit 200 mg (5.3 mmol) Natriumborhydrid versetzt und 2 Stunden bei Raumtemperatur gerührt. Es wird Eisessig zugegeben, 10 Minuten gerührt und eingeengt. Der Rückstand wird in Dichlormethan aufgenommen und nacheinander mit Kaliumhydrogensulfat-Lösung, 15%iger Kaliumcarbonat-Lösung und Natriumhydrogencarbonat-Lösung gewaschen. Anschließend wird die organische Phase mit Natriumsulfat getrocknet und vom Lösungsmittel befreit. Der Rückstand wird säulenchromatographisch gereinigt (Kieselgel; Ethylacetat/Methanol/Ammoniak-Gradient). Umkristallisation aus Diisopropylether. Weißer Feststoff. Ausbeute: 690 mg (49%); Massenspektroskopie: [M+H]⁺ = 558.

### d) 1-(2-{1-[2-Hydroxy-2-(5-hydroxy-3-oxo-3,4-dihydro-2H-benzo[1,4]oxazin-8-yl)-ethylamino]-cyclopropyl}-ethyl)-4,4-dimethyl-1,4-dihydro-benzo[d][1,3]oxazin-2-on

650 mg (1.17 mmol) 1-(2-{1-[2-(5-Benzyloxy-3-oxo-3,4-dihydro-2H-benzo[1,4]oxazin-8-yl)-2-hydroxy-ethylamino]-cyclopropyl}-ethyl)-4,4-dimethyl-1,4-dihydrobenzo[d][1,3]oxazin-2-on werden in 30 mL Methanol gelöst, mit Palladium auf Kohle (10%ig) versetzt und bei Raumtemperatur und 3 bar Wasserstoffdruck hydriert. Ausbeute: 240 mg (44%); Massenspektroskopie: [M+H]⁺ = 468.

### Beispiel 17: 1-(2-{1-[2-Hydroxy-2-(6-hydroxy-3-oxo-3,4-dihydro-2H-benzo[1,4]oxazin-8-yl)-ethylamino]-cyclopropyl}-ethyl)-4,4-dimethyl-1,4-dihydro-benzo[d][1,3]oxazin-2-on

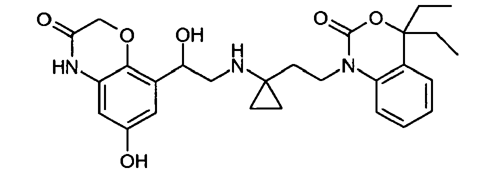

### a) 1-(2-{1-[2-(6-Benzyloxy-3-oxo-3,4-dihydro-2H-benzo[1,4]oxazin-8-yl)-2-hydroxyethylamino]-cyclopropyl}-ethyl)-4,4-dimethyl-1,4-dihydro-benzo[d][1,3]oxazin-2-on

Herstellung aus 900 mg (2.5 mmol) 6-Benzyloxy-8-(2-ethoxy-2-hydroxy-acetyl)-4H-benzo[1,4]oxazin-3-on und 700 mg (2.7 mmol) 1-[2-(1-Amino-cyclopropyl)-ethyl]-4,4-dimethyl-1,4-dihydro-benzo[d][1,3]oxazin-2-on in Analogie zu dem für Beispiel 16a beschriebenen Verfahren. Weißer Feststoff. Ausbeute: 630 mg (45%); Massenspektroskopie: [M+H]⁺ = 558.

### b) 1-(2-{1-[2-Hydroxy-2-(6-hydroxy-3-oxo-3,4-dihydro-2H-benzo[1,4]oxazin-8-yl)-ethylamino]-cyclopropyl}-ethyl)-4,4-dimethyl-1,4-dihydro-benzo[d][1,3]oxazin-2-o

590 mg (1.06 mmol) 1-(2-{1-[2-(6-Benzyloxy-3-oxo-3,4-dihydro-2H-benzo[1,4]oxazin-8-yl)-2-hydroxy-ethylamino]-cyclopropyl}-ethyl)-4,4-dimethyl-1,4-dihydrobenzo[d][1,3]oxazin-2-on werden in 30 mL Methanol gelöst und in Gegenwart von Palladium auf Kohle (10%ig) bei Raumtemperatur und 3 bar Wasserstoffdruck hydriert. Ausbeute: 180 mg (36%); Massenspektroskopie: [M+H]⁺ = 468.

Die nachfolgend aufgeführten Beispiele werden in Analogie zu den vorstehend beschriebenen Verfahren erhalten.

### Beispiel 18: 1-{3-[2-Hydroxy-2-(6-hydroxy-3-oxo-3,4-dihydro-2H-benzo[1,4]oxazin-8-yl)-ethylamino]-3-methyl-buty}-spiro(cyclohexan-1,4'-2H-3',1'-benzoxazin)-2'-on

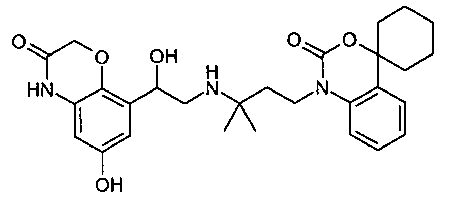

### Beispiel 19: 1-{3-[2-Hydroxy-2-(5-hydroxy-3-oxo-3,4-dihydro-2H-benzo[1,4]oxazin-8-yl)-ethylamino]-3-methyl-butyl}-spiro(cyclohexan-1,4'-2H-3',1'-benzoxazin)-2'-on

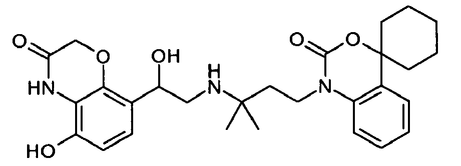

### Beispiel 20: 4,4-Dimethyl-1-{3-{2-hydroxy-2-(6-hydroxy-3-oxo-3,4-dihydro-2H-benzo[1,4]oxazin-8-yl)-ethylamino]propyl}-1,4-dihydro-benzo[1,3]oxazin-2-on

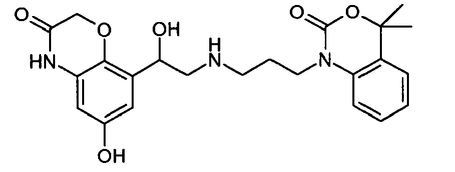

### Beispiel 21: 4,4-Dimethyl-1-{3-[2-hydroxy-2-(5-hydroxy-3-oxo-3,4-dihydro-2H-benzo[1,4]oxazin-8-yl)-ethylamino]propyl}-1,4-dihydro-benzo[d][1,3]oxazin-2-on

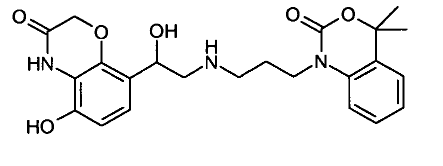

aus den vorstehend beispielhaft genannten Verbindungen sind die entsprechenden enantiomerenreinen Verbindungen, so auch die Verbindungen der Formel **1**, in denen das zum Phenylring benzylische, asymmetrische Kohlenstoffzentrum "-CH(OH)-" R-konfiguriert ist, nach im Stand der Technik bekannten Methoden erhältlich.

## Patentansprüche

1. Arzneimittelkombinationen, die neben einer oder mehrerer Verbindungen der allgemeinen Formel **1** 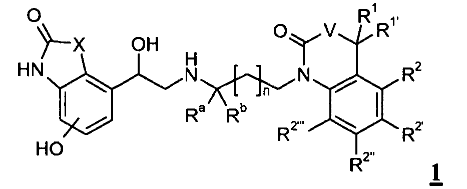 worin
X ein Gruppe -O-, -NH-, -CH₂-O-, -CHMe-O-, -C(Me)₂-O-, -CH₂-NH-, -CHMe-NH-, -C(Me)₂-NH-, -CH=CH- oder -CH₂-CH₂-;
V eine zweibindige Gruppe ausgewählt aus der Gruppe bestehend aus CH₂, NH und O, bevorzugt CH₂ und O, besonders bevorzugt O;
R^{a} und R^{b} gleich oder verschieden, ein Rest ausgewählt aus der Gruppe bestehend aus Wasserstoff, C₁₋₄-Alkyl, und Halogen-C₁₋₄-alkyl, oder
R^{a} und R^{b} gemeinsam eine C₂₋₅-alkylen-Brücke, in der ein oder mehrere Wasserstoffatome gegebenenfalls ersetzt sein können durch Halogen;
R¹ und R^{1'} gleich oder verschieden, ein Rest ausgewählt aus der Gruppe bestehend aus Wasserstoff, C₁₋₆-Alkyl, C₃₋₆-Cycloalkyl, Halogen-C₁₋₆-alkyl, Halogen-C₃₋₆-cycloalkyl oder C₁₋₆-Alkylen-C₃₋₆-cycloalkyl, oder
R¹ und R^{1'} gemeinsam eine C₂₋₅-alkylen-Brücke in der ein oder mehrere Wasserstoffatome gegebenenfalls ersetzt sein können durch Halogen;
R², R^{2'}, R^{2"} und R^{2"'} gleich oder verschieden, ein Rest ausgewählt aus der Gruppe bestehend aus Wasserstoff, C₁₋₆-Alkyl, Halogen-C₁₋₆-alkylen, OH, HO-C₁₋₆-alkylen, -O-C₁₋₆-Alkyl, C₆₋₁₀-Aryl, C₆₋₁₀-Aryl-C₁₋₄-alkylen, C₆₋₁₀-Aryl-C₁₋₆-alkylen-O-, COOH, COOC₁₋₆-alkyl, O-C₁₋₆-alkylen-COOH, O-C₁₋₆-alkylen-COOC₁₋₆-alkyl, NHSO₂-C-₁₋₆-alkyl, CN, NH₂, NH-C₁₋₆-Alkyl, N(C₁₋₆-Alkyl)₂, NO₂, S-C₁₋₆-Alkyl, SO₂-C₁₋₆-Alkyl, SO-C₁₋₆-Alkyl, O(CO)C₁₋₆-Alkyl, COC₁₋₆-Alkyl, NHCOC₁₋₆-Alkyl oder Halogen;
n 0, 1 oder 2; bevorzugt 1;
bedeuten, als weiteren Wirkstoff **2** eine oder mehrere Verbindungen enthalten, die ausgewählt sind aus den Klassen der Anticholinergika (**2a**), PDEIV-Inhibitoren (**2b**), Steroide (**2c**), LTD4-Antagonisten (**2d**) und EGFR-Hemmer (**2e**).

2. Arzneimittelkombinationen nach Anspruch 1, die eine oder mehrere Verbindungen der allgemeinen Formel **1** enthalten, worin
X -O-, -CH₂-O-, -C(Me)₂-O- oder -CH=CH-;
V eine zweibindige Gruppe ausgewählt aus der Gruppe bestehend aus CH₂, NH und O, bevorzugt CH₂ und O, besonders bevorzugt O;
R^{a} und R^{b} gleich oder verschieden, ein Rest ausgewählt aus der Gruppe bestehend aus Wasserstoff, C₁₋₄-Alkyl und Fluor-C₁₋₄-alkyl, oder
R^{a} und R^{b} gemeinsam eine Gruppe ausgewählt aus -CH₂-CH₂-, -CH₂-CH₂-CH₂-CH₂- und -CH₂-CH₂-CH₂-CH₂-CH₂-, in der ein oder mehrere Wasserstoffatome gegebenenfalls ersetzt sein können durch Fluor oder Chlor, bevorzugt Fluor;
R¹ und R^{1'} gleich oder verschieden, ein Rest ausgewählt aus der Gruppe bestehend aus Wasserstoff, C₁₋₆-Alkyl, C₃₋₆-Cycloalkyl, Halogen-C₁₋₆-alkyl oder C₁₋₆-Alkylen-C₃₋₆-Cycloalkyl, oder
R¹ und R^{1'} gemeinsam eine Gruppe ausgewählt aus -CH₂-CH₂-, -CH₂-CH₂-CH₂-CH₂- und -CH₂-CH₂-CH₂-CH₂-CH₂-, in der ein oder mehrere Wasserstoffatome gegebenenfalls ersetzt sein können durch Fluor oder Chlor, bevorzugt Fluor;
R², R^{2'}, R^{2"} und R^{2"'} gleich oder verschieden, ein Rest ausgewählt aus der Gruppe bestehend aus Wasserstoff, C₁₋₄-Alkyl, CF₃, CHF₂, CH₂F, OH, -O-C₁₋₄-Alkyl, Phenyl, Phenylethyl, Benzyl, Phenyloxy, Benzyloxy, COOH, COOC₁₋₄-alkyl, OCH₂COOH, OCH₂COOC₁₋₄-alkyl, NHSO₂-C₁₋₄-alkyl, Fluor, Chlor oder Brom;
n 0, 1 oder 2; bevorzugt 1;
bedeuten.

3. Arzneimittelkombinationen nach Anspruch 1 oder 2, die eine oder mehrere Verbindungen der allgemeinen Formel **1** enthalten, worin
X -O-, -CH₂-O-, -C(Me)₂-O- oder -CH=CH-;
V eine zweibindige Gruppe ausgewählt aus der Gruppe bestehend aus CH₂ und O, bevorzugt O;
R^{a} und R^{b} gleich oder verschieden, ein Rest ausgewählt aus der Gruppe bestehend aus Wasserstoff, Methyl, Ethyl und CF₃, bevorzugt Wasserstoff, Methyl oder Ethyl, oder
R^{a} und R^{b} gemeinsam eine Gruppe ausgewählt aus -CH₂-CH₂- und -CH₂-CH₂-CH₂-CH₂-, bevorzugt -CH₂-CH₂-;
R¹ und R^{1'} gleich oder verschieden, ein Rest ausgewählt aus der Gruppe bestehend aus Wasserstoff, Methyl, Ethyl, Propyl, Cyclopropyl oder Methylcyclopropyl, oder
R¹ und R^{1'} gemeinsam -CH₂-CH₂-CH₂-CH₂- oder -CH₂-CH₂-CH₂-CH₂-CH₂-;
R², R^{2'}, R^{2"} und R^{2"'} gleich oder verschieden, ein Rest ausgewählt aus der Gruppe bestehend aus Wasserstoff, Methyl, Ethyl, Propyl, CF₃, CHF₂, CH₂F, OH, Methyloxy, Ethyloxy, Propyloxy, COOH, COOCH₃, COOCH₂CH₃, OCH₂COOH, OCH₂COOCH₃, NHSO₂-CH₃, Fluor, Chlor oder Brom;
n 0, 1 oder 2; bevorzugt 1
bedeuten.

4. Arzneimittelkombinationen nach einem der Ansprüche 1 bis 3, die eine oder mehrere Verbindungen der allgemeinen Formel **1** in Form der einzelnen optischen Isomeren, Mischungen der einzelnen Enantiomeren oder Racemate enthalten.

5. Arzneimittelkombinationen nach einem der Ansprüche 1 bis 4, die eine oder mehrere Verbindungen der allgemeinen Formel **1** in Form der Säureadditionssalze mit pharmakologisch unbedenklichen Säuren sowie gegebenenfalls in Form der Solvate und/oder Hydrate enthalten.

6. Arzneimittelkombinationen nach einem der Ansprüche 1 bis 5, die neben einer oder mehrerer Verbindungen der allgemeinen Formel **1** als weiteren Wirkstoff **2** ein Anticholinergikum (**2a**) enthalten.

7. Arzneimittelkombinationen nach Anspruch 6, die neben einer oder mehrerer Verbindungen der allgemeinen Formel **1** ein Anticholinergikum (**2a**) ausgewählt aus der Gruppe bestehend aus Tiotropiumsalzen (**2a.1**), Oxitropiumsalzen (**2a.2**), Flutropiumsalzen (**2a.3**), Ipratropiumsalzen (**2a.4**), Glycopyrroniumsalzen (**2a.5**), Trospiumsalzen (**2a.6**) enthalten.

8. Arzneimittelkombinationen nach einem der Ansprüche 1 bis 5, die neben einer oder mehrerer Verbindungen der allgemeinen Formel **1** als weiteren Wirkstoff **2** ein Steroid (**2c**) enthalten.

9. Arzneimittelkombinationen nach einem der Ansprüche 1 bis 5, **dadurch gekennzeichnet, dass** sie neben therapeutisch wirksamen Mengen von **1** ferner therapeutisch wirksame Mengen eines Anticholinergikums (**2a**), sowie therapeutische Mengen eines Steroids (**2c**), sowie gegebenenfalls einen pharmazeutisch verträglichen Trägerstoff enthalten.

10. Arzneimittelkombination nach einem der Ansprüche 1 bis 9, **dadurch gekennzeichnet, dass** sie in Form einer für die Inhalation geeigneten Darreichungsform vorliegt.

11. Arzneimittelkombination nach Anspruch 10, **dadurch gekennzeichnet, dass** es sich um eine Darreichungsform ausgewählt aus der Gruppe Inhalationspulver, treibgashaltige Dosieraerosole und treibgasfreie Inhalationslösungen oder -suspensionen handelt.

12. Verwendung einer Arzneimittelkombination nach einem der Ansprüche 1 bis 11, zur Herstellung eines Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

13. Verwendung nach Anspruch 12, zur Herstellung eines Arzneimittels zur Behandlung von obstruktiven Lungenerkrankungen die ausgewählt sind aus der Gruppe bestehend aus Asthma Bronchiale, pädiatrisches Asthma, schweres Asthma, akuter Asthma-Anfall, chronische Bronchitis und COPD (chronisch obstruktive pulmonale Erkrankung), wobei die Verwendung zur Herstellung eines Arzneimittels zur Behandlung von Asthma Bronchiale und COPD erfindungsgemäß besonders bevorzugt ist.

14. Verwendung nach Anspruch 12, zur Herstellung eines Arzneimittels zur Behandlung von Lungenemphysemen, die ihren Ursprung haben in COPD oder α1-Proteinase-Inhibitor-Mangel.

15. Verwendung nach Anspruch 12, zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, bronchoalveoläres Karzinom und Lymphome.

16. Verwendung nach Anspruch 12, zur Herstellung eines Arzneimittels zur Behandlung von Interstitiellen Lungenerkrankungen, die ausgewählt sind aus der Gruppe bestehend aus infektiös bedingte Pneumonien, wie beispielsweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, systemische Sklerodermie oder Sarkoidose, Granulomatosen, wie beispielsweise Morbus Boeck, idiopathische interstitielle Pneumonie oder idiopathische pulmonäre Fibrose (IPF).

17. Verwendung nach Anspruch 12, zur Herstellung eines Arzneimittels zur Behandlung von Zystischer Fibrose bzw. Mukoviszidose.

18. Verwendung nach Anspruch 12, zur Herstellung eines Arzneimittels zur Behandlung von Bronchitiden, wie Bronchitis aufgrund bakterieller oder viraler Infektion, Allergische Bronchitis und Toxische Bronchitis.

19. Verwendung nach Anspruch 12, zur Herstellung eines Arzneimittels zur Behandlung von Bronchiektasen.

20. Verwendung nach Anspruch 12, zur Herstellung eines Arzneimittels zur Behandlung von ARDS (adult respiratory distress syndrom).

21. Verwendung nach Anspruch 12, zur Herstellung eines Arzneimittels zur Behandlung von Lungenödemen.

## Claims

1. Medicament combinations which contain, in addition to one or more compounds of general formula **1** 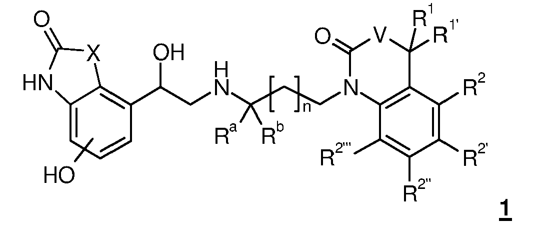 wherein
X denotes a group -O-, -NH-, -CH₂-O-, -CHMe-O-, -C(Me)₂-O-, -CH₂-NH-, -CHMe-NH-, -C(Me)₂-NH-, -CH=CH- or -CH₂-CH₂-;
V denotes a double-bonded group selected from among CH₂, NH and O, preferably CH₂ and O, particularly preferably O;
R^{a} and R^{b} which may be identical or different, denote a group selected from among hydrogen, C₁₋₄-alkyl, and halogen-C₁₋₄-alkyl, or
R^{a} and R^{b} together denote a C₂₋₅-alkylene bridge, wherein one or more hydrogen atoms may optionally be replaced by halogen;
R¹ and R^{1'} which may be identical or different, denote a group selected from among hydrogen, C₁₋₆-alkyl, C₃₋₆-cycloalkyl, halogen-C₁₋₆-alkyl, halogen-C₃₋₆-cycloalkyl or C₁₋₆-alkylene-C₃₋₆-cydoalkyl, or
R¹ and R^{1'} together denote a C₂₋₅-alkylene bridge wherein one or more hydrogen atoms may optionally be replaced by halogen;
R², R^{2'}, R^{2"} and R^{2'"}, which may be identical or different, denote a group selected from among hydrogen, C₁₋₆-alkyl, halogen-C₁₋₆-alkylene, OH, HO-C₁₋₆-alkylene, -O-C₁₋₆-alkyl, C₆₋₁₀-aryl, C₆₋₁₀-aryl-C₁₋₄-alkylene, C₆₋₁₀-aryl-C₁₋₆-alkylene-O, COOH, COOC₁₋₆-alkyl, O-C₁₋₆-alkylene-COOH, O-C₁₋₆-alkylene-COOC₁₋₆-alkyl, NHSO₂-C₁₋₆-alkyl, CN, NH₂, NH-C₁₋₆-alkyl, N(C₁₋₆-alkyl)₂, NO₂, S-C₁₋₆-alkyl, SO₂-C₁₋₆-alkyl, SO-C₁₋₆-alkyl, O(CO)C₁₋₆-alkyl, COC₁₋₆-alkyl, NHCOC₁₋₆-alkyl or halogen;
n denotes 0, 1 or 2; preferably 1;
as a further active substance **2** one or more compounds which are selected from among the categories of the anticholinergics (**2a**), PDEIV-inhibitors (**2b**), steroids (**2c**), LTD4-antagonists (**2d**) and EGFR-inhibitors (**2e**).

2. Medicament combinations according to claim 1, which contain one or more compounds of general formula **1**, wherein
X denotes -O-, -CH₂-O-, -C(Me)₂-O- or -CH=CH-;
V denotes a double-bonded group selected from among CH₂, NH and O, preferably CH₂ and O, particularly preferably O;
R^{a} and R^{b} which may be identical or different, denote a group selected from among hydrogen, C₁₋₄-alkyl and fluoro-C₁₋₄-alkyl, or
R^{a} and R^{b} together denote a group selected from -CH₂-CH₂-, -CH₂-CH₂-CH₂-CH₂- and -CH₂-CH₂-CH₂-CH₂-CH₂-, wherein one or more hydrogen atoms may optionally be replaced by fluorine or chlorine, preferably fluorine;
R¹ and R^{1'} which may be identical or different, denote a group selected from among hydrogen, C₁₋₆-alkyl, C₃₋₆-cycloalkyl, halogen-C₁₋₆-alkyl or C₁₋₆-alkylene-C₃₋₆-cydoalkyl, or
R¹ and R^{1'} together denote a group selected from -CH₂-CH₂, -CH₂-CH₂-CH₂-CH₂- and -CH₂-CH₂-CH₂-CH₂-CH₂, wherein one or more hydrogen atoms may optionally be replaced by fluorine or chlorine, preferably fluorine;
R², R^{2'}, R^{2"} and R^{2"'} which may be identical or different, denote a group selected from among hydrogen, C₁₋₄-alkyl, CF₃, CHF₂, CH₂F, OH, -O-C₁₋₄-alkyl, phenyl, phenylethyl, benzyl, phenyloxy, benzyloxy, COOH, COOC₁₋₄-alkyl, OCH₂COOH, OCH₂COOC₁₋₄-alkyl, NHSO₂-C₁₋₄-alkyl, fluorine, chlorine or bromine;
n denotes 0, 1 or 2; preferably 1.

3. Medicament combinations according to claim 1 or 2, which contain one or more compounds of general formula **1**, wherein
X denotes -O-, -CH₂-O-, -C(Me)₂-O- or -CH=CH-;
V denotes a double-bonded group selected from among CH₂ and O, preferably O;
R^{a} and R^{b} which may be identical or different, denote a group selected from among hydrogen, methyl, ethyl and CF₃, preferably hydrogen, methyl or ethyl, or
R^{a} and R^{b} together denote a group selected from -CH₂-CH₂- and -CH₂-CH₂-CH₂-CH₂-, preferably -CH₂-CH₂-;
R¹ and R^{1'} which may be identical or different, denote a group selected from among hydrogen, methyl, ethyl, propyl, cyclopropyl or methylcyclopropyl, or
R¹ and R^{1'} together denote -CH₂-CH₂-CH₂-CH₂- or -CH₂-CH₂-CH₂-CH₂-CH₂-;
R², R^{2'}, R^{2"} and R^{2"'}, which may be identical or different, denote a group selected from among hydrogen, methyl, ethyl, propyl, CF₃, CHF₂, CH₂F, OH, methyloxy, ethyloxy, propyloxy, COOH, COOCH₃, COOCH₂CH₃, OCH₂COOH, OCH₂COOCH₃, NHSO₂-CH₃, fluorine, chlorine or bromine;
n denotes 0, 1 or 2; preferably 1.

4. Medicament combinations according to one of claims 1 to 3, which contain one or more compounds of general formula **1** in the form of the individual optical isomers, mixtures of the individual enantiomers or racemates.

5. Medicament combinations according to one of claims 1 to 4, which contain one or more compounds of general formula **1** in the form of the acid addition salts with pharmacologically acceptable acids as well as optionally in the form of the solvates and/or hydrates.

6. Medicament combinations according to one of claims 1 to 5 which contain an anticholinergic (**2a**) as an additional active substance **2** in addition to one or more compounds of general formula **1**.

7. Medicament combinations according to claim 6 which contain an anticholinergic (**2a**) selected from among tiotropium salts (**2a.1**), oxitropium salts (**2a.2**), flutropium salts (**2a.3**), ipratropium salts (**2a.4**), glycopyrronium salts (**2a.5**) and trospium salts (**2a.6**), in addition to one or more compounds of general formula **1**.

8. Medicament combinations according to one of claims 1 to 5 which contain a steroid (**2c**) as an additional active substance **2** in addition to one or more compounds of general formula **1**.

9. Medicament combinations according to one of claims 1 to 5, **characterised in that**, in addition to therapeutically effective amounts of **1**, they further contain therapeutically effective amounts of an anticholinergic (**2a**) as well as therapeutic amounts of a steroid (**2c**), as well as optionally a pharmaceutically acceptable carrier.

10. Medicament combination according to one of claims 1 to 9, **characterised in that** it is in the form of a formulation suitable for inhalation.

11. Medicament combination according to claim 10, **characterised in that** it is a preparation selected from the group comprising inhalable powders, propellant-driven metered-dose aerosols and propellant-free inhalable solutions or suspensions.

12. Use of a medicament combination according to one of claims 1 to 11 for preparing a medicament for the treatment of respiratory complaints selected from the group comprising obstructive pulmonary diseases of various origins, pulmonary emphysema of various origins, restrictive pulmonary diseases, interstitial pulmonary diseases, cystic fibrosis, bronchitis of various origins, bronchiectasis, ARDS (adult respiratory distress syndrome) and all forms of pulmonary oedema.

13. Use according to claim 12, for preparing a medicament for the treatment of obstructive pulmonary diseases selected from among bronchial asthma, paediatric asthma, severe asthma, acute asthma attacks, chronic bronchitis and COPD (chronic obstructive pulmonary disease), the use for preparing a medicament for the treatment of bronchial asthma and COPD being particularly preferred according to the invention.

14. Use according to claim 12, for preparing a medicament for the treatment of pulmonary emphysema which has its origins in COPD or α1-proteinase inhibitor deficiency.

15. Use according to claim 12, for preparing a medicament for the treatment of restrictive pulmonary diseases selected from among allergic alveolitis, restrictive pulmonary diseases triggered by occupational noxious substances, such as asbestosis or silicosis, and restriction caused by lung tumours, such as for example lymphangiosis carcinomatosa, bronchoalveolar carcinoma and lymphomas.

16. Use according to claim 12, for preparing a medicament for the treatment of interstitial pulmonary diseases selected from among pneumonia caused by infections, such as for example infection by viruses, bacteria, fungi, protozoa, helminths or other pathogens, pneumonitis caused by various factors, such as for example aspiration and left heart insufficiency, radiation-induced pneumonitis or fibrosis, collagenoses, such as for example lupus erythematodes, systemic scleroderma or sarcoidosis, granulomatoses, such as for example Boeck's disease, idiopathic interstitial pneumonia or idiopathic pulmonary fibrosis (IPF).

17. Use according to claim 12, for preparing a medicament for the treatment of cystic fibrosis or mucoviscidosis.

18. Use according to claim 12, for preparing a medicament for the treatment of bronchitis, such as bronchitis caused by bacterial or viral infection, allergic bronchitis and toxic bronchitis.

19. Use according to claim 12, for preparing a medicament for the treatment of bronchiectasis.

20. Use according to claim 12, for preparing a medicament for the treatment of ARDS (adult respiratory distress syndrome).

21. Use according to claim 12, for preparing a medicament for the treatment of pulmonary oedema.

## Revendications

1. Combinaisons médicamenteuses qui contiennent,
outre un ou plusieurs composés de formule générale 1 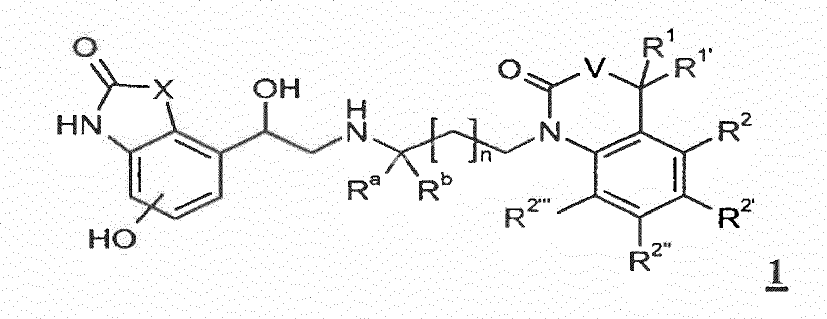 dans laquelle
X est un groupe -O-, -NH-, -CH₂-O-, -CHMe-O-, - C(Me)₂-O-, -CH₂-NH-, -CHMe-NH-, -C(Me)₂-NH-, -CH=CH- ou -CH₂-CH₂- ;
V est un groupe à deux liaisons choisi dans le groupe comprenant CH₂, NH et O, de préférence, CH₂ et O, de manière particulièrement préférée O ;
R^{a} et R^{b}, identiques ou différents, désignent un radical choisi dans le groupe comprenant un atome d'hydrogène, un groupe alkyle en C₁ à C₄ et un groupe halogéno-alkyle en C₁ à C_{4;}
ou
R^{a} et R^{b} désignent conjointement un pont alkylène en C₂ à C₅ dans lequel un ou plusieurs atomes d'hydrogène peuvent être éventuellement substitués par un atome d'halogène ;
R¹ et R^{1'}, identiques ou différents, désignent un radical choisi dans le groupe comprenant un atome d'hydrogène, un groupe alkyle en C₁ à C₆, cycloalkyle en C₃ à C₆, halogéno-alkyle en C₁ à C₆, halogéno-cycloalkyle en C₃ à C₆ ou alkylène en C₁ à C₆-cycloalkyle en C₃ à C₆, ou
R¹ et R^{1'} désignent conjointement un pont alkylène en C₂ à C₅ dans lequel un ou plusieurs atomes d'hydrogène peuvent être substitués éventuellement par un atome d'halogène ;
R², R^{2'}, R^{2"} et R^{2"'} identiques ou différents, désignent un radical choisi dans le groupe comprenant un atome d'hydrogène, un groupe alkyle en C₁ à C₆, halogéno-alkylène en C₁ à C₆, OH, HO-alkylène en C₁ à C₆, -O-allyle en C₁ à C₆, aryle en C₁ à C₆, aryle en C₆ à C₁₀-alkylène en C₁ à C₄, aryle en C₆ à C₁₀-alkylène en C₁ à C₆-O-, COOH, COOC-alkyle en C₁ à C₆, O-alkylène en C₁ à C₆-COOH, O-alkylène en C₁ à C₆-COOC-alkyle en C₁ à C₆, NHSO₂-alkyle en C₁ à C₆,, CN, NH₂, NH-alkyle en C₁ à C₆, N(alkyle en C₁ à C₆)₂, NO₂, S-alkyle en C₁ à C₆, SO₂-alkyle en C₁ à C₆; SO-alkyle en C₁ à C₆, O(CO)-alkyle en C₁ à C₆, COC-alkyle en C₁ à C₆, NHCOC-alkyle en C₁ à C₆ ou un atome d'halogéne ;
n est 0, 1 ou 2 ; de préférence 1 ;
comme autre substance active 2, un ou plusieurs composés qui sont choisis dans les classes d'anti-cholinergiques (2a), les inhibiteurs de PDEIV (2b), les stéroïdes (2c), les antagonistes de LTD4 (2d) et les inhibiteurs de l'EGFR (2e).

2. Combinaisons médicamenteuses selon la revendication 1, qui contiennent un ou plusieurs composés de formule générale 1, dans laquelle
X est un groupe -O-, -CH₂-O-, -C(Me)₂-O- ou - CH=CH- ;
V est un groupe à deux liaisons choisi dans le groupe comprenant CH₂, NH et O, de préférence, CH₂ et O, de manière particulièrement préférée O ;
R^{a} et R^{b}, identiques ou différents, désignent un radical choisi dans le groupe comprenant un atome d'hydrogène, un groupe alkyle en C₁ à C₄ et un groupe fluoro-alkyle en C₁ à C₄,
ou
R^{a} et R^{b} désignent conjointement un groupe choisi parmi -CH₂-CH₂-, -CH₂-CH₂-CH₂₋CH₂- et -CH₂-CH₂-CH₂-CH₂-CH₂- dans lequel un ou plusieurs atomes d'hydrogène peuvent être éventuellement substitués par un atome de fluor ou de chlore, de préférence, de fluor ;
R¹ et R^{1'}, identiques ou différents, désignent un radical choisi dans le groupe comprenant un atome d'hydrogène, un groupe alkyle en C₁ à C₆, cycloalkyle en C₃ à C₆, halogéno-alkyle en C₁ à C₆ ou alkylène en C₁ à C₆-cycloalkyle en C₃ à C₆, ou
R¹ et R^{1'} désignent conjointement un groupe choisi parmi -CH₂-CH₂-, -CH₂-CH₂-CH₂-CH₂- et -CH₂-CH₂-CH₂-CH₂-CH₂- dans lequel un ou plusieurs atomes d'hydrogène peuvent être substitués éventuellement par un atome de fluor ou de chlore, de préférence de fluor ;
R², R^{2'}, R^{2"} et R^{2"'} identiques ou différents, désignent un radical choisi dans le groupe comprenant un atome d'hydrogène, un groupe alkyle en C₁ à C₄, CF₃, CHF₂, CH₂F, OH, -O-alkyle en C₁ à C₄, phényle, phényléthyle, benzyle, phényloxy, benzyloxy, COOH, COOC-alkyle en C₁ à C₄, OCH₂COOH, OCH₂COOC-alkyle en C₁ à C₄, NHSO₂-alkyle en C₁ à C₄, un atome de fluor, de chlore ou de brome ;
n est 0, 1 ou 2 ; de préférence 1.

3. Combinaisons médicamenteuses selon la revendication 1 ou 2, qui contiennent un ou plusieurs composés de formule générale 1, dans laquelle
X est un groupe -O-, -CH₂-O-, -C(Me)₂-O- ou - CH=CH- ;
V est un groupe à deux liaisons choisi dans le groupe comprenant CH₂ et O, de préférence, O ;
R^{a} et R^{b}, identiques ou différents, désignent un radical choisi dans le groupe comprenant un atome d'hydrogène, un groupe méthyle, éthyle et CF₃, de préférence un atome d'hydrogène, un groupe méthyle ou éthyle, ou
ou
R^{a} et R^{b} désignent conjointement un groupe choisi parmi CH₂-CH₂- et -CH₂-CH₂-CH₂-CH₂-CH₂-, de préférence, -CH₂-CH₂- ;
R¹ et R^{1'}, identiques ou différents, désignent un radical choisi dans le groupe comprenant un atome d'hydrogène, un groupe méthyle, éthyle, propyle, cyclopropyle ou méthylcyclopropyle, ou
R¹ et R^{1'} désignent conjointement -CH₂-CH₂-CH₂-CH₂- ou -CH₂-CH₂-CH₂-CH₂-CH₂- ;
R², R^{2'}, R^{2"} et R^{2"'} identiques ou différents, désignent un radical choisi dans le groupe comprenant un atome d'hydrogène, un groupe méthyle, éthyle, propyle, CF₃, CHF₂, CH₂F, OH, méthyloxy, éthyloxy, propyloxy, COOH, COOCH₃, COOHCH₂CH₃, OCH₂COOH, OCH₂COOCH₃, NHSO₂-CH₃, un atome de fluor, de chlore ou de brome ;
n est 0, 1 ou 2 ; de préférence 1.

4. Combinaisons médicamenteuses selon l'une des revendications 1 à 3, qui contiennent un ou plusieurs composés de formule générale 1 sous forme d'isomères optiques individuels, de mélanges des énantiomères individuels ou de racémates.

5. Combinaisons médicamenteuses selon l'une des revendications 1 à 4, qui contiennent un ou plusieurs composés de formule générale 1 sous forme de sels d'addition acides avec des acides pharmacologiquement acceptables et éventuellement sous forme de solvates et/ou d'hydrates.

6. Combinaisons médicamenteuses selon l'une des revendications 1 à 5, qui contiennent outre un ou plusieurs composés de formule générale 1, un anti-cholinergique (2a) comme autre substance active 2.

7. Combinaisons médicamenteuses selon la revendication 6, qui contiennent outre un ou plusieurs composés de formule générale 1, un anti-cholinergique (2a) choisi dans le groupe comprenant des sels de tiotropium (2a.1), des sels d'oxytropium (2a.2), des sels de flutropium (2a.3), des sels d'ipratropium (2a.4), des sels de glycopyrronium (2a.5), des sels de trospium (2a.6).

8. Combinaisons médicamenteuses selon l'une des revendications 1 à 5, qui contiennent, outre un ou plusieurs composés de formule générale 1, un stéroïde (2c) comme autre substance active 2.

9. Combinaisons médicamenteuses selon l'une des revendications 1 à 5, **caractérisées en ce que** qu'elles contiennent, outre des quantités thérapeutiquement efficaces du composé 1, également des quantités thérapeutiquement efficaces d'un anti-cholinergique (2a) et des quantités thérapeutiques d'un stéroïde (2c) et éventuellement d'un véhicule pharmaceutiquement acceptable.

10. Combinaison médicamenteuse selon l'une des revendications 1 à 9, **caractérisée en ce qu'**elle se présente sous la forme d'une forme galénique appropriée pour l'inhalation.

11. Combinaison médicamenteuse selon la revendication 10, **caractérisée en ce qu'**il s'agit d'une forme galénique choisie dans le groupe comprenant des poudres pour inhalation, des aérosols doseurs contenant un gaz propulseur et des solutions ou suspensions pour inhalation sans gaz propulseur.

12. Utilisation d'une combinaison médicamenteuse selon l'une des revendications 1 à 11, pour la production d'un médicament pour le traitement de maladies des voies respiratoires qui sont choisies dans le groupe comprenant des maladies pulmonaires obstructives d'origines diverses, des emphysèmes pulmonaires d'origines diverses, des maladies pulmonaires restrictives, des maladies pulmonaires interstitielles, des fibroses kystiques, des bronchopathies d'origines diverses, des bronchiectasies, le SDRA (syndrome de détresse respiratoire de l'adulte et toutes les formes d'oedèmes pulmonaires.

13. Utilisation selon la revendication 12, pour la production d'un médicament pour le traitement de maladies pulmonaires obstructives qui sont choisies dans le groupe comprenant l'asthme, l'asthme pédiatrique, l'asthme sévère, les crises d'asthme aiguës, la bronchite chronique et la BPCO (broncho-pneumopathie chronique obstructive), l'utilisation pour la production d'un médicament destiné au traitement de l'asthme et de la BPCO selon l'invention étant particulièrement préférée.

14. Utilisation selon la revendication 12, pour la production d'un médicament pour le traitement des emphysèmes pulmonaires qui ont leur origine dans la BPCO ou le déficit de l'inhibiteur de l'α1-protéinase.

15. Utilisation selon la revendication 12, pour la production d'un médicament pour le traitement des maladies pulmonaires restrictives qui sont choisies dans le groupe comprenant l'alvéolite allergique, les maladies pulmonaires restrictives déclenchées par des polluants professionnels telles que l'asbestose ou la silicose et la restriction due à des tumeurs pulmonaires telles que par exemple, la lymphangiose carcinomateuse, le carcinome broncho-alvéolaire et les lymphomes.

16. Utilisation selon la revendication 12, pour la production d'un médicament pour le traitement de maladies pulmonaires interstitielles qui sont choisies dans le groupe comprenant les pneumonies d'origine infectieuse telles que par exemple, dues à une infection par des virus, des bactéries, des champignons, des protozoaires, des vers ou autres agents pathogènes, la pneumonite d'origines diverses telle que par exemple, l'aspiration ou l'insuffisance cardiaque gauche, la pneumonite induite par des rayons ou les fibroses, les collagénoses telles que par exemple, le lupus érythémateux, la sclérodermie systémique ou la sarcoïdose, les granulomatoses telles que par exemple, la maladie de Boeck, la pneumonie interstitielle idiopathique ou la fibrose pulmonaire idiopathique (IPF).

17. Utilisation selon la revendication 12, pour la production d'un médicament pour le traitement de fibroses kystiques ou de mucoviscidose.

18. Utilisation selon la revendication 12, pour la production d'un médicament pour le traitement des bronchopathies telles que la bronchite due à une infection bactérienne ou virale, la bronchite allergique et la bronchite toxique.

19. Utilisation selon la revendication 12, pour la production d'un médicament pour le traitement des bronchiectasies.

20. Utilisation selon la revendication 12, pour la production d'un médicament pour le traitement du SDRA (syndrome de détresse respiratoire de l'adulte).

21. Utilisation selon la revendication 12, pour la production d'un médicament pour le traitement des oedèmes pulmonaires.
